# EUROPEAN PATENT APPLICATION

(11) **EP 4 813 996 A1**
(43) Date of publication of application: **30.09.2026**
(21) Application number: 24896440.5
(22) Date of filing: 22.11.2024
(51) Int. Cl.: C07F 9/572, C07F 9/6561, C07F 5/02, C07D 487/04, H10K 85/60, H10K 30/50

(54) **ORGANIC COMPOUND, SOLAR CELL, AND USE THEREOF**

(30) Priority: 27.11.2023 CN 202311596550
(71) Applicant: Contemporary Amperex Future Energy Research Institute (Shanghai) Limited, Shanghai 200241 (CN); Contemporary Amperex Technology Co., Limited, Ningde, Fujian 352100 (CN)
(72) Inventor: JIA, Boyu, Shanghai 200241 (CN); LIANG, Weifeng, Shanghai 200241 (CN); ZHOU, Chenhong, Shanghai 200241 (CN); SHI, Ruoxuan, Shanghai 200241 (CN); YIN, Zhaoyi, Shanghai 200241 (CN); ZHENG, Yi, Shanghai 200241 (CN); MENG, Ke, Shanghai 200241 (CN); CHEN, Junchao, Shanghai 200241 (CN); GUO, Yongsheng, Shanghai 200241 (CN)
(74) Representative: Gong, Jinping
(86) International application number: PCT/CN2024/133939
(87) International publication number: WO 2025/113349

(57) **Abstract**

This application relates to an organic compound, a solar cell, and an application thereof. The organic compound is represented by formula (1), where Ar is selected from any one of a substituted or unsubstituted aromatic group having 6 to 50 ring-forming atoms, a substituted or unsubstituted heteroaromatic group having 5 to 50 ring-forming atoms, a group represented by formula (A), and a group represented by formula (B); Ar' is selected from electrically neutral organic groups; L is selected from chain alkylene groups having 1 to 10 carbon atoms; R₁ is an oxyacid group; n1 is any integer selected from 1 to 3; and m1 is any integer selected from 1 to 10. Alternatively, the organic compound is an oxyanion salt of a compound represented by formula (1). The organic compound can serve not only as a passivation material but also as a hole transport material. When the organic compound is used for preparing a solar cell, the photoelectric conversion efficiency of the solar cell can be improved.

## Description

### CROSS-REFERENCE TO RELATED APPLICATIONS

This application claims priority to Chinese Patent Application No. 202311596550.X, filed on November 27, 2023, entitled "ORGANIC COMPOUND, SOLAR CELL, AND APPLICATION THEREOF", which is incorporated herein by reference in its entirety.

### TECHNICAL FIELD

This application relates to the technical field of solar cells, and in particular, to an organic compound, a solar cell, and an application thereof.

### BACKGROUND

Perovskite solar cells possess excellent photoelectric properties, high light absorption coefficients, long carrier lifetimes, and long diffusion lengths, making them leaders among third-generation novel solar cells.

However, defects are typically present in bulk phase and surface interfaces of perovskite materials. For example, perovskite films prepared by solution methods typically exhibit grain boundary defects and crystal defects. These defects not only lead to a decrease in crystal quality, affecting carrier transport, but also accelerate the penetration of moisture/oxygen, hastening the degradation of the perovskite, thereby adversely affecting both the efficiency and long-term stability of perovskite solar cells. In addition, conventional hole transport layer materials are unstable and have excessive defects, which also reduce the photoelectric conversion efficiency and stability of solar cells.

Therefore, there is still room for improvement in conventional technologies.

### SUMMARY

According to various embodiments of this application, this application provides an organic compound, a solar cell, and an application thereof and aims to improve the photoelectric conversion efficiency of the solar cell.

This application is implemented through the following technical solutions.

According to a first aspect of this application, this application provides an organic compound, where the organic compound is represented by formula (1): 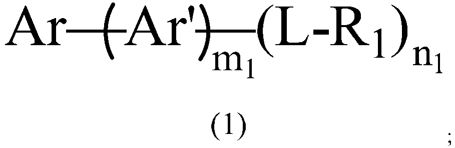
where Ar is selected from any one of a substituted or unsubstituted aromatic group having 6 to 50 ring-forming atoms, a substituted or unsubstituted heteroaromatic group having 5 to 50 ring-forming atoms, a group represented by formula (A), and a group represented by formula (B): 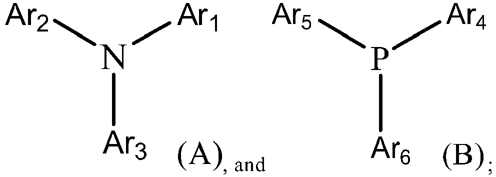
Ar' is selected from any one of a substituted or unsubstituted aryl group having 6 to 30 ring-forming atoms and a substituted or unsubstituted heteroaryl group having 5 to 30 ring-forming atoms;
Ar₁ to Ar₆ are each independently selected from any one of H, a substituted or unsubstituted alkyl group having 1 to 20 carbon atoms, a substituted or unsubstituted alkenyl group having 2 to 30 carbon atoms, and a substituted or unsubstituted aromatic group having 6 to 30 ring-forming atoms, where at least one of Ar₁ to Ar₃ is selected from substituted or unsubstituted aromatic groups having 6 to 30 ring-forming atoms, and at least one of Ar₄ to Ar₆ is selected from substituted or unsubstituted aromatic groups having 6 to 30 ring-forming atoms;
L is selected from a chain alkylene group having 1 to 10 carbon atoms; and
R₁ is an oxyacid group; n₁ is selected from any integer from 1 to 3; and m₁ is selected from any integer from 1 to 10;
or, the organic compound is an oxyanion salt of a compound represented by formula (1).

When the above organic compound is used for preparing a solar cell, the photoelectric conversion efficiency and stability of the solar cell can be improved. The organic compound enables an organic combination of Ar, Ar', L, and R₁ with specific group structures to form the compound of formula (1) or further form an oxyanion salt, where Ar is a terminal group, L is a linking group L, and R₁ is a head group. When the organic compound is used for preparing the solar cell, the terminal group Ar with an aromatic group enables π-π interactions between molecules of the organic compound through π-bonds of the aromatic group, inducing the formation of an ordered self-assembled molecular film. R₁ can bind with metal ions such as trivalent nickel, anchor the hole transport layer, or interact with A-site cations in the perovskite through hydrogen bonds, thereby passivating metal ions. Meanwhile, L reduces steric hindrance. Additionally, introducing a second aryl group Ar' between the terminal group Ar and the linking group L increases the dipole moment of the organic compound while minimally altering molecular energy levels of the organic compound, making the work function of the molecular film formed after self-assembly more compatible with the perovskite layer. When the organic compound is used for preparing the solar cell, the solar cell can exhibit high photoelectric conversion efficiency.

In some embodiments, Ar' at each occurrence is independently selected from any one of the following groups Ar'1 to Ar'7 or any combination thereof: 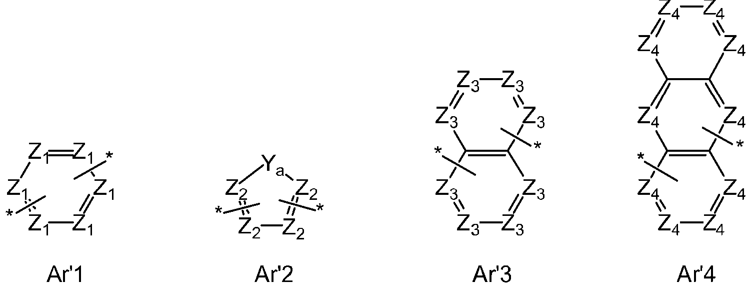 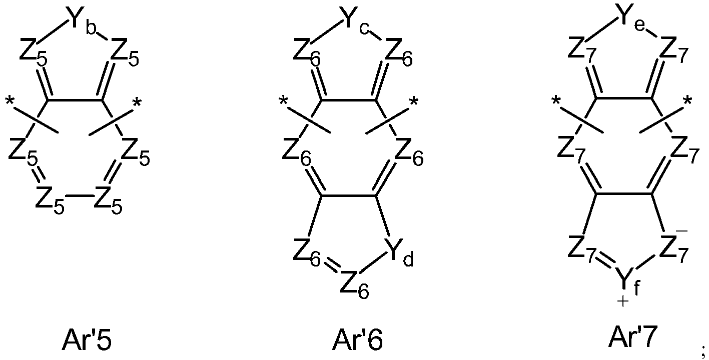
where Yₐ to Y_{f} are each independently selected from any one of -C(R₂R₃)-, -N(R₄)-, -O-, -Si(R₆R₇)-, - P(R₈)-, -S-, -As-, -Se-, -C(=O)-, -C(=S)-, -C(=NR₉)-, and -C(=CR₁₀)-;
Z₁ to Z₇ at each occurrence are each independently selected from C(R₁₁) or N;
R₂ to R₁₁ are each independently selected from H, a halogen group, a substituted or unsubstituted alkyl group having 1 to 5 carbon atoms, an alkoxy group having 1 to 5 carbon atoms, an aromatic group having 6 to 10 ring-forming atoms, a heteroaromatic group having 5 to 10 ring-forming atoms, -OC(=O)R₁₂, -NHC(=O)R₁₃, -N(R₁₄)₂, - L₁N⁺(R₁₅)₃X₁⁻, and -L₂P⁺(R₁₆)₃X₂⁻;
L₁ and L₂ are each independently selected from any one of a single bond and an alkylene group having 1 to 5 carbon atoms; R₁₂ to R₁₆ are each independently selected from H or an alkyl group having 1 to 5 carbon atoms, and R₁₂ and R₁₃ are not H; and X₁⁻ and X₂⁻ are each independently selected from a halogen ion; and
* represents a connection site.

In some embodiments, Ar' satisfies at least one of the following conditions (1) to (3):
(1) Yₐ to Y_{f} are each independently selected from any one of -C(R₂R₃)-, -N(R₄)-, -O-, -Si(R₆R₇)-, -P(R₈)-, and -S-;
(2) R₂ to R₁₀ are each independently selected from any one of H, a halogen group, an alkyl group having 1 to 5 carbon atoms, a halogen-substituted alkyl group having 1 to 5 carbon atoms, and an alkoxy group having 1 to 5 carbon atoms; and
(3) R₁₁ is selected from any one of H, a halogen group, an alkyl group having 1 to 5 carbon atoms, a halogen-substituted alkyl group having 1 to 5 carbon atoms, an aromatic group having 6 to 10 ring-forming atoms, and a heteroaromatic group having 5 to 10 ring-forming atoms.

In some embodiments, Ar' at each occurrence is independently selected from any one of the following groups or any combination thereof: 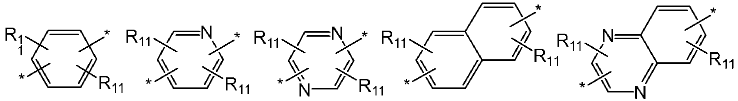 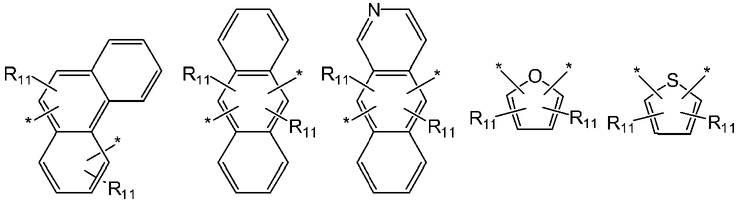 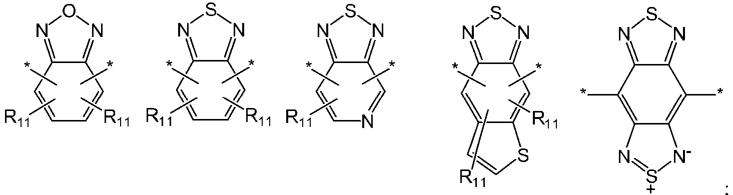 where * represents a connection site.

In some embodiments, Ar is selected from any one of groups formed by removing one hydrogen atom from structures represented by formulas (A) to (G): 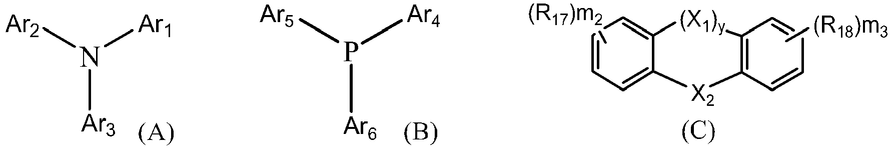 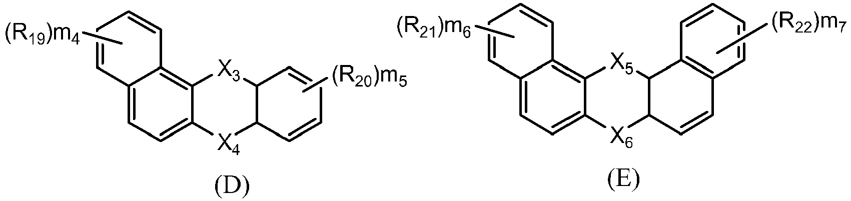 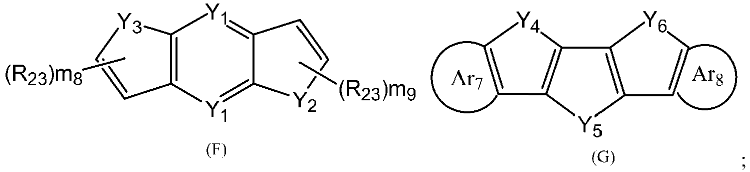
where X₁ to X₆ are each independently selected from any one of a single bond, C(R₂₄R₂₅), O, S, N, NR₂₆, C=O, or S=O, X₁ and X₂ are not both single bonds, X₃ and X₄ are not both single bonds, and X₅ and X₆ are not both single bonds; y is selected from any integer from 1 to 3; and when y is greater than or equal to 2, X₁ is selected from C(R₂₄R₂₅);
Y₁ at each occurrence is independently selected from CR₂₇ or N;
Y₂ to Y₆ are each independently selected from any one of C(R₂₈R₂₉), O, S, N, NR₃₀, C=O, or S=O;
R₁₇ to R₃₀ at each occurrence are each independently selected from any one of H, a halogen group, -N(R₃₁)₂, -CONR₃₂, -OCOR₃₃, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms;
R₃₁ to R₃₃ at each occurrence are each independently selected from any one of H, D, a substituted or unsubstituted alkyl group having 1 to 30 carbon atoms, a substituted or unsubstituted alkenyl group having 2 to 30 carbon atoms, a substituted or unsubstituted aromatic group having 6 to 30 ring-forming atoms, and a substituted or unsubstituted heteroaromatic group having 5 to 30 ring-forming atoms, and R₃₂ and R₃₃ are not H or D;
Ar₇ and Ar₈ are each independently selected from any one of H, a substituted or unsubstituted aromatic group having 6 to 30 ring-forming atoms, and a substituted or unsubstituted heteroaromatic group having 5 to 30 ring-forming atoms; and
m₂, m₃, and m₅ are each independently selected from any integer from 1 to 4; m₄, m₆, and m₇ are each independently selected from any integer from 1 to 6; and m₈ and m₉ are each independently selected from any integer from 1 to 2.

In some embodiments, in formula (C), when X₁ is a single bond and X₂ is selected from NR₂₆, at least one R₁₇ or at least one R₁₈ is not H.

In some embodiments, Ar₇ and Ar₈ are each independently selected from H or any one of the following structures: 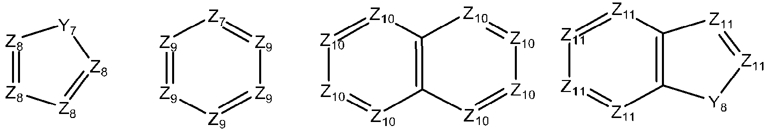 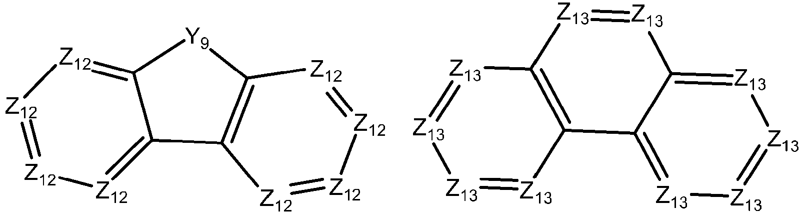 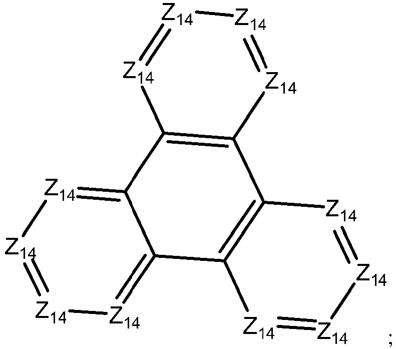
where Y₇ to Y₉ are each independently selected from any one of CR₂₈R₂₉, O, S, S=O, and C=O;
Z₈ to Z₁₄ at each occurrence are each independently selected from CR₂₉ or N, and Z₈ to Z₁₄ in a same structural formula are not all N; and
R₃₄ to R₃₆ at each occurrence are each independently selected from any one of H, D, a substituted or unsubstituted linear alkyl group having 1 to 10 carbon atoms, a substituted or unsubstituted branched alkyl group having 3 to 20 carbon atoms, and a substituted or unsubstituted cycloalkyl group having 3 to 20 carbon atoms.

In some embodiments, Ar is selected from any one of the following groups: 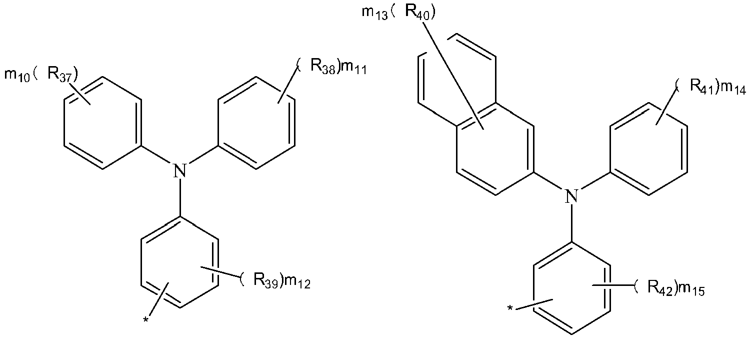 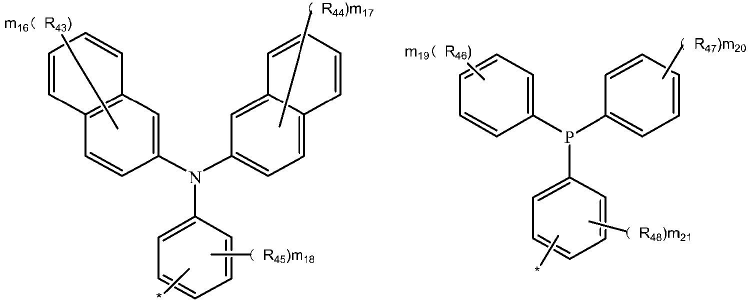 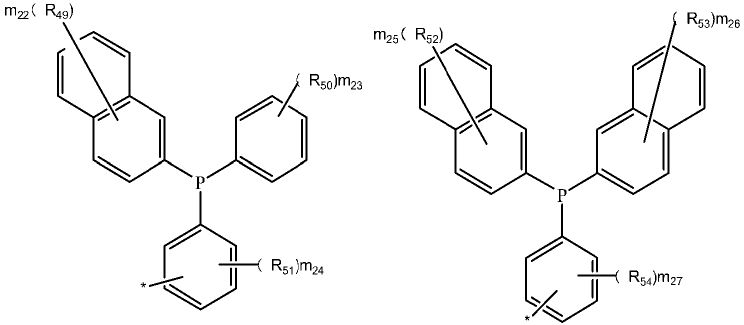 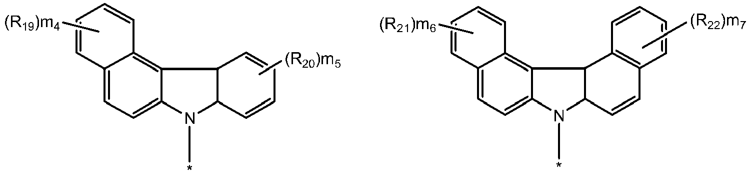 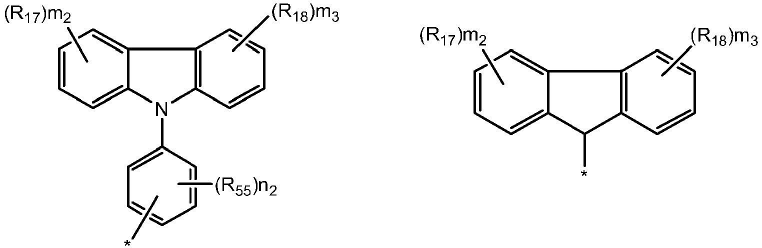 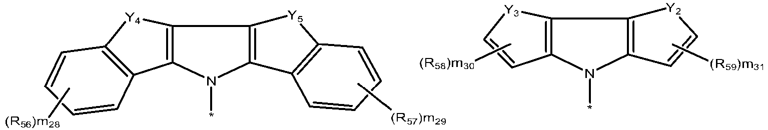 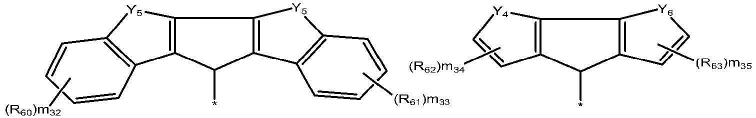 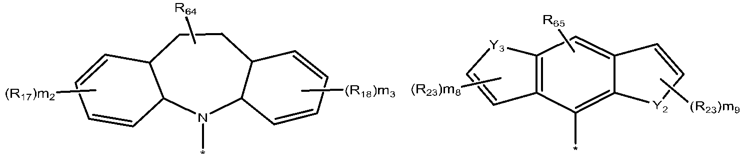 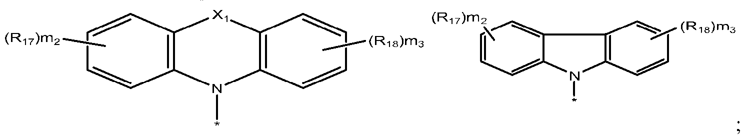
where R₃₇ to R₆₅ at each occurrence are each independently selected from any one of H, a halogen group, -N(R₆₆)₂, -CONR₆₇, -OCOR₆₈, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms; and R₆₆ to R₆₈ are each independently selected from any one of an alkyl group having 1 to 15 carbon atoms, a halogen-substituted alkyl group having 1 to 15 carbon atoms, an aromatic group having 6 to 30 ring-forming atoms, a halogen-substituted aromatic group having 6 to 30 ring-forming atoms, and a heteroaromatic group having 5 to 30 ring-forming atoms; and
m₁₀, m₁₁, m₁₄, m₁₉, m₂₀, and m₂₃ are each independently selected from any integer from 1 to 5; m₁₃, m₁₆, m₁₇, m₂₂, m₂₅, and m₂₆ are each independently selected from any integer from 1 to 6; m₁₂, m₁₅, m₁₈, m₂₁, m₂₄, m₂₇ to m₂₉, m₃₂, m₃₃, and n₂ are each independently selected from any integer from 1 to 4; and m₃₀, m₃₁, m₃₄, and m₃₅ are each independently selected from any integer from 1 to 2.

In some embodiments, the organic compound satisfies at least one of the following conditions (1) and (2):
(1) R₁₇ to R₃₀ and R₃₇ to R₆₅ at each occurrence are each independently selected from any one of H, a halogen group, an alkyl group having 1 to 15 carbon atoms, a halogen-substituted alkyl group having 1 to 15 carbon atoms, an aromatic group having 6 to 30 ring-forming atoms, a halogen-substituted aromatic group having 6 to 30 ring-forming atoms, and a heteroaromatic group having 5 to 30 ring-forming atoms;
   optionally, R₁₇ to R₃₀ and R₃₇ to R₆₅ at each occurrence are each independently selected from any one of H, a halogen group, an alkyl group having 1 to 10 carbon atoms, a halogen-substituted alkyl group having 1 to 10 carbon atoms, an aromatic group having 6 to 15 ring-forming atoms, a halogen-substituted aromatic group having 6 to 15 ring-forming atoms, and a heteroaromatic group having 1 to 15 ring-forming atoms; and
   optionally, R₁₇ to R₃₀ and R₃₇ to R₆₅ at each occurrence are each independently selected from any one of H, a halogen group, a chain alkyl group having 1 to 5 carbon atoms, a halogen-substituted chain hydrocarbon group having 1 to 5 carbon atoms, an aromatic group having 6 to 10 ring-forming atoms, a halogen-substituted aromatic group having 6 to 10 ring-forming atoms, and a heteroaromatic group having 1 to 10 ring-forming atoms; and
(2) R₃₁ to R₃₃ at each occurrence are each independently selected from any one of H, D, an alkyl group having 1 to 15 carbon atoms, a halogen-substituted alkyl group having 1 to 15 carbon atoms, an aromatic group having 6 to 15 ring-forming atoms, a halogen-substituted aromatic group having 6 to 15 ring-forming atoms, a heteroaromatic group having 5 to 15 ring-forming atoms, and a halogen-substituted heteroaromatic group having 5 to 15 ring-forming atoms; and
   optionally, R₃₁ to R₃₃ at each occurrence are each independently selected from any one of H, D, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, a heteroaromatic group having 5 to 10 ring-forming atoms, and a halogen-substituted heteroaromatic group having 5 to 10 ring-forming atoms.

In some embodiments, R₁ at each occurrence is independently selected from any one of a phosphonic acid group, a sulfonic acid group, a carboxylic acid group, a sulfinic acid group, a boric acid group, or a silicic acid group; and
optionally, R₁ at each occurrence is independently selected from any one of the following structures: 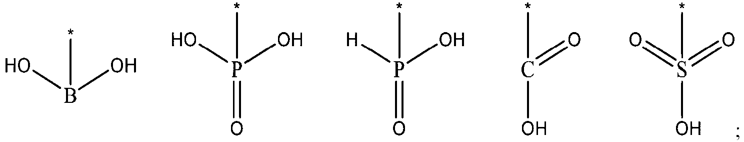
where * represents a connection site.

In some embodiments, the oxyanion salt of the compound represented by formula (1) includes an anion and a cation, where the anion is formed by the loss of H from at least one hydroxyl group in the oxyacid group of the compound represented by formula (1), and the cation is selected from a metal ion or NH₄⁺.

In some embodiments, the organic compound includes at least one of the compounds represented by formulas (SAM1) to (SAM17) and oxyanion salts of the compounds represented by formulas (SAM1) to (SAM17):

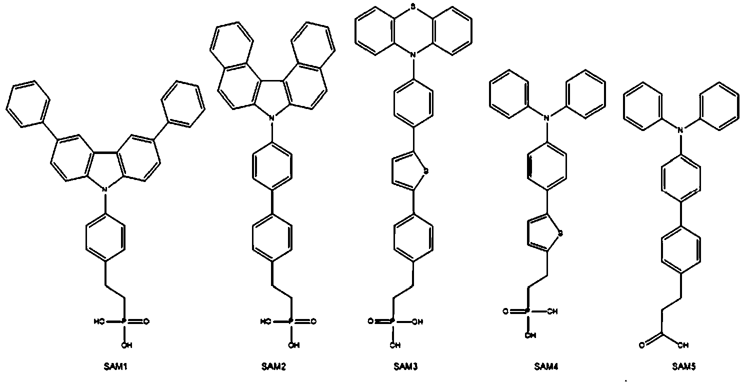

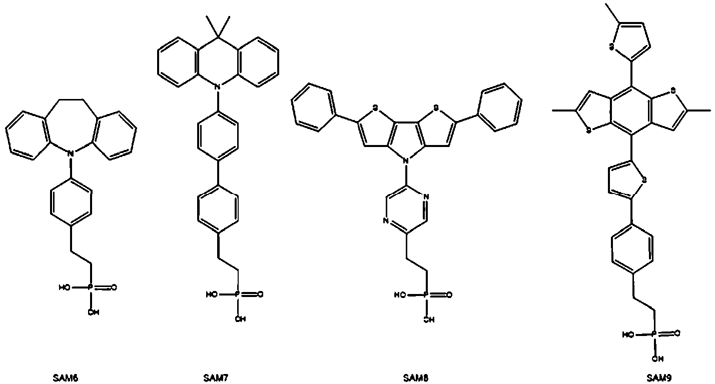

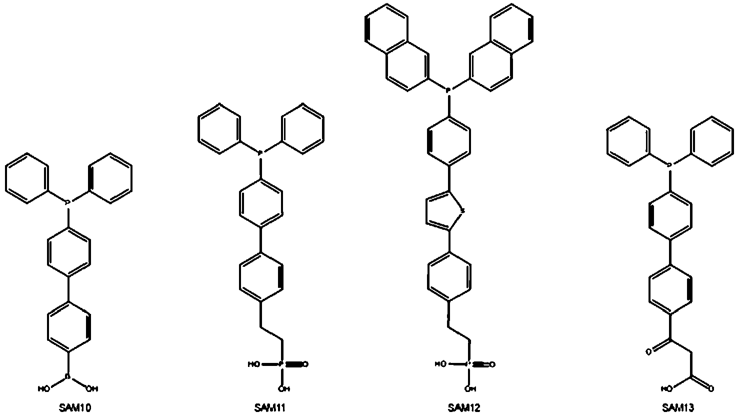

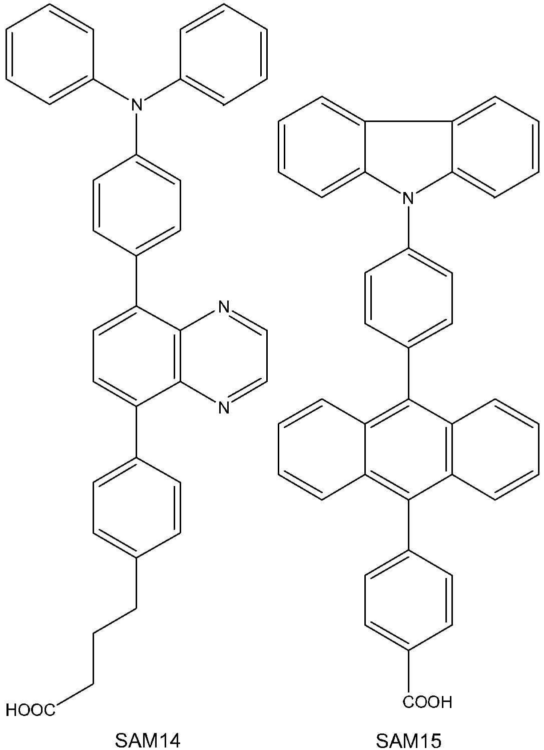

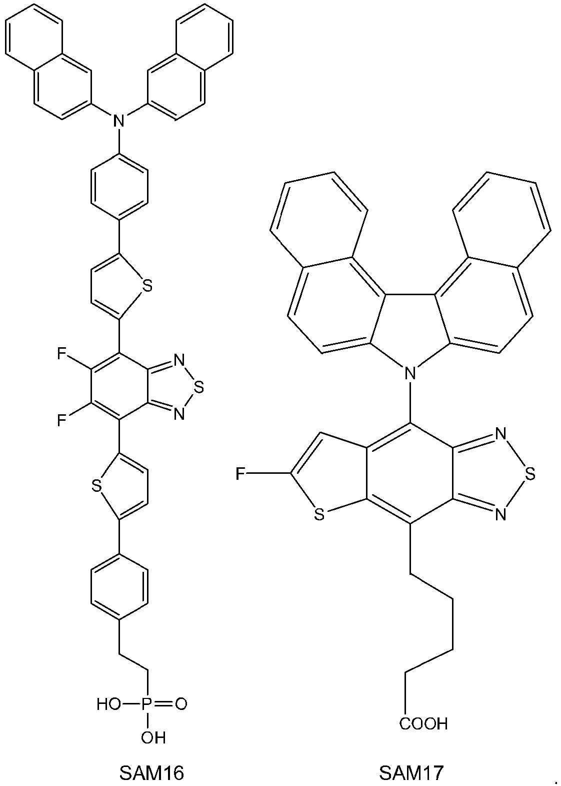

According to a second aspect of this application, an application of the organic compound according to the first aspect as a passivation material or a hole transport material is provided.

According to a third aspect of this application, a solar cell is provided, where the solar cell includes the organic compound according to the first aspect.

In some embodiments, the solar cell satisfies any one of the following conditions (1) to (3):
(1) the solar cell includes a perovskite layer, where the perovskite layer includes the organic compound;
(2) the solar cell includes a perovskite layer and a hole transport layer arranged in a stacked manner, where at least one of the perovskite layer and the hole transport layer includes the organic compound; and
(3) the solar cell includes a perovskite layer and a hole transport layer arranged in a stacked manner, and a passivation layer disposed on at least one side surface of the hole transport layer, where at least one of the perovskite layer, the hole transport layer, and the passivation layer includes the organic compound; and
optionally, a passivation layer is disposed between the perovskite layer and the hole transport layer.

In some embodiments, the solar cell satisfies at least one of the following conditions (1) to (3):
(1) the passivation layer includes the organic compound, where a mass percentage of the organic compound in the passivation layer is K1, and 0 < K1 ≤ 100%;
(2) the hole transport layer includes the organic compound, where a mass percentage of the organic compound in the hole transport layer is K2, and 0 < K2 ≤ 100%; and
(3) the perovskite layer includes the organic compound, where a mass percentage of the organic compound in the perovskite layer is K3, and 0.01% < K3 ≤ 0.5%.

According to a fourth aspect of this application, a photovoltaic module is provided and includes the solar cell according to the third aspect.

According to a fifth aspect of this application, a photovoltaic system is provided and includes the photovoltaic module according to the fourth aspect.

According to a sixth aspect of this application, an electric apparatus is further provided and includes at least one of the solar cell according to the third aspect and the photovoltaic module according to the fourth aspect.

### BRIEF DESCRIPTION OF DRAWINGS

To more clearly illustrate the technical solutions of the embodiments of this application, the drawings required for describing the embodiments of this application are briefly described below. Apparently, the drawings described below are merely some embodiments of this application, and those of ordinary skill in the art can obtain other drawings based on these drawings without creative effort. In the drawings:
FIG. 1 is a schematic diagram of a solar cell according to an embodiment of this application; and
FIG. 2 is a schematic diagram of a solar cell according to another embodiment of this application.

### Description of reference signs:

10. perovskite solar cell; 11. first electrode; 12. hole transport layer; 13. passivation layer; 14. perovskite layer; 15. electron transport layer; 16. hole blocking layer; 17. second electrode;
20. perovskite solar cell; 21. first electrode; 22. hole transport layer; 23. perovskite layer; 24. electron transport layer; 25. hole blocking layer; and 26. second electrode.

### DESCRIPTION OF EMBODIMENTS

The technical solutions of this application are described in detail below in conjunction with the drawings. The following embodiments are merely intended for a clearer description of the technical solutions of this application and therefore are used as just examples which do not constitute any limitations on the protection scope of this application.

Unless otherwise defined, all technical and scientific terms used herein have the same meanings as commonly understood by those skilled in the technical field of this application. The terms used herein are for the purpose of describing specific embodiments only and are not intended to limit this application. The terms "include", "have", and any variations thereof in the description, claims, and drawings of this application are intended to cover non-exclusive inclusion.

In the description of the embodiments of this application, the technical terms "first", "second", and the like are used only to distinguish different objects and should not be understood as indicating or implying relative importance or implicitly indicating the number, specific order, or hierarchical relationship of the technical features indicated. In the description of the embodiments of this application, "multiple" means two or more, unless explicitly and specifically defined otherwise.

The term "embodiment" described herein means that specific features, structures, or characteristics in combination with the description of the embodiments may be incorporated in at least one embodiment of this application. The word "embodiment" appearing in various places in this specification does not necessarily refer to the same embodiment or an independent or alternative embodiment that is exclusive of other embodiments. Those skilled in the art explicitly and implicitly understand that the embodiments described herein can be combined with other embodiments.

In the description of the embodiments of this application, the term "multiple" refers to two or more (including two), similarly, "multiple groups" refers to two or more groups (including two groups), and "multiple pieces" refers to two or more pieces (including two pieces).

In this application, unless otherwise specified, "room temperature" generally refers to 4°C to 30°C, preferably 20 ± 5°C.

In this application, the term "alkyl group" refers to a group formed by the removal of one hydrogen atom from alkane. For example, a methyl group is formed by the removal of one hydrogen atom from methane. The term "alkylene group or alkylidene group" refers to a group formed by the removal of two hydrogen atoms from alkane. For example, a methylene group is formed by the removal of two hydrogen atoms from methane.

The term "chain alkyl group" refers to a group formed by the removal of one hydrogen atom from an alkane, where all carbon atoms of the alkane are connected by carbon-carbon single bonds and do not form a ring, with the remaining valence bonds bonded to hydrogen; and the group includes linear alkyl groups and branched alkyl groups.

In this application, the "chain alkyl group" may have 1 to 20 carbon atoms, including 1, 2, 3, 4, 5, 6, 7, 8, 9, or 10, referring to linear alkyl groups having 1 to 20 carbon atoms and branched alkyl groups having 3 to 20 carbon atoms. Non-limiting examples include groups formed by the removal of one hydrogen atom, including methane, ethane, n-propane, isopropane, n-butane, isobutane, 2-ethylbutane, 3,3-dimethylbutane, n-pentane, isopentane, neopentane, 1-methylpentane, 3-methylpentane, 2-ethylpentane, 4-methyl-2-pentane, n-hexane, 1-methylhexane, 2-ethylhexane, 2-butylhexane, n-heptane, 1-methylheptane, 2,2-dimethylheptane, 2-ethylheptane, n-octane, n-nonane, n-decane, and the like.

In this application, the "number of ring-forming atoms" refers to the number of atoms forming a ring, and when the ring is substituted by a substituent, the atoms included in the substituent are not included in the ring-forming atoms. The same applies to the "number of ring-forming atoms" described below unless otherwise specified. For example, a benzene ring has 6 ring-forming atoms, a naphthalene ring has 10 ring-forming atoms, and a thiophene ring has 5 ring-forming atoms.

The term "aromatic group" refers to a hydrocarbon group with aromaticity, including monocyclic aryl groups and fused-ring aryl groups. A fused-ring aryl group refers to a group formed by two or more single aromatic rings connected through two adjacent shared ring atoms, that is, a fused ring. Furthermore, the π-electrons of the aromatic group should satisfy the 4n+2 (Huckel rule).

The term "heteroaromatic group" refers to a group having at least one ring-forming atom being a heteroatom and having aromaticity. The heteroatom includes but is not limited to N, P, O, and S.

Non-limiting examples of the "aromatic group" in this application include benzene, naphthalene, anthracene, fluoranthene, phenanthrene, benzophenanthrene, perylene, tetracene, or fluorene. Non-limiting examples of the "heteroaromatic group" include pyridine, pyrimidine, pyrazine, triazine, imidazole, furan, thiophene, benzofuran, benzothiophene, indole, carbazole, pyrroloimidazole, pyrrolopyrrole, thienopyrrole, thienothiophene, furanopyrrole, furanofuran, thienofuran, benzisoxazole, benzisothiazole, benzimidazole, quinoline, isoquinoline, cinnoline, quinoxaline, phenanthridine, perimidine, quinazoline, quinazolinone, dibenzofuran, dibenzothiophene, carbazole, and the like. Non-limiting examples of the "aromatic amine group" include substituted or unsubstituted aniline, substituted or unsubstituted diphenylamine, or substituted or unsubstituted triphenylamine.

In this application, when a connection site is not specified for a group, it means that any connectable site in the group can serve as the connection site.

In this application, when a single bond connected to a substituent crosses a corresponding ring, it indicates that the substituent can be connected to any substitutable site on the ring. For example, in 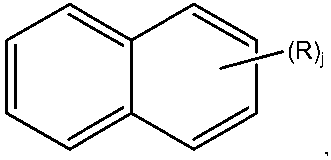 R is connected to any substitutable site on the naphthalene ring. When the same substituent R appears multiple times, it can be independently selected from different groups. For instance, a naphthalene ring has 6 substitutable sites, that is, j may be 6, and all R groups may be the same or different. When R is H, it indicates that no substituent is present, and 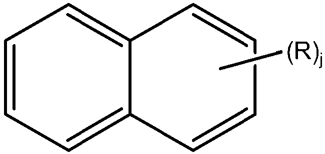 in this case is naphthalene.

In this application, "substituted or unsubstituted" means that the defined group may be substituted or unsubstituted. When the defined group is substituted, it should be understood as being optionally substituted by groups acceptable in the art, including but not limited to: C1 to C30 alkyl groups, heterocyclic groups containing 3 to 20 ring-forming atoms, aryl groups containing 5 to 20 ring-forming atoms, heteroaryl groups containing 5 to 20 ring-forming atoms, and halogens.

In this application, in a case that two groups are connected by a single connection point, for example, in 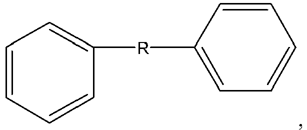 when R is selected as a single bond, it means that the two groups are directly connected by a single bond without requiring a specific group, that is, 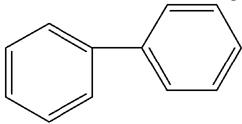 is formed.

In this application, when two cyclic structures in a schematic structural diagram share at least two ring-forming atoms, it indicates that the two cyclic structures are fused. If one of the cyclic structure groups is selected as H, it means that the cyclic structure does not exist. For example, in " 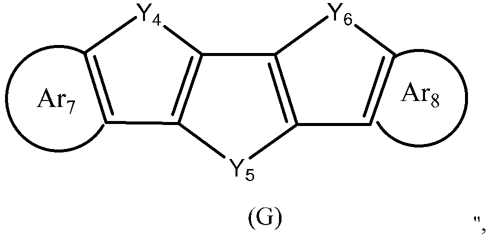 when Ar₇ or Ar₈ is selected as H, it means that Ar₇ or Ar₈ does not exist. If Ar₇ or Ar₈ is H, the structure is "

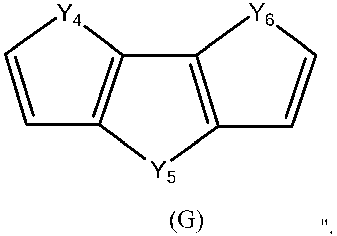

In an embodiment of this application, an organic compound is provided, where the organic compound is represented by formula (1): 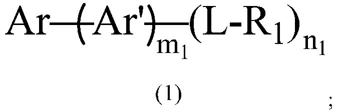
where Ar is selected from any one of a substituted or unsubstituted aromatic group having 6 to 50 ring-forming atoms, a substituted or unsubstituted heteroaromatic group having 5 to 50 ring-forming atoms, a group represented by formula (A), and a group represented by formula (B): 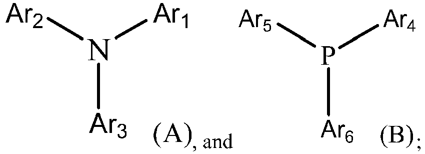
Ar' is selected from any one of a substituted or unsubstituted aryl group having 6 to 30 ring-forming atoms and a substituted or unsubstituted heteroaryl group having 5 to 30 ring-forming atoms;
Ar₁ to Ar₆ are each independently selected from any one of H, a substituted or unsubstituted alkyl group having 1 to 20 carbon atoms, a substituted or unsubstituted alkenyl group having 2 to 30 carbon atoms, and a substituted or unsubstituted aromatic group having 6 to 30 ring-forming atoms, where at least one of Ar₁ to Ar₃ is selected from a substituted or unsubstituted aromatic group having 6 to 30 ring-forming atoms, and at least one of Ar₄ to Ar₆ is selected from a substituted or unsubstituted aromatic group having 6 to 30 ring-forming atoms;
L is selected from a chain alkylene group having 1 to 10 carbon atoms; and
R₁ is an oxyacid group; n₁ is selected from any integer from 1 to 3; and m₁ is selected from any integer from 1 to 10;
or, the organic compound is an oxyanion salt of a compound represented by formula (1).

When the above organic compound is used for preparing a solar cell, the photoelectric conversion efficiency and stability of the solar cell can be improved. The organic compound enables an organic combination of Ar, Ar', L, and R₁ with specific group structures to form the compound of formula (1) or further form an oxyanion salt, where Ar is a terminal group, L is a linking group, and R₁ is a head group. When the organic compound is used for preparing the solar cell, the terminal group Ar with an aromatic group enables π-π interactions between molecules of the organic compound through the π-bonds of the aromatic group, inducing the formation of an ordered self-assembled molecular film. R₁ can bind with metal ions such as trivalent nickel, anchor the hole transport layer, or interact with A-site cations in the perovskite through hydrogen bonds, thereby passivating metal ions. Meanwhile, L reduces steric hindrance. Additionally, introducing a second aryl group Ar' between the terminal group Ar and the linking group L increases the dipole moment of the organic compound while minimally altering molecular energy levels of the organic compound, making the work function of the molecular film formed after self-assembly more compatible with the perovskite layer. When the organic compound is used for preparing the solar cell, the solar cell can exhibit high photoelectric conversion efficiency.

In some embodiments, Ar' at each occurrence is independently selected from any one of the following groups Ar'1 to Ar'7 or any combination thereof:

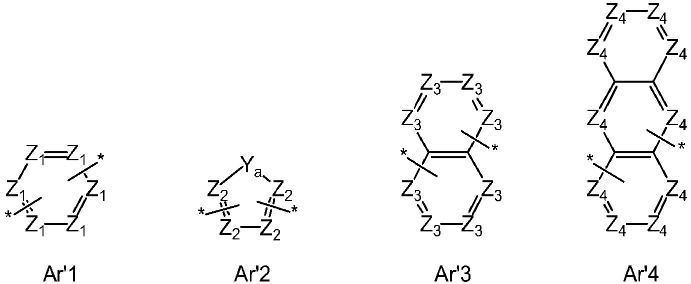

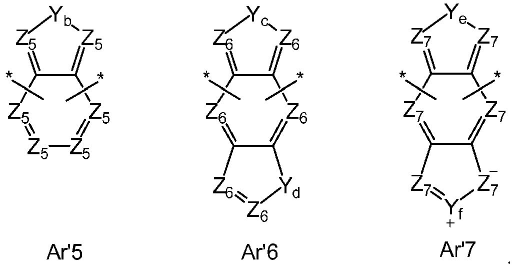

It is understood that the above "any combination thereof" refers to any combination of multiple groups connected by single bonds. Further, * represents a connection site.

Yₐ to Y_{f} are each independently selected from any one of -C(R₂R₃)-, -N(R₄)-, -O-, -Si(R₆R₇)-, -P(R₈)-, - S-, -As-, -Se-, -C(=O)-, -C(=S)-, -C(=NR₉)-, and -C(=CR₁₀)-.

"Y_{f}⁺" is a group formed by Y losing one electron.

Z₁ to Z₇ at each occurrence are each independently selected from -C(R₁₁)- or N.

"Z₇⁻" is a group formed by Z₇ gaining one electron.

R₂ to R₁₁ are each independently selected from H, a halogen group, a substituted or unsubstituted alkyl group having 1 to 5 carbon atoms, an alkoxy group having 1 to 5 carbon atoms, an aromatic group having 6 to 10 ring-forming atoms, a heteroaromatic group having 5 to 10 ring-forming atoms, -OC(=O)R₁₂, -NHC(=O)R₁₃, -N(R₁₄)₂, - L₁N⁺(R₁₅)₃X₁⁻, and -L₂P⁺(R₁₆)₃X₂⁻.

L₁ and L₂ are each independently selected from any one of a single bond and an alkylene group having 1 to 5 carbon atoms; R₁₂ to R₁₆ are each independently selected from H or an alkyl group having 1 to 5 carbon atoms, and R₁₂ and R₁₃ are not H; and X₁⁻ and X₂⁻ are each independently selected from a halogen ion.

* represents a connection site.

In some embodiments, Yₐ to Y_{f} at each occurrence are each independently selected from any one of - C(R₂R₃)-, -N(R₄)-, -O-, -Si(R₆R₇)-, -P(R₈)-, -C(=O)-, -C(=S)-, -C(=NR₉)-, and -C(=CR₁₀)-.

In some embodiments, Yₐ to Y_{f} at each occurrence are each independently selected from any one of - C(R₂R₃)-, -N(R₄)-, -O-, -Si(R₆R₇)-, -P(R₈)-, and -S-.

In some embodiments, Yₐ to Y_{f} at each occurrence are each independently selected from any one of - C(R₂R₃)-, -N(R₄)-, -O-, and -Si(R₆R₇)-.

In some embodiments, R₂ to R₁₀ are each independently selected from any one of H, a halogen group, a substituted or unsubstituted alkyl group having 1 to 5 carbon atoms, an alkoxy group having 1 to 5 carbon atoms, an aromatic group having 6 to 10 ring-forming atoms, and a heteroaromatic group having 5 to 10 ring-forming atoms.

In some embodiments, R₂ to R₁₀ are each independently selected from any one of H, F, Cl, an alkyl group having 1 to 5 carbon atoms, a halogen-substituted alkyl group having 1 to 5 carbon atoms, an alkoxy group having 1 to 5 carbon atoms, an aromatic group having 6 to 10 ring-forming atoms, and a heteroaromatic group having 5 to 10 ring-forming atoms.

In some embodiments, R₂ to R₁₀ are each independently selected from any one of H, a halogen group, an alkyl group having 1 to 5 carbon atoms, a halogen-substituted alkyl group having 1 to 5 carbon atoms, and an alkoxy group having 1 to 5 carbon atoms.

In some embodiments, R₂ to R₁₀ are each independently selected from any one of H, a halogen group, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, and an alkoxy group having 1 to 5 carbon atoms.

In some embodiments, R₂ to R₁₀ are each independently selected from any one of H, a halogen group, a linear alkyl group having 1 to 3 carbon atoms, a halogen-substituted linear alkyl group having 1 to 3 carbon atoms, and an alkoxy group having 1 to 5 carbon atoms.

In some embodiments, R₁₁ is selected from any one of H, a halogen group, an alkyl group having 1 to 5 carbon atoms, a halogen-substituted alkyl group having 1 to 5 carbon atoms, an aromatic group having 6 to 10 ring-forming atoms, and a heteroaromatic group having 5 to 10 ring-forming atoms.

In some embodiments, R₁₁ is selected from any one of H, F, Cl, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 10 ring-forming atoms, and a heteroaromatic group having 5 to 10 ring-forming atoms.

In some embodiments, R₁₁ is selected from any one of H, F, Cl, a linear alkyl group having 1 to 3 carbon atoms, a halogen-substituted linear alkyl group having 1 to 3 carbon atoms, an aromatic group having 6 to 8 ring-forming atoms, and a heteroaromatic group having 5 to 8 ring-forming atoms.

In some embodiments, R₁₂ to R₁₆ are each independently selected from H or a chain alkyl group having 1 to 5 carbon atoms.

In some embodiments, R₁₂ to R₁₆ are each independently selected from H or a chain alkyl group having 1 to 3 carbon atoms.

In some embodiments, R₁₂ to R₁₆ are each independently selected from H or a linear alkyl group having 1 to 3 carbon atoms.

In some embodiments, Ar' at each occurrence is independently selected from any one of the following groups or any combination thereof: 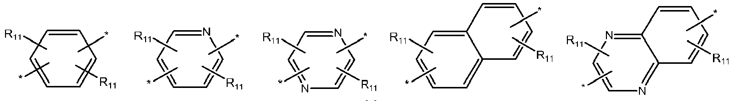 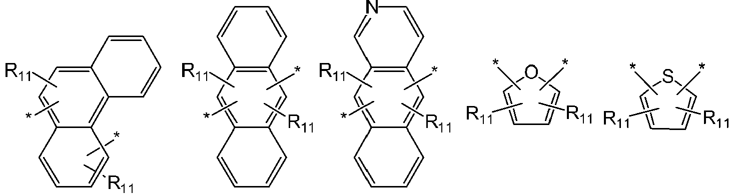 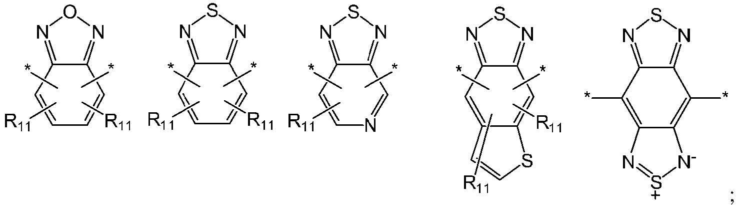 where * represents a connection site.

When Ar' is selected from a combination of multiple groups as described above, each group may appear once or multiple times, and the groups may be connected by single bonds or through fusion; further, the groups may be connected by forming carbon-carbon single bonds or through fusion of their respective ring-forming atoms; even further, the groups may be connected by forming carbon-carbon double bonds through the carbon atoms of the ring-forming atoms or through fusion of the carbon atoms of their respective ring-forming atoms.

In some embodiments, non-limiting examples of combinations of multiple groups as described above include but are not limited to the following structures:

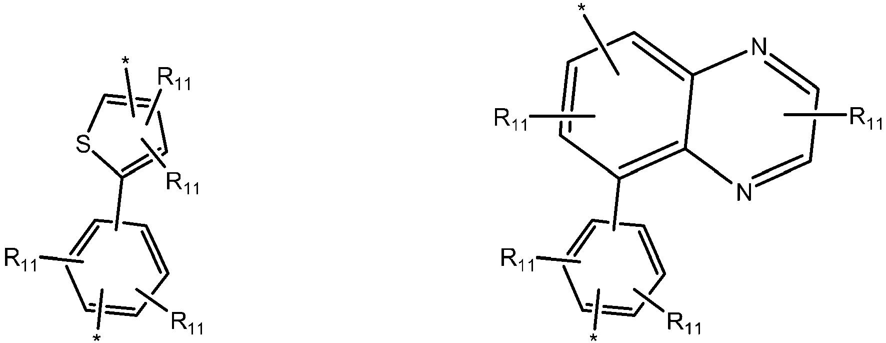

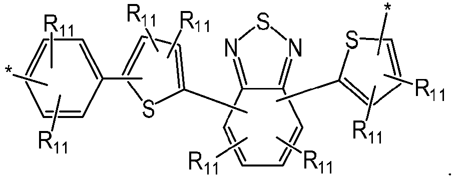

Ar is selected from any one of the groups formed by removing one hydrogen atom from structures represented by formulas (A) to (G): 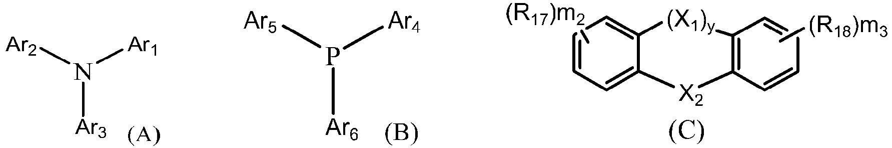 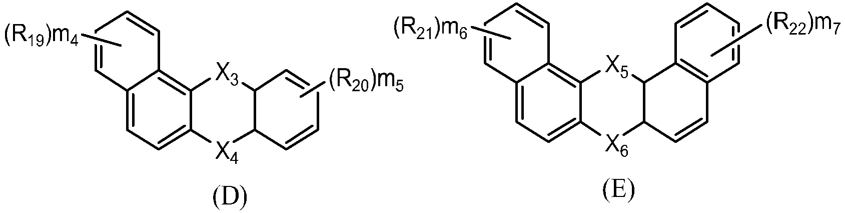 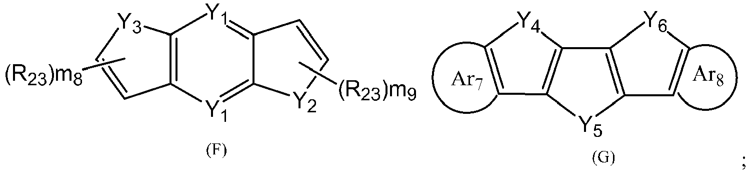
where X₁ to X₆ are each independently selected from any one of a single bond, C(R₂₄R₂₅), O, S, N, NR₂₆, C=O, or S=O, X₁ and X₂ are not both single bonds, X₃ and X₄ are not both single bonds, and X₅ and X₆ are not both single bonds; y is selected from any integer from 1 to 3; and when y is greater than or equal to 2, X₁ is selected from C(R₂₄R₂₅);
Y₁ at each occurrence is independently selected from CR₂₇ or N;
Y₂ to Y₆ are each independently selected from any one of C(R₂₈R₂₉), O, S, N, NR₃₀, C=O, or S=O;
R₁₇ to R₃₀ at each occurrence are each independently selected from any one of H, a halogen group, -N(R₃₁)₂, -CONR₃₂, -OCOR₃₃, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms;
R₃₁ to R₃₃ at each occurrence are each independently selected from any one of H, D, a substituted or unsubstituted alkyl group having 1 to 30 carbon atoms, a substituted or unsubstituted alkenyl group having 2 to 30 carbon atoms, a substituted or unsubstituted aromatic group having 6 to 30 ring-forming atoms, and a substituted or unsubstituted heteroaromatic group having 5 to 30 ring-forming atoms, and R₃₂ and R₃₃ are not H or D;
Ar₇ and Ar₈ are each independently selected from any one of H, a substituted or unsubstituted aromatic group having 6 to 30 ring-forming atoms, and a substituted or unsubstituted heteroaromatic group having 5 to 30 ring-forming atoms; and
m₂, m₃, and m₅ are each independently selected from any integer from 1 to 4; m₄, m₆, and m₇ are each independently selected from any integer from 1 to 6; and m₈ and m₉ are each independently selected from any integer from 1 to 2.

In some embodiments, m₂, m₃, and m₅ are each independently selected from 1, 2, 3, or 4.

In some embodiments, m₄, m₆, and m₇ are each independently selected from any integer of 1, 2, 3, 4, 5, or 6.

In some embodiments, m₈ and m₉ are each independently selected from 1 or 2.

In some embodiments, in formula (C), when X₁ is a single bond and X₂ is selected from NR₂₆, at least one R₁₇ or at least one R₁₈ is not H.

In some embodiments, X₁ to X₆ are each independently selected from any one of a single bond, C(R₂₄R₂₅), O, S, N, and NR₂₆.

In some embodiments, X₁ is selected from any one of a single bond, C(R₂₄R₂₅), O, S, and N.

Y₂ to Y₆ are each independently selected from any one of C(R₂₈R₂₉), O, S, N, and NR₃₀.

In some embodiments, R₁₇ and R₁₈ at each occurrence are each independently selected from any one of H, a halogen group, -N(R₂₅)₂, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms.

In some embodiments, R₁₇ and R₁₈ at each occurrence are each independently selected from any one of H, a halogen group, -N(R₂₅)₂, an alkyl group having 1 to 20 carbon atoms, a halogen-substituted alkyl group having 1 to 20 carbon atoms, an aromatic group having 6 to 20 ring-forming atoms, a halogen-substituted aromatic group having 6 to 20 ring-forming atoms, a heteroaromatic group having 5 to 20 ring-forming atoms, a halogen-substituted heteroaromatic group having 5 to 20 ring-forming atoms, and a heteroaromatic group having 5 to 20 ring-forming atoms and substituted by an alkyl group having 1 to 5 carbon atoms.

In some embodiments, R₁₇ and R₁₈ at each occurrence are each independently selected from any one of H, a halogen group, -N(R₂₅)₂, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, a heteroaromatic group having 5 to 10 ring-forming atoms, a halogen-substituted heteroaromatic group having 5 to 10 ring-forming atoms, and a heteroaromatic group having 5 to 10 ring-forming atoms and substituted by an alkyl group having 1 to 3 carbon atoms.

In some embodiments, R₁₇ and R₁₈ at each occurrence are each independently selected from any one of H, a halogen group, -N(R₂₅)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 10 ring-forming atoms, a halogen-substituted aromatic group having 6 to 10 ring-forming atoms, a heteroaromatic group having 5 to 10 ring-forming atoms, a halogen-substituted heteroaromatic group having 5 to 10 ring-forming atoms, and a heteroaromatic group having 5 to 7 ring-forming atoms and substituted by an alkyl group having 1 to 3 carbon atoms.

In some embodiments, R₁₉ and R₂₀ at each occurrence are each independently selected from any one of H, a halogen group, -N(R₂₅)₂, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms.

In some embodiments, R₁₉ and R₂₀ at each occurrence are each independently selected from any one of H, a halogen group, -N(R₂₅)₂, an alkyl group having 1 to 20 carbon atoms, a halogen-substituted alkyl group having 1 to 20 carbon atoms, an aromatic group having 6 to 20 ring-forming atoms, a halogen-substituted aromatic group having 6 to 20 ring-forming atoms, a heteroaromatic group having 5 to 20 ring-forming atoms, and a halogen-substituted heteroaromatic group having 5 to 20 ring-forming atoms.

In some embodiments, R₁₉ and R₂₀ at each occurrence are independently selected from any one of H, a halogen group, -N(R₂₅)₂, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, a heteroaromatic group having 5 to 10 ring-forming atoms, and a halogen-substituted heteroaromatic group having 5 to 10 ring-forming atoms.

In some embodiments, R₁₉ and R₂₀ at each occurrence are each independently selected from any one of H, a halogen group, -N(R₂₅)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 10 ring-forming atoms, a halogen-substituted aromatic group having 6 to 10 ring-forming atoms, a heteroaromatic group having 5 to 10 ring-forming atoms, and a halogen-substituted heteroaromatic group having 5 to 10 ring-forming atoms.

In some embodiments, R₂₁ and R₂₂ at each occurrence are each independently selected from any one of H, a halogen group, -N(R₂₅)₂, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms.

In some embodiments, R₂₁ and R₂₂ at each occurrence are each independently selected from any one of H, a halogen group, -N(R₂₅)₂, an alkyl group having 1 to 20 carbon atoms, a halogen-substituted alkyl group having 1 to 20 carbon atoms, an aromatic group having 6 to 20 ring-forming atoms, a halogen-substituted aromatic group having 6 to 20 ring-forming atoms, a heteroaromatic group having 5 to 20 ring-forming atoms, and a halogen-substituted heteroaromatic group having 5 to 20 ring-forming atoms.

In some embodiments, R₂₁ and R₂₂ at each occurrence are each independently selected from any one of H, a halogen group, -N(R₂₅)₂, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, a heteroaromatic group having 5 to 10 ring-forming atoms, and a halogen-substituted heteroaromatic group having 5 to 10 ring-forming atoms.

In some embodiments, R₂₁ and R₂₂ at each occurrence are each independently selected from any one of H, a halogen group, -N(R₂₅)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 10 ring-forming atoms, a halogen-substituted aromatic group having 6 to 10 ring-forming atoms, a heteroaromatic group having 5 to 10 ring-forming atoms, and a halogen-substituted heteroaromatic group having 5 to 10 ring-forming atoms.

In some embodiments, R₂₃ at each occurrence is independently selected from any one of H, a halogen group, -N(R₂₅)₂, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms.

In some embodiments, R₂₃ at each occurrence is independently selected from any one of H, a halogen group, -N(R₂₅)₂, an alkyl group having 1 to 20 carbon atoms, a halogen-substituted alkyl group having 1 to 20 carbon atoms, an aromatic group having 6 to 20 ring-forming atoms, a halogen-substituted aromatic group having 6 to 20 ring-forming atoms, an aromatic group having 6 to 20 ring-forming atoms and substituted by an alkyl group having 1 to 3 carbon atoms, a heteroaromatic group having 5 to 20 ring-forming atoms, a halogen-substituted heteroaromatic group having 5 to 20 ring-forming atoms, and a heteroaromatic group having 2 to 20 ring-forming atoms and substituted by an alkyl group having 1 to 3 carbon atoms.

In some embodiments, R₂₃ at each occurrence is independently selected from any one of H, a halogen group, -N(R₂₅)₂, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, a halogen-substituted heteroaromatic group having 5 to 10 ring-forming atoms, and a heteroaromatic group having 6 to 10 ring-forming atoms and substituted by an alkyl group having 1 to 3 carbon atoms.

In some embodiments, R₂₃ at each occurrence is independently selected from any one of H, a halogen group, -N(R₂₅)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 10 ring-forming atoms, a halogen-substituted aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, a halogen-substituted heteroaromatic group having 5 to 10 ring-forming atoms, and a heteroaromatic group having 5 to 10 ring-forming atoms and substituted by an alkyl group having 1 to 3 carbon atoms.

In some embodiments, R₂₄ and R₂₅ at each occurrence are independently selected from any one of H, a halogen group, -N(R₂₅)₂, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms.

In some embodiments, R₂₄ and R₂₅ at each occurrence are each independently selected from any one of H, a halogen group, -N(R₂₅)₂, an alkyl group having 1 to 20 carbon atoms, a halogen-substituted alkyl group having 1 to 20 carbon atoms, an aromatic group having 6 to 20 ring-forming atoms, a halogen-substituted aromatic group having 6 to 20 ring-forming atoms, a heteroaromatic group having 5 to 20 ring-forming atoms, and a halogen-substituted heteroaromatic group having 5 to 20 ring-forming atoms.

In some embodiments, R₂₄ and R₂₅ at each occurrence are each independently selected from any one of H, a halogen group, -N(R₂₅)₂, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, a halogen-substituted heteroaromatic group having 5 to 10 ring-forming atoms, and a heteroaromatic group having 5 to 10 ring-forming atoms and substituted by an alkyl group having 1 to 3 carbon atoms.

In some embodiments, R₂₄ and R₂₅ at each occurrence are each independently selected from any one of H, a halogen group, -N(R₂₅)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 10 ring-forming atoms, a halogen-substituted aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, a halogen-substituted heteroaromatic group having 5 to 10 ring-forming atoms, and a heteroaromatic group having 5 to 10 ring-forming atoms and substituted by an alkyl group having 1 to 3 carbon atoms.

In some embodiments, R₂₆ and R₂₇ at each occurrence are each independently selected from any one of H, a halogen group, -N(R₂₅)₂, an alkyl group having 1 to 20 carbon atoms, a halogen-substituted alkyl group having 1 to 20 carbon atoms, an aromatic group having 6 to 20 ring-forming atoms, a halogen-substituted aromatic group having 6 to 20 ring-forming atoms, an aromatic group having 6 to 20 ring-forming atoms and substituted by an alkyl group having 1 to 3 carbon atoms, a heteroaromatic group having 5 to 20 ring-forming atoms, a halogen-substituted heteroaromatic group having 5 to 20 ring-forming atoms, and a heteroaromatic group having 5 to 20 ring-forming atoms and substituted by an alkyl group having 1 to 3 carbon atoms.

In some embodiments, R₂₆ and R₂₇ at each occurrence are each independently selected from any one of H, a halogen group, -N(R₂₅)₂, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, a halogen-substituted heteroaromatic group having 5 to 10 ring-forming atoms, and a heteroaromatic group having 5 to 10 ring-forming atoms and substituted by an alkyl group having 1 to 3 carbon atoms.

In some embodiments, R₂₆ and R₂₇ at each occurrence are each independently selected from any one of H, a halogen group, -N(R₂₅)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 10 ring-forming atoms, a halogen-substituted aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, a halogen-substituted heteroaromatic group having 5 to 10 ring-forming atoms, and a heteroaromatic group having 5 to 10 ring-forming atoms and substituted by an alkyl group having 1 to 3 carbon atoms.

In some embodiments, R₂₈ to R₃₀ at each occurrence are each independently selected from any one of H, D, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₂₈ to R₃₀ at each occurrence are each independently selected from any one of H, D, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₂₈ to R₃₀ at each occurrence are each independently selected from any one of H, D, an alkyl group having 1 to 10 carbon atoms, a halogen-substituted alkyl group having 1 to 10 carbon atoms, a halogen-substituted aromatic group having 6 to 10 ring-forming atoms, an aromatic group having 6 to 10 ring-forming atoms and substituted by an alkyl group having 1 to 3 carbon atoms, an aromatic group having 6 to 10 ring-forming atoms, a halogen-substituted heteroaromatic group having 5 to 10 ring-forming atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₂₈ to R₃₀ at each occurrence are each independently selected from any one of H, D, a chain alkyl group having 1 to 5 carbon atoms, a halogen-substituted chain alkyl group having 1 to 5 carbon atoms, a halogen-substituted aromatic group having 6 to 10 ring-forming atoms, an aromatic group having 6 to 10 ring-forming atoms and substituted by an alkyl group having 1 to 3 carbon atoms, an aromatic group having 6 to 10 ring-forming atoms, a halogen-substituted heteroaromatic group having 5 to 10 ring-forming atoms, a heteroaromatic group having 5 to 10 ring-forming atoms and substituted by an alkyl group having 1 to 3 carbon atoms, and a heteroaromatic group having 5 to 10 ring-forming atoms.

In some embodiments, R₂₈ to R₃₀ at each occurrence are each independently selected from any one of H, D, a linear alkyl group having 1 to 5 carbon atoms, a branched alkyl group having 3 to 5 carbon atoms, a halogen-substituted linear alkyl group having 1 to 5 carbon atoms, a halogen-substituted branched alkyl group having 3 to 5 carbon atoms, a halogen-substituted aromatic group having 6 to 10 ring-forming atoms, an aromatic group having 6 to 10 ring-forming atoms and substituted by an alkyl group having 1 to 3 carbon atoms, an aromatic group having 6 to 10 ring-forming atoms, a halogen-substituted heteroaromatic group having 5 to 10 ring-forming atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₃₁ to R₃₃ at each occurrence are each independently selected from any one of H, D, a substituted or unsubstituted alkyl group having 1 to 15 carbon atoms, a substituted or unsubstituted alkenyl group having 2 to 15 carbon atoms, a substituted or unsubstituted aromatic group having 6 to 15 ring-forming atoms, and a substituted or unsubstituted heteroaromatic group having 5 to 15 ring-forming atoms.

In some embodiments, R₃₁ to R₃₃ at each occurrence are each independently selected from any one of H, D, a substituted or unsubstituted alkyl group having 1 to 10 carbon atoms, a substituted or unsubstituted alkenyl group having 2 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₃₁ to R₃₃ at each occurrence are each independently selected from any one of H, D, a substituted or unsubstituted alkyl group having 1 to 5 carbon atoms, a substituted or unsubstituted alkenyl group having 2 to 5 carbon atoms, a substituted or unsubstituted aromatic group having 6 to 10 ring-forming atoms, and a substituted or unsubstituted heteroaromatic group having 5 to 10 ring-forming atoms.

In some embodiments, R₃₁ to R₃₃ at each occurrence are each independently selected from any one of H, D, a linear alkyl group having 1 to 5 carbon atoms, a branched alkyl group having 3 to 5 carbon atoms, a halogen-substituted linear alkyl group having 1 to 5 carbon atoms, a halogen-substituted branched alkyl group having 3 to 5 carbon atoms, a halogen-substituted aromatic group having 6 to 10 ring-forming atoms, an aromatic group having 6 to 10 ring-forming atoms and substituted by an alkyl group having 1 to 3 carbon atoms, an aromatic group having 6 to 10 ring-forming atoms, a halogen-substituted heteroaromatic group having 5 to 10 ring-forming atoms, a heteroaromatic group having 5 to 10 ring-forming atoms, and a heteroaromatic group having 5 to 10 ring-forming atoms and substituted by an alkyl group having 1 to 3 carbon atoms.

Ar₇ and Ar₈ are each independently selected from any one of H, a substituted or unsubstituted aromatic group having 10 ring-forming atoms, and a substituted or unsubstituted heteroaromatic group having 5 to 10 ring-forming atoms.

In some embodiments, Ar₇ and Ar₈ are each independently selected from H or any one of the following structures: 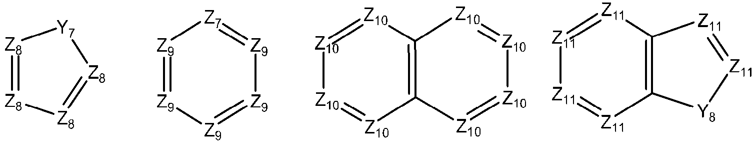 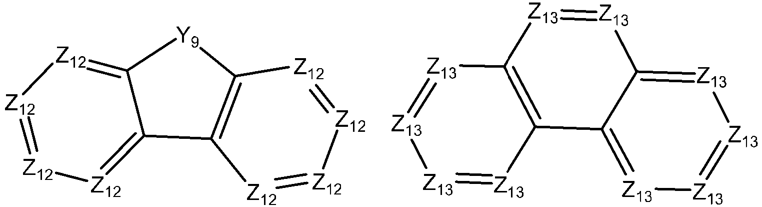 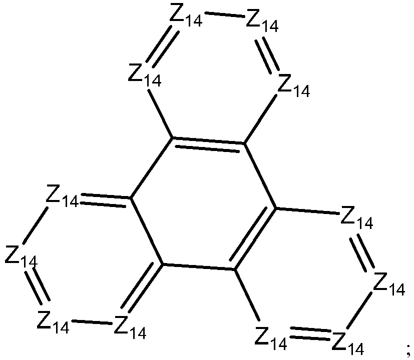
where Y₇ to Y₉ are selected from any one of CR₃₄R₃₅, O, S, S=O, and C=O; and
Z₈ to Z₁₄ at each occurrence are each independently selected from CR₃₆ or N, and Z₈ to Z₁₄ in a same structural formula are not all N.

In the same structural formula, Z₈ to Z₁₄ are not all N, meaning that in the same structural formula, groups Z₈ are not all N, groups Z₉ are not all N, groups Z₁₀ are not all N, groups Z₁₁ are not all N, groups Z₁₂ are not all N, groups Z₁₃ are not all N, and groups Z₁₄ are not all N. R₃₄ to R₃₆ at each occurrence are each independently selected from any one of H, D, a substituted or unsubstituted linear alkyl group having 1 to 10 carbon atoms, a substituted or unsubstituted branched alkyl group having 3 to 20 carbon atoms, and a substituted or unsubstituted cycloalkyl group having 3 to 20 carbon atoms.

In some embodiments, Ar₇ and Ar₈ are each independently selected from H or any one of the following structures:

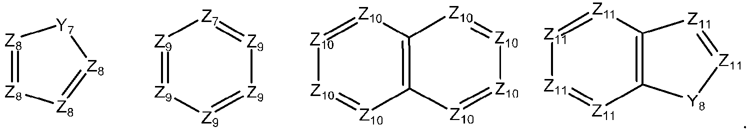

In some embodiments, Y₇ to Y₉ are each independently selected from any one of CR₃₄R₃₅, O, and S.

In some embodiments, Y₇ to Y₉ are each independently selected from any one of CR₃₄R₃₅ and O.

In some embodiments, R₃₄ to R₃₆ at each occurrence are each independently selected from any one of H, D, a linear alkyl group having 1 to 10 carbon atoms, a halogen-substituted linear alkyl group having 1 to 10 carbon atoms, a branched alkyl group having 3 to 10 carbon atoms, and a halogen-substituted branched alkyl group having 3 to 10 carbon atoms.

In some embodiments, R₃₄ to R₃₆ at each occurrence are each independently selected from any one of H, D, a linear alkyl group having 1 to 5 carbon atoms, a halogen-substituted linear alkyl group having 1 to 5 carbon atoms, a branched alkyl group having 3 to 5 carbon atoms, and a halogen-substituted branched alkyl group having 3 to 5 carbon atoms.

In some embodiments, Ar₇ and Ar₈ are each independently selected from any one of H and a phenyl group.

In some embodiments, Ar is selected from any one of the following groups: 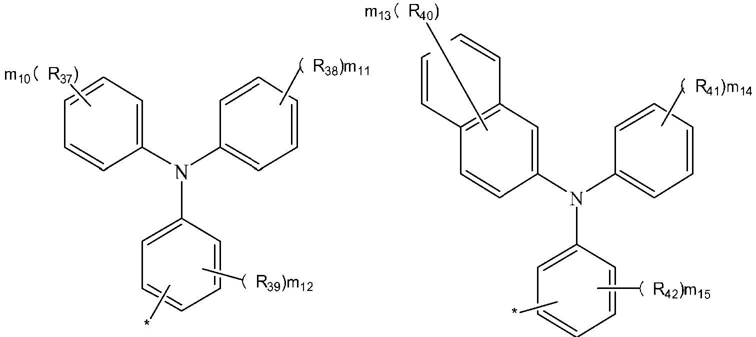 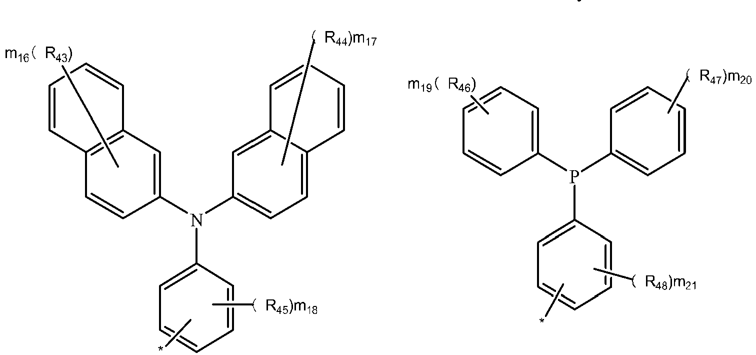 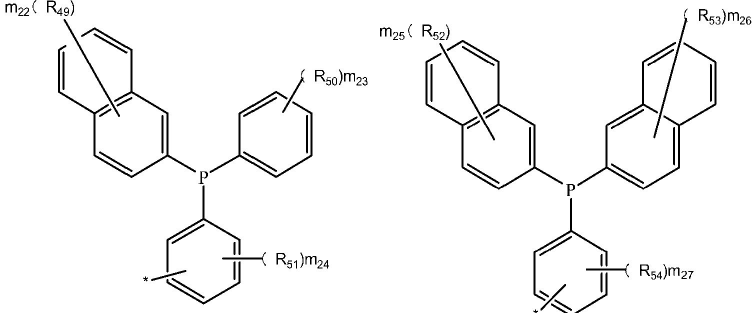 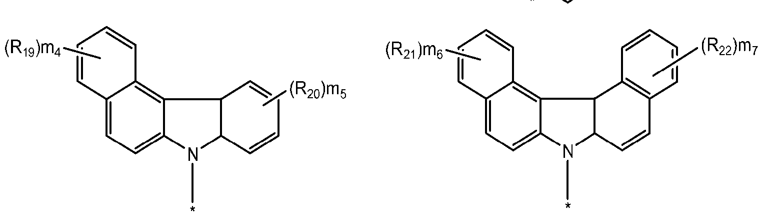 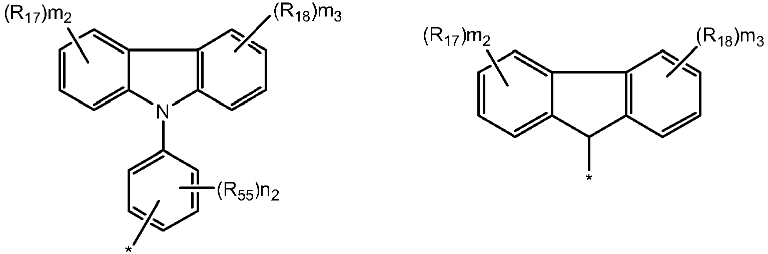 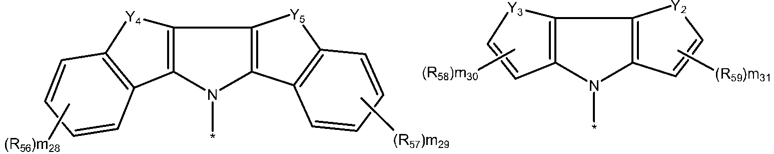 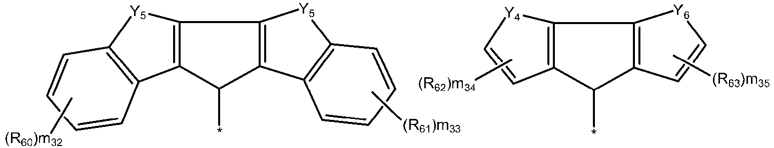 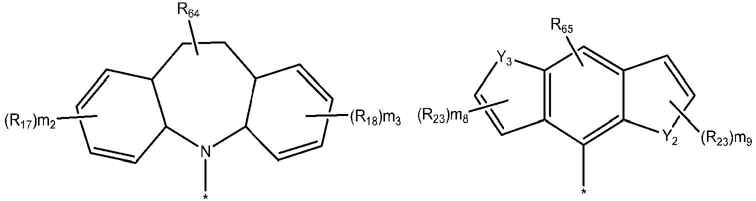 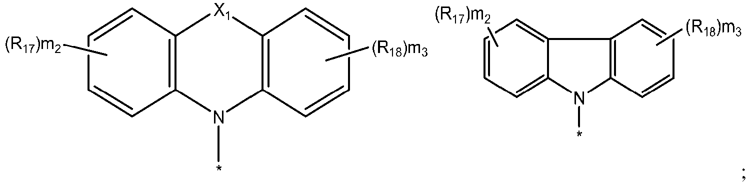 where R₃₇ to R₆₅ at each occurrence are each independently selected from any one of H, a halogen group, -N(R₆₆)₂, -CONR₆₇, -OCOR₆₈, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms.

R₆₆ to R₆₈ are each independently selected from any one of an alkyl group having 1 to 15 carbon atoms, a halogen-substituted alkyl group having 1 to 15 carbon atoms, an aromatic group having 6 to 30 ring-forming atoms, a halogen-substituted aromatic group having 6 to 30 ring-forming atoms, and a heteroaromatic group having 5 to 30 ring-forming atoms.

In some embodiments, R₁₇ to R₃₀ and R₃₇ to R₆₅ at each occurrence are each independently selected from any one of H, a halogen group, an alkyl group having 1 to 15 carbon atoms, a halogen-substituted alkyl group having 1 to 15 carbon atoms, an aromatic group having 6 to 30 ring-forming atoms, a halogen-substituted aromatic group having 6 to 30 ring-forming atoms, and a heteroaromatic group having 5 to 30 ring-forming atoms.

Optionally, R₁₇ to R₃₀ and R₃₇ to R₆₅ at each occurrence are each independently selected from any one of H, a halogen group, an alkyl group having 1 to 10 carbon atoms, a halogen-substituted alkyl group having 1 to 10 carbon atoms, an aromatic group having 6 to 15 ring-forming atoms, a halogen-substituted aromatic group having 6 to 15 ring-forming atoms, an aromatic group having 6 to 15 ring-forming atoms and substituted by an alkyl group having 1 to 3 carbon atoms, a heteroaromatic group having 5 to 15 ring-forming atoms, and an aromatic group having 5 to 15 ring-forming atoms and substituted by an alkyl group having 1 to 3 carbon atoms.

Optionally, R₁₇ to R₃₀ and R₃₇ to R₆₅ at each occurrence are each independently selected from any one of H, a halogen group, a chain alkyl group having 1 to 5 carbon atoms, a halogen-substituted chain hydrocarbon group having 1 to 5 carbon atoms, an aromatic group having 6 to 10 ring-forming atoms, a halogen-substituted aromatic group having 6 to 10 ring-forming atoms, an aromatic group having 6 to 10 ring-forming atoms and substituted by an alkyl group having 1 to 3 carbon atoms, a heteroaromatic group having 1 to 10 ring-forming atoms, and an aromatic group having 5 to 10 ring-forming atoms and substituted by an alkyl group having 1 to 3 carbon atoms.

In some embodiments, R₃₇ to R₃₉ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₃₇ to R₃₉ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₃₇ to R₃₉ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₃₇ to R₃₉ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, and a substituted or unsubstituted heteroaromatic group having 5 to 10 ring-forming atoms.

In some embodiments, R₃₇ to R₃₉ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, and a substituted or unsubstituted heteroaromatic group having 5 to 8 ring-forming atoms.

In some embodiments, R₄₀ to R₄₂ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₄₀ to R₄₂ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 15 ring-forming atoms, and a substituted or unsubstituted heteroaromatic group having 5 to 15 ring-forming atoms.

In some embodiments, R₄₀ to R₄₂ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₄₀ to R₄₂ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, and a substituted or unsubstituted heteroaromatic group having 5 to 10 ring-forming atoms.

In some embodiments, R₄₀ to R₄₂ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₄₃ to R₄₅ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₄₃ to R₄₅ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₄₃ to R₄₅ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₄₃ to R₄₅ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, and a substituted or unsubstituted heteroaromatic group having 5 to 10 ring-forming atoms.

In some embodiments, R₄₃ to R₄₅ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₄₆ to R₄₈ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₄₆ to R₄₈ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₄₆ to R₄₈ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₄₆ to R₄₈ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, and a substituted or unsubstituted heteroaromatic group having 5 to 10 ring-forming atoms.

In some embodiments, R₄₆ to R₄₈ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₄₉ to R₅₁ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₄₉ to R₅₁ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₄₉ to R₅₁ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₄₉ to R₅₁ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₄₉ to R₅₁ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, and a substituted or unsubstituted heteroaromatic group having 5 to 8 ring-forming atoms.

In some embodiments, R₅₂ to R₅₅ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₅₂ to R₅₅ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₅₂ to R₅₅ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₅₂ to R₅₅ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₅₂ to R₅₅ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₅₆ and R₅₇ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₅₆ and R₅₇ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₅₆ and R₅₇ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₅₆ and R₅₇ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₅₆ and R₅₇ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₅₈ and R₅₉ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₅₈ and R₅₉ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₅₈ and R₅₉ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₅₈ and R₅₉ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₅₈ and R₅₉ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, and a substituted or unsubstituted heteroaromatic group having 5 to 8 ring-forming atoms.

In some embodiments, R₆₀ and R₆₁ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₆₀ and R₆₁ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₆₀ and R₆₁ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, an aromatic group having 6 to 0 ring-forming atoms and substituted by an alkyl group having 1 to 3 carbon atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₆₀ and R₆₁ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₆₀ and R₆₁ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₆₂ and R₆₃ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₆₂ and R₆₃ at each occurrence are each independently selected from any one of H, a halogen group, -N(R₆₆)₂, a substituted or unsubstituted alkyl group having 1 to 20 carbon atoms, a substituted or unsubstituted aromatic group having 6 to 20 ring-forming atoms, and a substituted or unsubstituted heteroaromatic group having 5 to 20 ring-forming atoms.

In some embodiments, R₆₂ and R₆₃ at each occurrence are each independently selected from any one of H, a halogen group, -N(R₆₆)₂, a substituted or unsubstituted alkyl group having 1 to 10 carbon atoms, a substituted or unsubstituted aromatic group having 6 to 10 ring-forming atoms, and a substituted or unsubstituted heteroaromatic group having 5 to 10 ring-forming atoms.

In some embodiments, R₆₂ and R₆₃ at each occurrence are each independently selected from any one of H, a halogen group, -N(R₆₆)₂, a chain alkyl group having 1 to 10 carbon atoms, a halogen-substituted chain alkyl group having 1 to 10 carbon atoms, an aromatic group having 6 to 10 ring-forming atoms, an aromatic group having 6 to 10 ring-forming atoms and substituted by an alkyl group having 1 to 3 carbon atoms, a heteroaromatic group having 5 to 10 ring-forming atoms, and a heteroaromatic group having 5 to 10 ring-forming atoms and substituted by an alkyl group having 1 to 3 carbon atoms.

In some embodiments, R₆₂ and R₆₃ at each occurrence are each independently selected from any one of H, a halogen group, -N(R₆₆)₂, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, an aromatic group having 6 to 8 ring-forming atoms and substituted by an alkyl group having 1 to 3 carbon atoms, a heteroaromatic group having 5 to 8 ring-forming atoms, and a heteroaromatic group having 5 to 8 ring-forming atoms and substituted by an alkyl group having 1 to 3 carbon atoms.

In some embodiments, R₆₄ to R₆₈ at each occurrence are each independently selected from any one of an alkyl group having 1 to 15 carbon atoms, a halogen-substituted alkyl group having 1 to 15 carbon atoms, an aromatic group having 6 to 15 ring-forming atoms, a halogen-substituted aromatic group having 6 to 15 ring-forming atoms, a heteroaromatic group having 5 to 15 ring-forming atoms, and a halogen-substituted heteroaromatic group having 5 to 15 ring-forming atoms.

Optionally, R₆₄ to R₆₈ at each occurrence are each independently selected from any one of H, D, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, a heteroaromatic group having 5 to 10 ring-forming atoms, and a halogen-substituted heteroaromatic group having 5 to 10 ring-forming atoms.

In some embodiments, R₆₄ to R₆₈ at each occurrence are each independently selected from any one of H, D, a linear alkyl group having 1 to 5 carbon atoms, a halogen-substituted linear alkyl group having 1 to 5 carbon atoms, a branched alkyl group having 3 to 5 carbon atoms, a halogen-substituted branched alkyl group having 3 to 5 carbon atoms, an aromatic group having 6 to 8 ring-forming atoms, a halogen-substituted aromatic group having 6 to 8 ring-forming atoms, a heteroaromatic group having 5 to 8 ring-forming atoms, and a halogen-substituted heteroaromatic group having 5 to 8 ring-forming atoms.

In some embodiments, m₁₀, m₁₁, m₁₄, m₁₉, m₂₀, and m₂₃ are each independently selected from 1, 2, 3, 4, or 5.

In some embodiments, m₁₃, m₁₆, m₁₇, m₂₂, m₂₅, and m₂₆ are each independently selected from 1, 2, 3, 4, 5, or 6.

In some embodiments, m₁₂, m₁₅, m₁₈, m₂₁, m₂₄, m₂₇ to m₂₉, m₃₂, m₃₃, and n₂ are each independently selected from 1, 2, 3, or 4.

In some embodiments, m₃₀, m₃₁, m₃₄, and m₃₅ are each independently selected from 1 or 2.

In some embodiments, R₁ at each occurrence is independently selected from any one of a phosphonic acid group, a sulfonic acid group, a carboxylic acid group, a sulfinic acid group, a boric acid group, or a silicic acid group.

Optionally, the phosphonic acid group includes at least one of an orthophosphonic acid group, a metaphosphonic acid group, and a pyrophosphonic acid group.

Optionally, R₁ at each occurrence is independently selected from any one of the following structures: 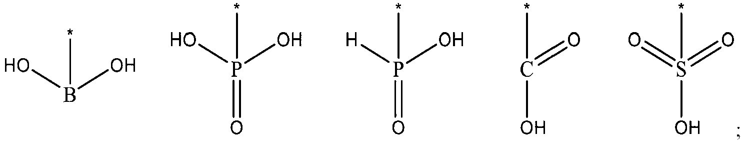 where * represents a connection site.

In some embodiments, the oxyanion salt of the compound represented by formula (1) includes an anion and a cation, where the anion is formed by the loss of H from at least one hydroxyl group in the oxyacid group of the compound represented by formula (1), and the cation is selected from a metal ion or NH₄⁺.

In some embodiments, the metal ion includes at least one of an alkali metal ion, a calcium ion, a magnesium ion, an iron ion, a copper ion, a zinc ion, and an aluminum ion.

In some embodiments, L at each occurrence is independently selected from a chain alkylene group having 1 to 10 carbon atoms.

In some embodiments, L at each occurrence is independently selected from a chain alkylene group having 2 to 8 carbon atoms.

In some embodiments, L at each occurrence is independently selected from a linear alkylene group having 2 to 10 carbon atoms or a branched alkylene group having 3 to 8 carbon atoms.

In some embodiments, L at each occurrence is independently selected from a chain alkylene group having 2 to 6 carbon atoms.

In some embodiments, L at each occurrence is independently selected from a chain alkylene group having 3 to 5 carbon atoms.

In some embodiments, L at each occurrence is independently selected from a linear alkylene group or a branched alkylene group having 3 to 5 carbon atoms.

In some embodiments, L at each occurrence is independently selected from any one of methyl, ethyl, propyl, isopropyl, n-butyl, tert-butyl, pentyl, hexyl, heptyl, octyl, nonyl, and decyl.

In some embodiments, the organic compound includes at least one of the compounds represented by formulas (SAM1) to (SAM17) and oxyanion salts of the compounds represented by formulas (SAM1) to (SAM17):

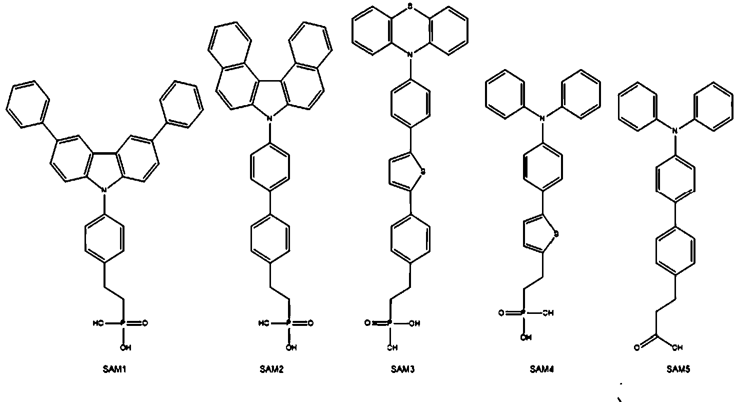

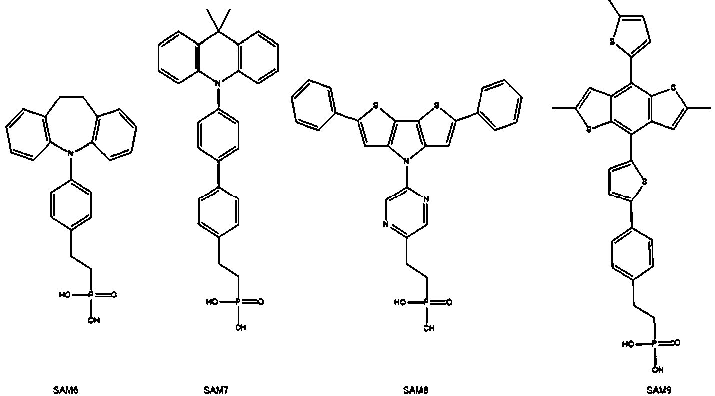

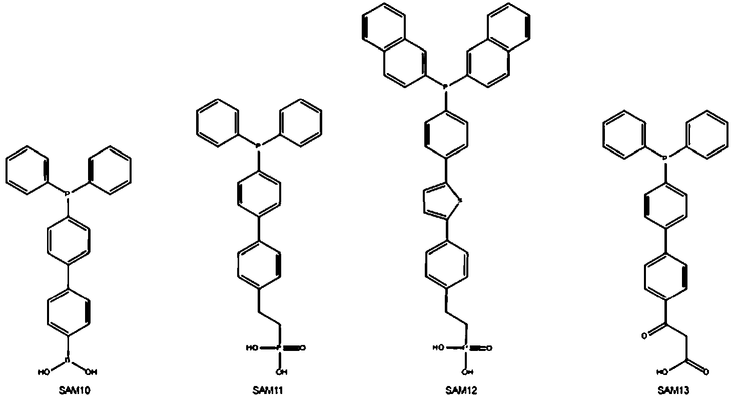

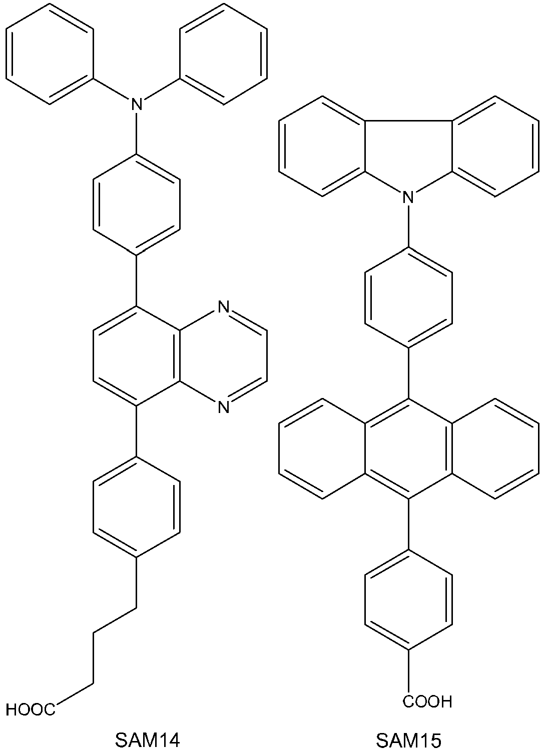

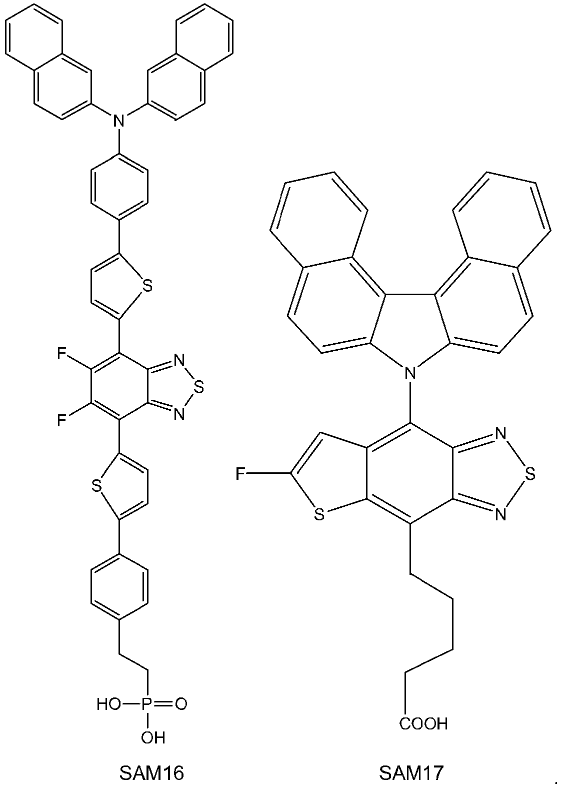

In some embodiments, the above organic compound can be prepared using common organic synthesis methods in the art. Here, a preparation method of a compound of formula (SAM1) is described as an example, including the following steps:
Compound 1 and compound 2 are subjected to a first substitution reaction to prepare compound 3, with a synthetic route as follows:

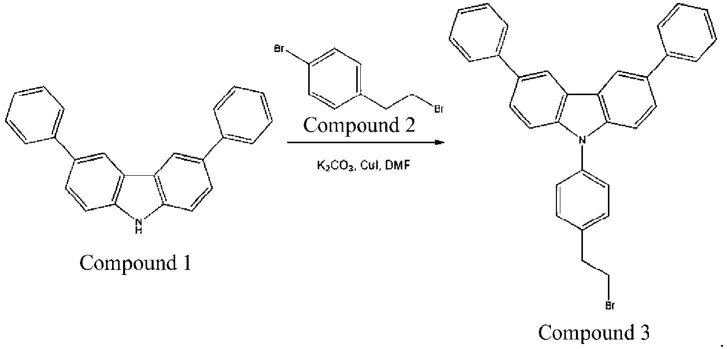

In some embodiments, the first substitution reaction is performed under the action of potassium carbonate (K₂CO₃) and copper(I) iodide (CuT).

In some embodiments, the first substitution reaction is performed at a temperature of 100°C to 145°C for a duration of 15 h to 25 h.

In some embodiments, the first substitution reaction is performed in N,N-dimethylformamide (MDF).

Compound 3 and triethyl phosphite (P(OEt)₃) are subjected to a second substitution reaction to prepare the compound of formula (SAM1), with a synthetic route as follows:

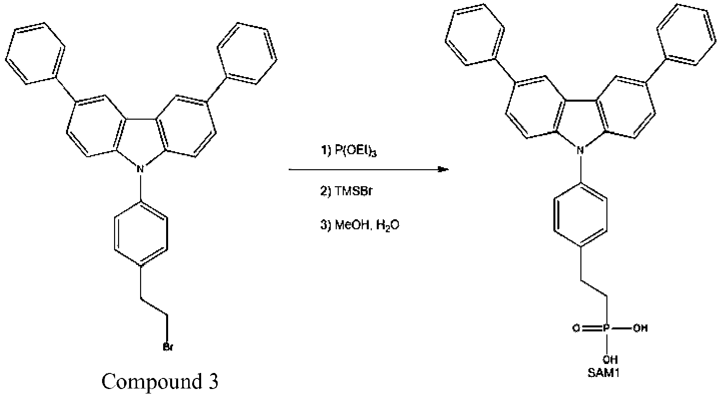

In some embodiments, the second substitution reaction is performed under the action of tributylbromosilane (TMSBr).

In some embodiments, the second substitution reaction is performed in methanol (MeOH).

In an embodiment of this application, an application of the above organic compound as a passivation material or a hole transport material is provided.

In an embodiment of this application, a solar cell is provided, where the solar cell includes the above organic compound.

The above organic compound can serve as both a passivation material and a hole transport material, capable of improving the photoelectric conversion efficiency of the solar cell.

In some embodiments, the solar cell includes a perovskite layer and a hole transport layer arranged in a stacked manner, and a passivation layer disposed on at least one side surface of the hole transport layer. In some embodiments, at least one of the perovskite layer, the hole transport layer, and the passivation layer includes the organic compound.

It can be understood that the hole transport layer has two opposite surfaces, with one surface closer to the perovskite layer and the other surface farther from the perovskite layer. The passivation layer may be disposed on at least one side surface. That is, any one of the following solutions is included:
the solar cell includes a perovskite layer, a hole transport layer, and a passivation layer arranged in a stacked manner; or
the solar cell includes a perovskite layer, a passivation layer, and a hole transport layer arranged in a stacked manner; or
the solar cell includes a perovskite layer, a passivation layer, a hole transport layer, and a passivation layer arranged in a stacked manner.

The above organic compound can be doped in the perovskite layer to provide a passivation effect, and can provide both a hole transport effect and a passivation effect in the hole transport layer or the passivation layer.

In some embodiments, a passivation layer is disposed between the perovskite layer and the hole transport layer.

It can be understood that the perovskite layer includes perovskite materials commonly used in the art.

In some embodiments, a chemical formula of the perovskite material satisfies ABX₃ or A₂CDX₆, where A is an inorganic cation, an organic cation, or a mixture of both, and may be at least one of formamidinium ion (FA), methylammonium ion (MA), and Cs ion; B is an inorganic metal cation, which may be at least one of Pb ion and Sn ion; C is a noble metal cation, commonly Ag⁺; D is a heavy metal or rare metal cation, which may be at least one of bismuth cation Bi³⁺, antimony cation Sb³⁺, and indium cation Tn³⁺; and X is oxygen or a halogen element, which may be at least one of O, Br, and I.

In some embodiments, the above perovskite layer has a bandgap of 1.20 eV to 2.30 eV and a thickness of 200 nm to 1000 nm.

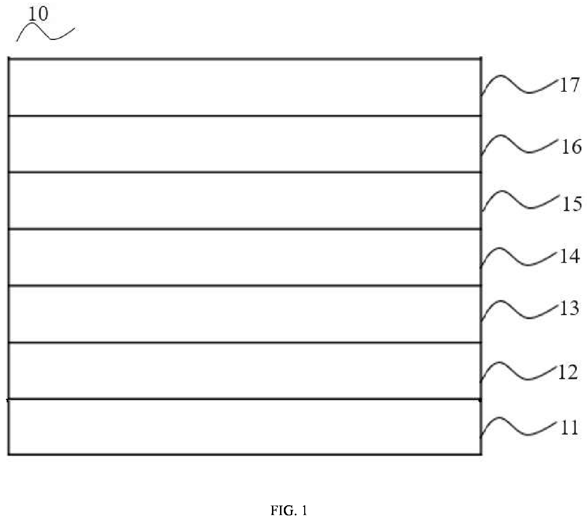

In some embodiments, referring to FIG. 1, a solar cell 10 is provided. The solar cell 10 includes a hole transport layer 12, a passivation layer 13, and a perovskite layer 14 arranged in a stacked manner. At least one of the passivation layer 13, the hole transport layer 12, and the perovskite layer 14 includes the above organic compound.

In some embodiments, a component of the passivation layer 13 includes the organic compound according to the first aspect, where a mass percentage of the organic compound in the passivation layer is K1, and 0 < K1 ≤ 100%.

Optionally, K1 may be 1wt% to 100wt%, for example, 10wt%, 20wt%, 30wt%, 40wt%, 50wt%, 60wt%, 70wt%, 80wt%, 90wt%, or 100wt%.

In some embodiments, when K1 is not 100%, the passivation layer may further include other passivation materials commonly used in the art, such as SAM18 and SAM19 as exemplified later. when K1 is 100%, it means that the material of the passivation layer is the above organic compound.

In an embodiment, a thickness of the passivation layer is 1 nm to 50 nm.

Optionally, the thickness of the passivation layer may be 1 nm, 5 nm, 10 nm, 15 nm, 20 nm, 25 nm, 30 nm, 35 nm, 40 nm, 45 nm, or 50 nm, or a range defined by any two of these values.

In some embodiments, when the passivation layer includes the above organic compound, a material of the hole transport layer may be at least one of various hole transport materials commonly used in the art and the above organic compound. In this case, a mass percentage of the above organic compound in the hole transport layer may be 0 to 100%; and optionally, the mass percentage of the above organic compound in the hole transport layer may be 0, 10%, 20%, 30%, 40%, 50%, 60%, 70%, 80%, 90%, or 100%.

Various hole transport materials commonly used in the art include but are not limited to at least one of the following materials and their derivatives: nickel oxide, zinc oxide, molybdenum oxide, 2,2',7,7'-tetrakis(N,N-dip-methoxyphenylamino)-9,9'-spirobifluorene (Spiro-OMeTAD), poly[bis(4-phenyl)(2,4,6-trimethylphenyl)amine] (PTAA), and [4-(3,6-dimethyl-9H-carbazol-9-yl)butyl]phosphonic acid (Me-4Pacz).

In some embodiments, when the passivation layer includes the above organic compound, a percentage of the above organic compound in the perovskite layer may be 0 to 0.5%; and optionally, the mass percentage of the above organic compound in the perovskite layer may be 0, 0.1%, 0.2%, 0.3%, 0.4%, or 0.5%.

In some embodiments, the hole transport layer includes the above organic compound, and a mass percentage of the organic compound in the hole transport layer is K2, and 0 < K2 ≤ 100%.

Optionally, K2 may be 1wt% to 100wt%, for example, 10wt%, 20wt%, 30wt%, 40wt%, 50wt%, 60wt%, 70wt%, 80wt%, 90wt%, or 100wt%, or a range defined by any two of these values.

In some embodiments, when K2 is not 100%, the hole transport layer may further include other hole transport layer materials commonly used in the art, such as at least one of nickel oxide, zinc oxide, molybdenum oxide, 2,2',7,7'-tetrakis(N,N-di-p-methoxyphenylamino)-9,9'-spirobifluorene (Spiro-OMeTAD), poly[bis(4-phenyl)(2,4,6-trimethylphenyl)amine] (PTAA), and [4-(3,6-dimethyl-9H-carbazol-9-yl)butyl]phosphonic acid (Me-4Pacz).

In some embodiments, when K2 is 100%, it means that the material of the hole transport layer is the above organic compound.

In an embodiment, a thickness of the hole transport layer is 1 nm to 50 nm.

Optionally, the thickness of the hole transport layer may be 1 nm, 5 nm, 10 nm, 15 nm, 20 nm, 25 nm, 30 nm, 35 nm, 40 nm, 45 nm, or 50 nm, or a range defined by any two of these values.

In some embodiments, when the hole transport layer includes the above organic compound, a percentage of the above organic compound in the perovskite layer may be 0 to 0.5%; and optionally, the mass percentage of the above organic compound in the perovskite layer may be 0, 0.1%, 0.2%, 0.3%, 0.4%, or 0.5%.

In some embodiments, when the hole transport layer includes the above organic compound, a percentage of the above organic compound in the passivation layer may be 0 to 100%; and optionally, the mass percentage of the above organic compound in the passivation layer may be 0, 10wt%, 20wt%, 30wt%, 40wt%, 50wt%, 60wt%, 70wt%, 80wt%, 90wt%, or 100wt%, or a range defined by any two of these values.

In some embodiments, the perovskite layer includes the above organic compound, where a mass percentage of the organic compound in the perovskite layer is K3, and 0.01% ≤ K3 ≤ 0.5%.

Optionally, 0.01% < K3 ≤ 0.5%. Further, K3 may be 0.1wt% to 0.5wt%, for example, 0.1wt%, 0.2wt%, 0.3wt%, 0.4wt%, or 0.5wt%, or a range defined by any two of these values.

In some embodiments, the above organic compound is doped by adding it to a perovskite precursor solution, where a concentration of the organic compound in the perovskite precursor solution is 0.1 mg/mL to 5 mg/mL.

In some embodiments, when the perovskite layer includes the above organic compound, a mass percentage of the above organic compound in the passivation layer may be 0 to 100% and further may be 0, 10%, 20%, 30%, 40%, 50%, 60%, 70%, 80%, 90%, or 100%.

In other words, when the perovskite layer includes the above organic compound, the passivation layer may contain the above organic compound, other passivation materials commonly used in the art, or a mixture thereof.

Other passivation materials commonly used in the art are as described above, which are not described herein again.

In some embodiments, when the perovskite layer includes the above organic compound, a mass percentage of the above organic compound in the hole transport layer may be 0 to 100% and further may be 0, 10%, 20%, 30%, 40%, 50%, 60%, 70%, 80%, 90%, or 100%.

In other words, when the perovskite layer includes the above organic compound, the hole transport may contain the above organic compound, other hole transport materials commonly used in the art, or a mixture thereof.

Other hole transport materials commonly used in the art are as described above, which are not described herein again.

Still referring to FIG. 1, the above solar cell 10 further includes a first electrode 11, an electron transport layer 15, a hole-blocking layer 16, and a second electrode 17.

The first electrode 11 is located on a surface of the hole transport layer 12 away from the passivation layer 13. The electron transport layer 15 is located on a surface of the perovskite layer 14 away from the passivation layer 13. The hole-blocking layer 16 is located on a surface of the electron transport layer 15 away from the perovskite layer 14. The second electrode 17 is located on a surface of the hole-blocking layer 16 away from the electron transport layer 15.

In some embodiments, the above solar cell 10 may be a regular solar cell (with an n-i-p planar structure) or an inverted solar cell (with a p-i-n planar structure).

It should be noted that when the first electrode 11 is a transparent electrode, that is, the first electrode side serves as a light incident side, the above solar cell 10 is an inverted solar cell. Conversely, when the second electrode 17 is a transparent electrode, that is, the second electrode side serves as a light incident side, the above solar cell 10 is a regular solar cell. In some embodiments, the first electrode 11 is a transparent conductive electrode, and a material of the first electrode 11 may be any one of fluorine-doped tin oxide (FTO), tin-doped indium oxide (ITO), boron-doped zinc oxide (BZO), aluminum-doped zinc oxide (AZO), and IZO.

In some embodiments, components of the electron transport layer 15 may be electron transport materials commonly used in the art, with non-limiting examples including: [6,6]-phenyl-C61-butyric acid methyl ester (PC61BM), [6,6]-phenyl-C71-butyric acid methyl ester (PC71BM), fullerene C60 (C60), fullerene C70 (C70), tin dioxide (SnO₂), and zinc oxide (ZnO).

In some embodiments, components of the hole-blocking layer 16 may be hole-blocking materials commonly used in the art, with non-limiting examples including: at least one of BCP (2,9-dimethyl-4,7-diphenyl-1,10-phenanthroline), 1,3,5-tris(1-phenyl-1H-benzimidazol-2-yl)benzene, and 4,4'-bis(2,2-diphenylvinyl)-1,1'-biphenyl.

In some embodiments, a material of the second electrode 16 may be an electrode material commonly used in the art, including but not limited to the following materials: Ag, Cu, C, Au, Al, ITO, AZO, BZO, IZO, and the like.

The preparation processes for the above first electrode, hole transport layer, perovskite layer, electron transport layer, and second electrode may use preparation methods commonly used in the art, including a solution method and a solid deposition method. The solution method includes any one of a spin coating method, a spray coating method, a blade coating method, and a slot-die coating method. The solid deposition method includes any one of vacuum evaporation, sputtering deposition, plasma deposition, and ion deposition.

In some embodiments, the above functional film layer is a hole transport layer, and components of the hole transport layer include the organic compound.

The hole transport material can directly serve as the component of the hole transport layer, providing both a passivation effect and a hole transport effect. Even when the hole transport layer is in direct contact with the perovskite layer, that is, an additional passivation layer is not provided, the efficiency of a device can still be improved.

In some embodiments, the solar cell includes an electron transport layer, a perovskite layer, and a hole transport layer arranged in a stacked manner, where the perovskite layer is in direct contact with the hole transport layer, and at least one of the perovskite layer and the hole transport layer includes the above organic compound.

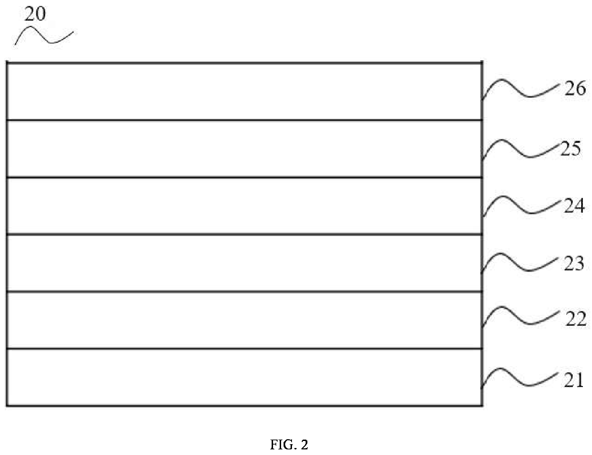

In some embodiments, referring to FIG. 2, a solar cell 20 is provided. The solar cell 20 includes a first electrode 21, a hole transport layer 22, a perovskite layer 23, an electron transport layer 24, a hole-blocking layer 25, and a second electrode 26 arranged in a stacked manner.

At least one of the perovskite layer 23 and the hole transport layer 22 includes at least one of the organic compound represented by formula (1) and an oxyanion salt thereof.

In some embodiments, the hole transport layer 22 includes the above organic compound, where a mass percentage of the organic compound in the hole transport layer is K2, and 0 < K2 ≤ 100%.

Optionally, K2 may be 1wt% to 100wt%, for example, 10wt%, 20wt%, 30wt%, 40wt%, 50wt%, 60wt%, 70wt%, 80wt%, 90wt%, or 100wt%, or a range defined by any two of these values.

In some embodiments, when K2 is not 100%, the hole transport layer may further include other hole transport layer materials commonly used in the art, such as at least one of nickel oxide, zinc oxide, molybdenum oxide, 2,2',7,7'-tetrakis(N,N-di-p-methoxyphenylamino)-9,9'-spirobifluorene (Spiro-OMeTAD), poly[bis(4-phenyl)(2,4,6-trimethylphenyl)amine] (PTAA), and [4-(3,6-dimethyl-9H-carbazol-9-yl)butyl]phosphonic acid (Me-4Pacz).

In some embodiments, when K2 is 100%, it means that the material of the hole transport layer is the above organic compound.

In some embodiments, when the hole transport layer 22 includes the above organic compound, a mass percentage of the above organic compound in the perovskite layer may be 0 to 0.5% and further may be 0, 0.1wt%, 0.2wt%, 0.3wt%, 0.4wt%, or 0.5wt%.

In other words, when the hole transport layer includes the above organic compound, the perovskite layer may be doped with the above organic compound. Further, the perovskite materials in the perovskite layer may be perovskite materials commonly used in the art, with types as described above, which are not described herein again.

In some embodiments, the perovskite layer includes the above organic compound, where a mass percentage of the organic compound in the perovskite layer is K3, and 0.01% < K3 ≤ 0.5%.

Optionally, K3 may be 0.1wt% to 0.5wt%, for example, 0.1wt%, 0.2wt%, 0.3wt%, 0.4wt%, or 0.5wt%, or a range defined by any two of these values.

In some embodiments, the above organic compound is doped by adding it to a perovskite precursor solution, where a concentration of the organic compound in the perovskite precursor solution is 0.1 mg/mL to 5 mg/mL.

In some embodiments, when the perovskite layer includes the above organic compound, a mass percentage of the above organic compound in the hole transport layer may be 0 to 100% and further may be 0, 10%, 20%, 30%, 40%, 50%, 60%, 70%, 80%, 90%, or 100%.

In other words, when the perovskite layer includes the above organic compound, the hole transport layer may contain the above organic compound, other hole transport materials commonly used in the art, or a mixture thereof.

The types of other hole transport materials commonly used in the art are as described above, which are not described herein again.

The material selected for the first electrode 21, the electron transport layer 24, the hole-blocking layer 25, and the second electrode 26 respectively correspond to the materials and types selected for the first electrode 11, the electron transport layer 15, the hole-blocking layer 26, and the second electrode 17 as described above, which are not described herein again.

In some embodiments, the solar cell includes a second electrode, an electron transport layer, a perovskite layer, and a first electrode arranged in a stacked manner, where the perovskite layer is in direct contact with the first electrode, and the perovskite layer includes the above organic compound.

In other words, when the perovskite layer includes the above organic compound, the photoelectric efficiency can still be improved even without additional hole transport or passivation layers.

It can be understood that the above perovskite layer includes perovskite materials which may be perovskite materials commonly used in the art. The perovskite materials commonly used in the art are as described above and are not described herein again.

In some embodiments, a mass percentage of the above organic compound in the perovskite layer is 0.01% to 0.5%, optionally, 0.1wt% to 0.5wt%, for example, 0.1wt%, 0.2wt%, 0.3wt%, 0.4wt%, or 0.5wt%, or a range defined by any two of these values.

In some embodiments, the above organic compound is doped by adding it to a perovskite precursor solution, where a concentration of the organic compound in the perovskite precursor solution is 0.1 mg/mL to 5 mg/mL.

In some embodiments, the above solar cell further includes a hole-blocking layer disposed on a surface of the electron transport layer away from the perovskite layer.

Materials selected for the first electrode, the hole-blocking layer, the electron transport layer, and the second electrode are as described above, which are not described herein again.

In an embodiment of this application, a photovoltaic module is further provided, where the photovoltaic module includes the above solar cell.

The above solar cell has high photoelectric conversion efficiency and high stability, which can improve the efficiency of the photovoltaic module.

The above photovoltaic module includes one or more solar cells, which can be selected based on specific application scenarios. Further, the above photovoltaic module includes multiple solar cells, where the multiple solar cells are connected in series or parallel to form a cell sheet.

In some embodiments, the above photovoltaic module further includes a photovoltaic glass layer, an adhesive layer, and a backsheet.

Two surfaces of the cell sheet are respectively provided with adhesive layers, where the backsheet is disposed on a surface of one adhesive layer away from the cell sheet, and the photovoltaic glass layer is disposed on a surface of the other adhesive layer away from the cell sheet.

The photovoltaic glass layer and the backsheet are used to protect the solar cell, providing sealing, insulation, and waterproofing. The adhesive layers are used for bonding the photovoltaic glass layer to the cell sheet and bonding the backsheet to the cell sheet.

Optionally, a material of the photovoltaic glass layer is tempered glass, a material of the backsheet is a TPT (polyvinyl fluoride) or TPE (thermoplastic elastomer) material, and a material of the adhesive layer is EVA (an ethylene-vinyl acetate copolymer).

Further, the above photovoltaic module further includes a junction box and an outer frame.

The junction box is configured to protect a power generation system of the entire photovoltaic module, acting as a current transfer station. If the cell sheet has a short circuit, the junction box can automatically disconnect a short-circuited cell string.

The outer frame can provide support and protection for the entire photovoltaic module, and the frame may be made of aluminum alloy, offering excellent strength and corrosion resistance.

Further, silicone is used to bond and seal junctions between the frame and other parts of the photovoltaic module. The photovoltaic module can convert solar energy into electrical energy which is transferred to a storage battery for storage or used for driving a load to operate.

In some embodiments, the above photovoltaic module is a solar panel.

In an embodiment of this application, a photovoltaic system is further provided, including the above photovoltaic module.

The photovoltaic system utilizes the photovoltaic effect of the solar cell in the above photovoltaic module to directly convert solar radiation energy into electrical energy, with high efficiency. Further, the above photovoltaic system is a photovoltaic power generation system.

The photovoltaic module is a core component of the photovoltaic power generation system. The above photovoltaic system includes one or more photovoltaic modules, which can be selected based on specific application scenarios. Further, when the above photovoltaic system includes multiple photovoltaic modules, the multiple photovoltaic modules form a photovoltaic array.

The above photovoltaic system may be an off-grid photovoltaic power generation system or a grid-connected photovoltaic power generation system.

The off-grid photovoltaic power generation system includes a photovoltaic array, a battery pack, a charge controller, a power electronic converter (inverter), and a load. The working principle of the off-grid photovoltaic power generation system is that solar radiation energy is first converted into electrical energy through the photovoltaic array, then transformed by the power electronic converter to supply power to the load, while excess electrical energy passes through the charge controller and is then stored in the form of chemical energy in an energy storage apparatus. Therefore, when sunlight is insufficient, the energy stored in the battery can be boosted by the power electronic inverter, a filter, and a power frequency transformer to be converted into alternating-current electrical energy with 220 V and 50 Hz for use by alternating-current loads.

The grid-connected photovoltaic power generation system includes a photovoltaic array, a high-frequency DC/DC boost circuit, a power electronic converter (inverter), and a system monitor. The working principle of the grid-connected photovoltaic power generation system is that solar radiation energy is converted by the photovoltaic array, transformed into high-voltage direct current through high-frequency direct current conversion, and then inverted by the power electronic inverter to output sinusoidal alternating current consistent in phase and frequency with the grid voltage to the grid.

The above two photovoltaic power generation systems each have characteristics and can be selected based on specific application scenarios.

In an embodiment of this application, an electric apparatus is further provided, including at least one of the above solar cell and the above photovoltaic module.

The above electric apparatus may include but is not limited to a mobile device, an electric vehicle, an electric train, a ship, a satellite, an energy storage system, and the like.

In some embodiments, the mobile device may be a mobile phone, a laptop computer, or the like.

In some embodiments, the electric vehicle includes but is not limited to pure electric vehicles, hybrid electric vehicles, plug-in hybrid electric vehicles, electric bicycles, electric scooters, electric golf carts, electric trucks, and the like.

The following describes this application with reference to specific examples, but this application is not limited to the examples below. It should be understood that the appended claims summarize the scope of this application, and those skilled in the art, guided by the concept of this application, should recognize that certain changes to the embodiments of this application will be covered by the spirit and scope of the claims of this application.

The following describes specific examples.

### Example 1

### Step I: Preparation of organic compound SAM1, with specific steps as follows:

(1) Compound 1 (1 mmol), potassium carbonate (K₂CO₃, 1.5 mmol), copper iodide (CuI, 2 mmol), compound 2 (1.1 mmol), and DMF (10 mL) were mixed, heated for reaction at 125°C for 20 hours under nitrogen protection, and then separated using a silica gel chromatographic column to obtain compound 3. The synthetic route was as follows:

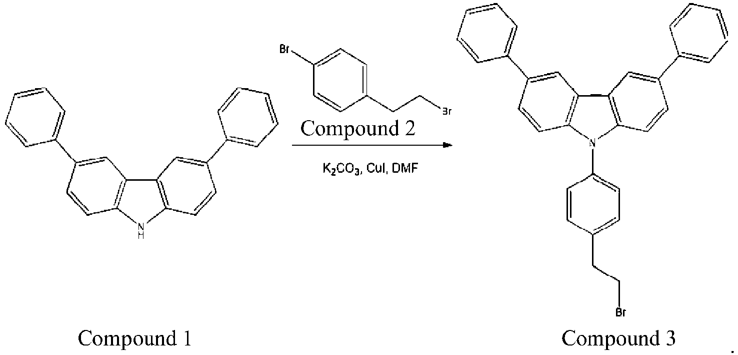

Compound 3 was subjected to ¹H NMR spectrum test, with results as follows: ¹H NMR (400 MHz, DMSO-d6) δ 8.18 (d, J = 7.2 Hz, 2H), 7.98 (d, J = 7.2 Hz, 2H), 7.89 (s, 2H), 7.75 (d, J = 7.2 Hz, 4H), 7.53-7.41 (m, 8H), 7.29 (d, J = 7.2 Hz, 2H), 3.66-3.61 (m, 2H), 3.08-3.03 (m, 2H).

The above results indicated that the target product was successfully obtained in the preparation steps. The yield of compound 3 was further calculated using the following formula to be 58%.
$Yield = moles of compound 3 / moles of compound 1 \times 100 % .$

(2) Compound 3 (1 mmol) and triethyl phosphite (P(OEt)₃, 10 mL) were mixed and then heated for reaction at 160°C for 20 hours under nitrogen protection. The remaining triethyl phosphite was removed using a vacuum distillation method. A crude product was mixed with tributylbromosilane (TMSBr, 0.72 mmol) and 1,4-dioxane (5 mL), and stirred at room temperature for 20 hours under nitrogen protection. Then, the solvent was removed. Methanol (MeOH, 5 mL) was added and stirred for 12 hours. Then, deionized water (1 mL) was added, and solid powder was precipitated. Then, SAM1 was obtained. The synthetic route was as follows:

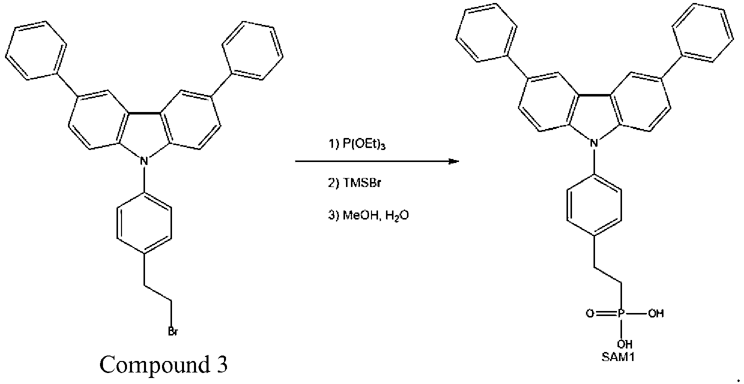

The product SAM1 was subjected to ¹H NMR spectrum test, with test results as follows:
¹H NMR (400 MHz, DMSO-d6) δ 8.18 (d, J = 7.2 Hz, 2H), 7.98 (d, J = 7.2 Hz, 2H), 7.89 (s, 2H), 7.75 (d, J = 7.2 Hz, 4H), 7.53-7.41 (m, 8H), 7.29 (d, J = 7.2 Hz, 2H), 2.83-2.79 (m, 2H), 2.03-2.00 (m, 2H).

The above results indicated that the target product SAM1 was successfully obtained in the preparation steps.

The yield of the product SAM1 was further calculated using the following formula to be 55%.
$Yield = moles of product SAM 1 / moles of compound 3 \times 100 %$

Step II: Preparation of solar cell, with specific steps as follows:
1. Cleaning of FTO conductive glass: A 2.0 cm × 2.0 cm FTO conductive glass was laser-etched to remove 0.35 cm from both ends, with a glass substrate exposed. Then, the FTO conductive glass was then sequentially ultrasonically cleaned in deionized water, acetone, and isopropanol for 10 minutes each. The cleaned FTO conductive glass was dried with a nitrogen gun to dry the solvent, and then placed into an ultraviolet-ozone cleaner for ultraviolet-ozone cleaning. Then, the resulting FTO conductive glass served as a first electrode.
2. Preparation of hole transport layer: A nano-tin oxide-containing methanol solution (10 mg/mL) was spin-coated at 2000 rpm on a surface of the FTO conductive glass, followed by annealing to remove the solvent and forming a nickel oxide film. That was, a hole transport layer with a thickness of 30 nm was obtained.
3. Preparation of passivation layer: The compound SAM1 was dissolved in methanol to obtain a self-assembled molecular solution (1 mg/mL). The self-assembled molecular solution was spin-coated at a speed of 3000 rpm on a surface of the hole transport layer, followed by annealing to form a self-assembled molecular layer. That was, a passivation layer with a thickness of 5 nm was obtained.
4. Preparation of perovskite layer: Lead iodide (726 mg), formamidinium iodide (240 mg), cesium iodide (19 mg), and lead bromide (11 mg) were weighed and dissolved in 1 mL of a mixed solvent with DMF and DMSO at a volume ratio of 4:1, stirred for 3 h, and filtered through a 0.22 µm organic filter film to obtain a perovskite precursor solution. The precursor solution was spin-coated at a speed of 3000 rpm on a surface of the passivation layer, followed by annealing at 100°C for 30 min, and cooling to room temperature, to form a perovskite layer with a CsFA system as an active material, with a thickness of 800 nm.
5. Preparation of electron transport layer: An electron transport material PC₆₁BM was spin-coated at a speed of 1500 rpm on a surface of the perovskite layer to form an electron transport layer with a thickness of 35 nm. A hole-blocking material BCP (2,9-dimethyl-4,7-diphenyl-1,10-phenanthroline) was then spin-coated at a speed of 5000 rpm, followed by annealing at 100°C for 10 min, to form a hole-blocking layer with a thickness of 15 nm.
6. Preparation of second electrode: A device obtained in step 5 was placed in a mask, and 80 nm of silver was evaporated on a surface of the hole-blocking layer using a vacuum evaporation device to form a second electrode, and a complete perovskite solar cell 10 was obtained.
   The specific structure of the perovskite solar cell 10, with reference to FIG. 1, included a first electrode 11, a hole transport layer 12, a passivation layer 13, a perovskite layer 14, an electron transport layer 15, a hole-blocking layer 16, and a second electrode 17 that were sequentially stacked.
7. Performance test: The photoelectric conversion efficiency was tested using an I-V measurement method, with the specific steps as follows:

Current measurement was performed while bias voltage points were varied to obtain the I-V characteristics of a tested sample.
(a) A test fixture mounted with a sample cell was placed on a sample holder, the sample cell was located within a measurement plane, and the sample cell was located at the center of a light spot emitted by a solar simulator (or the normal of a photovoltaic cell is parallel to a center line of a light beam emitted by the solar simulator).
(b) The Enli Tech solar simulator was used for test in accordance with the national standard IEC61215. The light intensity was calibrated using a crystalline silicon solar cell to achieve one sun intensity. Under an irradiance of 1000 W/m², a mask was mounted on the sample cell under test. The temperature of the sample cell was controlled using a temperature monitoring device, such that the temperature of the sample under test was maintained at (30 ± 5°C) during the measurement process.
(c) A scanning direction, a voltage range, a scanning interval voltage, and scanning interval time were set. It was recommended that the scanning interval was not greater than 0.02 V, and the interval time between adjacent points was not less than 0. s. The forward and reverse sweep current-voltage characteristics of the sample cell under test were tested. A maximum power point current (Vₘ), a maximum power point voltage, an open-circuit voltage V_{oc}, and a short-circuit current J_{sc} were recorded.

Calculation formulas: Fill factor FF = Jₘ × Vₘ / V_{oc} × J_{sc}, Photoelectric conversion efficiency PCE = V_{oc} × J_{sc} × FF / Pᵢₙ. Here, Pᵢₙ was the incident light intensity, equal to 10³ W/m². The perovskite solar cell was naturally aged for 10 days under a nitrogen atmosphere at room temperature. During this period, the photoelectric conversion efficiency was tested every 12 h according to the above steps, and the highest efficiency was recorded as the optimal efficiency. Specific results were shown in Table 1.

The photoelectric conversion efficiency PCE of the perovskite solar cell after stored for 30 days in an N₂ atmosphere at room temperature was recorded as P30, with results shown in Table 1.

### Example 2

Example 2 was substantially the same as Example 1 except that the preparation material of the passivation layer, the compound SAM1, was replaced with the compound SAM2. The specific preparation method was as follows:

Step (1): With reference to the preparation step (1) of the organic compound SAM1 in Example 1, compound 1 was replaced with compound 4 in an equimolar amount, and compound 2 was replaced with compound 5 in an equimolar amount. Then, the reaction was performed to obtain compound 6. The synthetic route was as follows:

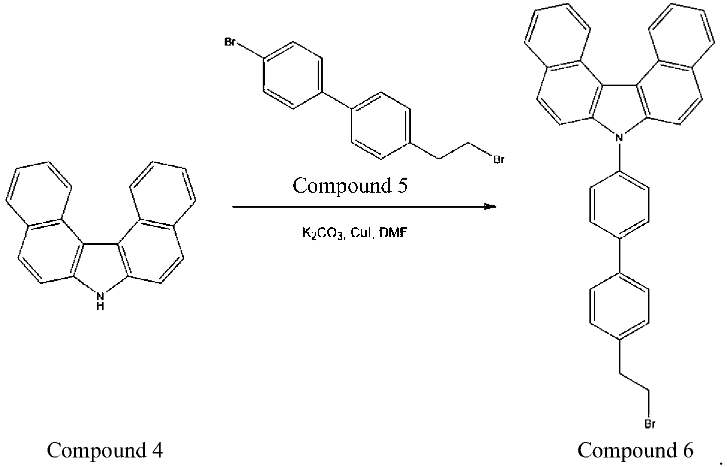

Compound 6 was subjected to ¹H NMR spectrum test, with results as follows: ¹H NMR (400 MHz, DMSO-*d6*) *δ* 8.54 (d, *J* = 7.2 Hz, 2H), 7.99-7.91 (m, 8H), 7.62-7.53 (m, 8H), 7.32 (d, *J* = 7.2 Hz, 2H), 3.66-3.61 (m, 2H), 3.08-3.03 (m, 2H).

The above results indicated that the target product was successfully obtained in the preparation steps. The yield of compound 6 was further calculated using the following formula to be 77%.
$Yield = moles of compound 6 / moles of compound 4 \times 100 % .$

Step (2): With reference to the preparation step (2) of the organic compound SAM1 in Example 1, compound 3 was replaced with compound 6 in an equimolar amount. Then, the reaction was performed to obtain compound SAM2. The synthetic route was as follows:

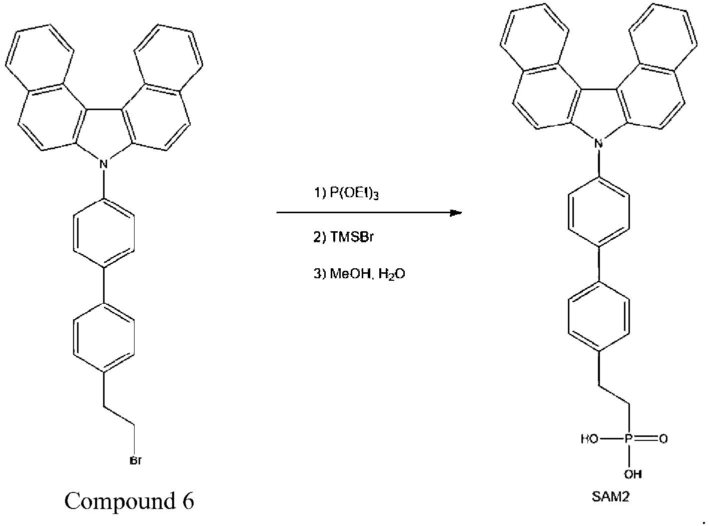

Compound SAM2 was subjected to ¹H NMR spectrum test, with results as follows: ¹H NMR (400 MHz, DMSO-*d₆*) *δ* 8.97 (d, *J =* 7.2 Hz, 2H), 8.31 (s, 6H), 8.12 (d, *J =* 7.2 Hz, 2H), 7.59 (d, *J =* 7.2 Hz, 2H), 7.28 (d, *J* = 7.2 Hz, 2H), 7.02 (s, 2H), 4.18-4.14 (m, 2H), 1.73-1.69 (m, 4H), 1.26-1.23 (m, 2H).

The above results indicated that the target product SAM2 was successfully obtained in the preparation steps. The yield of the product SAM2 was further calculated using the following formula to be 45%.
$Yield = moles of product SAM 2 / moles of compound 6 \times 100 % .$

The remaining steps were the same as those in Example 1, with specific results shown in Table 1.

### Example 3

Example 3 was substantially the same as Example 1 except that the preparation material of the passivation layer, the compound SAM1, was replaced with the compound SAM3. The specific preparation method was as follows:
Step (1): With reference to the preparation step (1) of the organic compound SAM1 in Example 1, compound 1 was replaced with compound 7 in an equimolar amount, and compound 2 was replaced with compound 8 in an equimolar amount. Then, the reaction was performed to obtain compound 9. The synthetic route was as follows:

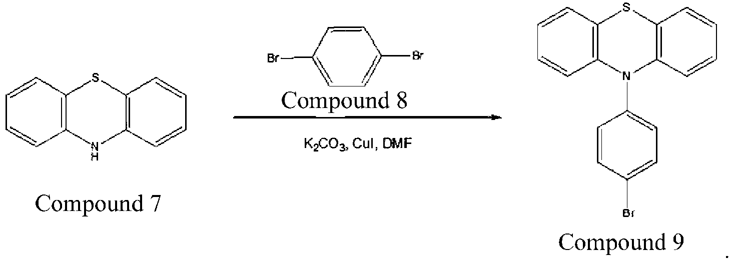

Compound 9 was subjected to ¹H NMR spectrum test, with results as follows: ¹H NMR (400 MHz, DMSO-*d6*) *δ* 7.38 (d, *J=* 7.2 Hz, 2H), 7.21-7.16 (m, 6H), 7.07 (d, *J* = 7.2 Hz, 2H), 7.00-6.96 (m, 2H).

The above results indicated that the target product was successfully obtained in the preparation steps. The yield of compound 9 was further calculated using the following formula to be 77%.
$Yield = moles of compound 9 / moles of compound 7 \times 100 % .$

Step (2): Compound 9 (1 mmol), compound 10 (1.1 mmol), potassium acetate (KOAc, 2 mmol), 1,1'-bis(diphenylphosphino)ferrocene palladium(II) dichloride (5% mmol), and 1,4-dioxane (10 mL) were mixed, heated for reaction at 85°C for 12 hours under nitrogen protection, and filtered through diatomaceous earth to obtain a filtrate. After the solvent was removed, a crude product was mixed with compound 11 (1.2 mmol), tetrakis(triphenylphosphine)palladium (5% mmol), toluene (10 mL), and an aqueous solution of potassium carbonate (2 M, 10 mL), heated for reaction at 110°C for 48 hours under nitrogen protection, and separated using a silica gel chromatographic column to obtain compound 12. The synthetic route was as follows:

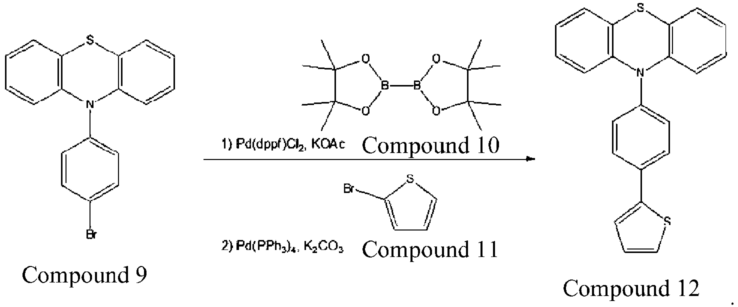

Compound 12 was subjected to ¹H NMR spectrum test, with results as follows: ¹H NMR (400 MHz, DMSO-*d6*) *δ* 7.70 (d, *J* = 7.2 Hz, 1H), 7.40-7.34 (m, 3H), 7.21-7.13 (m, 7H), 7.07 (d, *J* = 7.2 Hz, 2H), 7.00-6.96 (m, 2H).

The above results indicated that the target product was successfully obtained in the preparation steps. The yield of compound 12 was further calculated using the following formula to be 57%.
$Yield = moles of compound 12 / moles of compound 9 \times 100 % .$

Step (3): Compound 12 (1 mmol) was dissolved in tetrahydrofuran (THF, 10 mL), and an n-hexane solution of n-butyllithium (n-BuLi, 2.5 M, 0.5 mL) was added dropwise at -78°C. After the resulting solution was stirred for 2 h a tetrahydrofuran solution of zinc chloride (1 M, 1.3 mL) was added. After the resulting solution was stirred at room temperature for 2 h, tetrakis(triphenylphosphine)palladium (5% mmol) and compound 2 (1.1 mmol) were added. After the resulting solution was heated at 75°C, the resulting product was separated using a silica gel chromatographic column to obtain compound 13. The synthetic route was as follows:

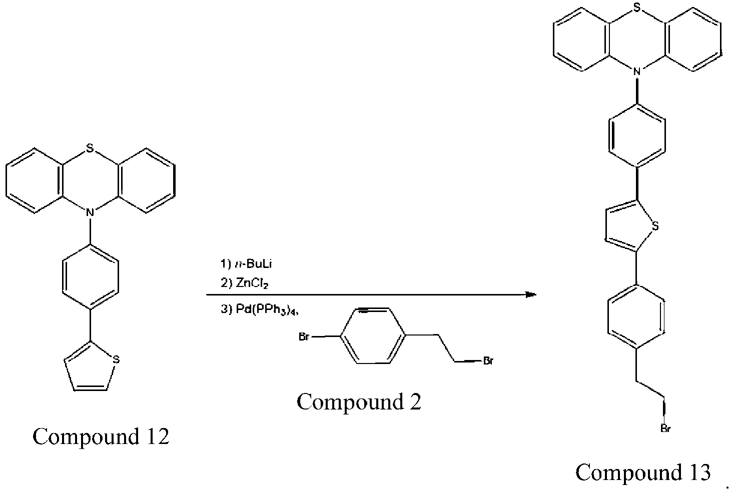

Compound 13 was subjected to ¹H NMR spectrum test, with results as follows: ¹H NMR (400 MHz, DMSO-*d₆*) *δ* 7.77 (d, *J* = 7.2 Hz, 2H), 7.37-7.30 (m, 6H), 7.21-7.15 (m, 6H), 7.00-6.96 (m, 4H), 3.66-3.60 (m, 2H), 3.08-3.03 (m, 2H).

The above results indicated that the target product SAM13 was successfully obtained in the preparation steps. The yield of the product 13 was further calculated using the following formula to be 58%.
$Yield = moles of product 13 / moles of compound 12 \times 100 % .$

Step (4): Compound 13 (1 mmol) and triethyl phosphite (P(OEt)₃, 10 mL) were mixed and then heated for reaction at 160°C for 20 hours under nitrogen protection. The remaining triethyl phosphite was removed using a vacuum distillation method. A crude product was mixed with tributylbromosilane (TMSBr, 0.72 mmol) and 1,4-dioxane (5 mL), and stirred at room temperature for 20 hours under nitrogen protection. Then, the solvent was removed. Methanol (5 mL) was added and stirred for 12 hours. Then, deionized water (1 mL) was added, and solid powder was precipitated. Then, SAM3 was obtained. The synthetic route was as follows:

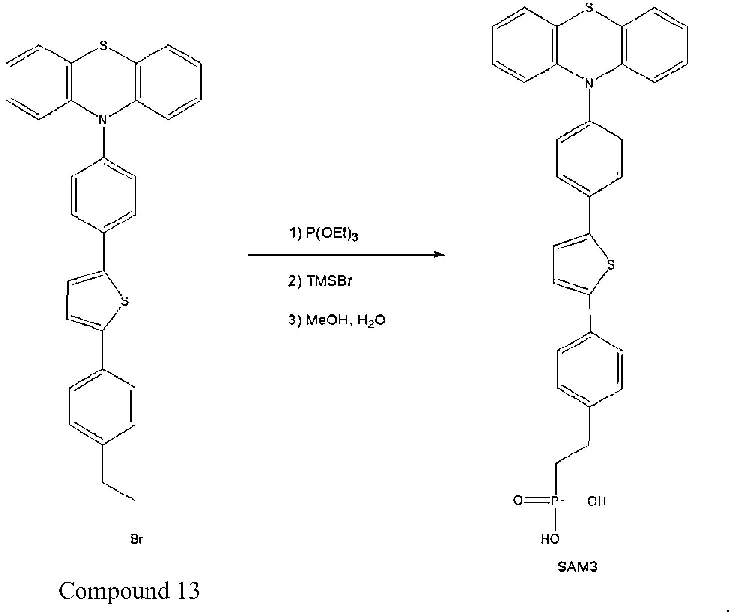

Compound SAM3 was subjected to ¹H NMR spectrum test, with results as follows: ¹H NMR (400 MHz, DMSO-*d₆*) *δ* 7.77 (d, *J* = 7.2 Hz, 2H), 7.37-7.30 (m, 6H), 7.21-7.15 (m, 6H), 7.00-6.96 (m, 4H), 2.84-2.78 (m, 2H), 2.04-1.99 (m, 2H).

The above results indicated that the target product SAM3 was successfully obtained in the preparation steps. The yield of the product SAM3 was further calculated using the following formula to be 45%.
$Yield = moles of product SAM 3 / moles of compound 13 \times 100 % .$

The remaining steps were the same as those in Example 1, with specific results shown in Table 1.

### Example 4

Example 4 was substantially the same as Example 1 except that the preparation material of the passivation layer, the compound SAM1, was replaced with the compound SAM4. The specific preparation method was as follows:
Step (1): Compound 14 (1 mmol), compound 15 (1.1 mmol), tetrakis(triphenylphosphine)palladium (5% mmol), toluene (10 mL), and an aqueous solution of potassium carbonate (2 M, 10 mL) were mixed, heated for reaction at 110°C for 48 hours under nitrogen protection, and separated using a silica gel chromatographic column to obtain compound 16. The synthetic route was as follows:

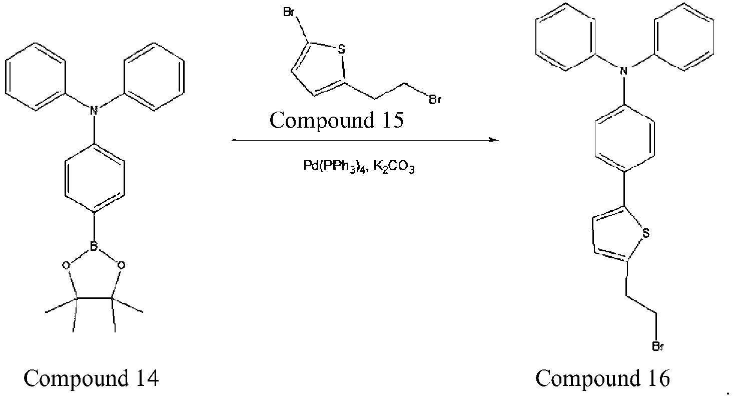

Compound 16 was subjected to ¹H NMR spectrum test, with results as follows: ¹H NMR (400 MHz, DMSO-*d₆*) *δ* 7.37-7.24 (m, 7H), 7.08-6.99 (m, 8H), 6.80 *(d, J* = 7.2 Hz, 1H), 3.56-3.51 (m, 2H), 3.38-3.33 (m, 2H).

The above results indicated that the target product was successfully obtained in the preparation steps. The yield of compound 16 was further calculated using the following formula to be 77%.
$Yield = moles of compound 16 / moles of compound 14 \times 100 % .$

Step (2): With reference to the preparation step (2) of the organic compound SAM1 in Example 1, compound 3 was replaced with compound 16 in an equimolar amount. Then, the reaction was performed to obtain compound SAM4. The synthetic route was as follows:

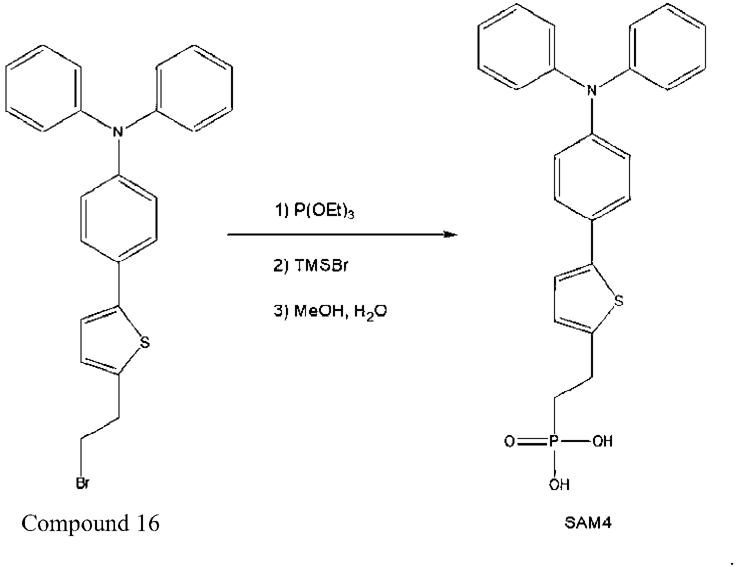

Compound SAM4 was subjected to ¹H NMR spectrum test, with results as follows: ¹H NMR (400 MHz, DMSO-*d₆*) *δ* 7.37-7.24 (m, 7H), 7.08-6.99 (m, 8H), 6.80 (d, *J* = 7.2 Hz, 1H), 3.11-3.05 (m, 2H), 1.98-1.93 (m, 2H).

The above results indicated that the target product SAM4 was successfully obtained in the preparation steps. The yield of the product SAM4 was further calculated using the following formula to be 45%.
$Yield = moles of product SAM 4 / moles of compound 16 \times 100 % .$

The remaining steps were the same as those in Example 1, with specific results shown in Table 1.

### Example 5

Example 5 was substantially the same as Example 1 except that the preparation material of the passivation layer, the compound SAM1, was replaced with the compound SAM5. The specific preparation method was as follows:
Step (1): Compound 14 (1 mmol), compound 17 (1.1 mmol), tetrakis(triphenylphosphine)palladium (5% mmol), toluene (10 mL), and an aqueous solution of potassium carbonate (2 M, 10 mL) were mixed, heated for reaction at 110°C for 48 hours under nitrogen protection, and separated using a silica gel chromatographic column to obtain compound 18. The synthetic route was as follows:

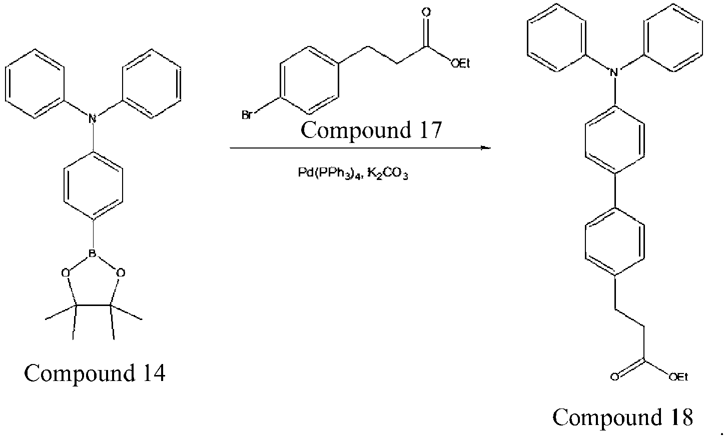

Compound 18 was subjected to ¹H NMR spectrum test, with results as follows: ¹H NMR (400 MHz, DMSO-*d₆*) *δ* 7.62 (d, *J* = 7.2 Hz, 2H), 7.55 (d, *J* = 7.2 Hz, 2H), 7.37-7.24 (m, 8H), 7.08-7.00 (m, 6H), 4.03-3.98 (m, 2H), 2.86-2.81 (m, 2H), 2.58-2.53 (m, 2H), 1.09-1.05 (m, 3H).

The above results indicated that the target product was successfully obtained in the preparation steps. The yield of compound 18 was further calculated using the following formula to be 89%.
$Yield = moles of compound 18 / moles of compound 14 \times 100 % .$

Step (2): Compound 18 (1 mmol) was dissolved in tetrahydrofuran (10 mL) and mixed with an aqueous solution of sodium hydroxide (NaOH, 2 M, 10 mL). The resulting solution was heated at 75°C for 20 h. Then, a concentrated hydrochloric acid was added dropwise until the pH of the solution was less than 1. The precipitate was collected to obtain SAM5. The synthetic route was as follows:

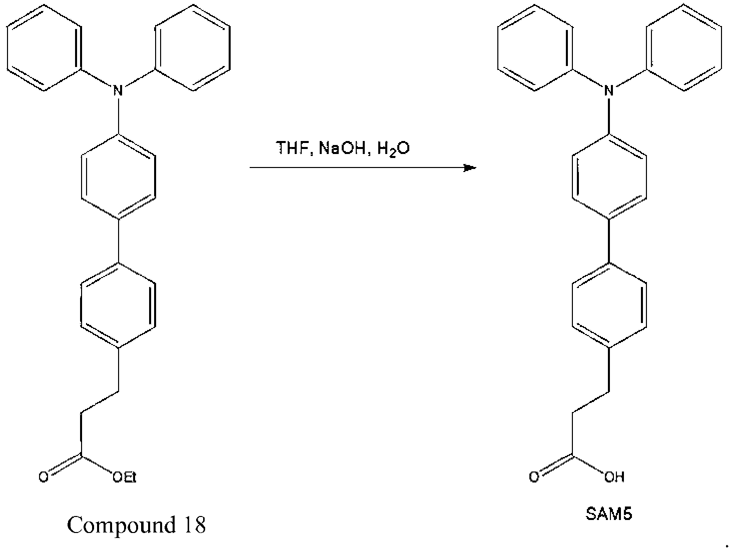

Compound SAM5 was subjected to ¹H NMR spectrum test, with results as follows: ¹H NMR (400 MHz, DMSO-*d₆*) *δ* 12.03 (s, 1H), 7.62 (d, *J* = 7.2 Hz, 2H), 7.55 (d, *J* = 7.2 Hz, 2H), 7.37-7.24 (m, 8H), 7.08-7.00 (m, 6H), 4.03-3.98 (m, 2H), 2.85-2.81 (m, 2H), 2.54-2.48 (m, 2H).

The above results indicated that the target product SAM5 was successfully obtained in the preparation steps. The yield of the product SAM5 was further calculated using the following formula to be 72%.
$Yield = moles of product SAM 5 / moles of compound 18 \times 100 % .$

The remaining steps were the same as those in Example 1, with specific results shown in Table 1.

### Example 6

Example 6 was substantially the same as Example 1 except that the preparation material of the passivation layer, the compound SAM1, was replaced with the compound SAM6. The specific preparation method was as follows:
Step (1): Compound 19 (1 mmol), compound 2 (1.1 mmol), tris(dibenzylideneacetone)dipalladium (Pd₂dba₃, 5% mmol), tri-tert-butylphosphine (*t*-Bu₃P, 10 mmol), and sodium tert-butoxide (2 mmol) were filtered to obtain compound 20. The synthetic route was as follows:

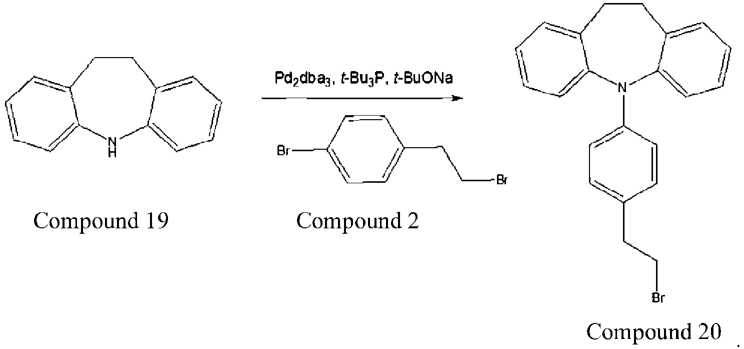

Compound 20 was subjected to ¹H NMR spectrum test, with results as follows: ¹H NMR (400 MHz, DMSO-*d₆*) *δ* 7.23-7.16 (m, 6H), 7.10-7.00 (m, 4H), 6.79-6.73 (m, 2H), 3.66-3.61 (m, 2H), 2.93-2.88 (m, 6H).

The above results indicated that the target product was successfully obtained in the preparation steps. The yield of compound 20 was further calculated using the following formula to be 55%.
$Yield = moles of compound 20 / moles of compound 19 \times 100 % .$

Step (2): Compound 20 (1 mmol) and triethyl phosphite (P(OEt)₃, 10 mL) were mixed and then heated at 160°C for 20 hours under nitrogen protection. The remaining triethyl phosphite was removed using a vacuum distillation method. A crude product was mixed with tributylbromosilane (TMSBr, 0.72 mmol) and 1,4-dioxane (5 mL), and stirred at room temperature for 20 hours under nitrogen protection. Then, the solvent was removed. Methanol (5 mL) was added and stirred for 12 hours. Then, deionized water (1 mL) was added, and solid powder was precipitated. Then, SAM6 was obtained. The synthetic route was as follows:

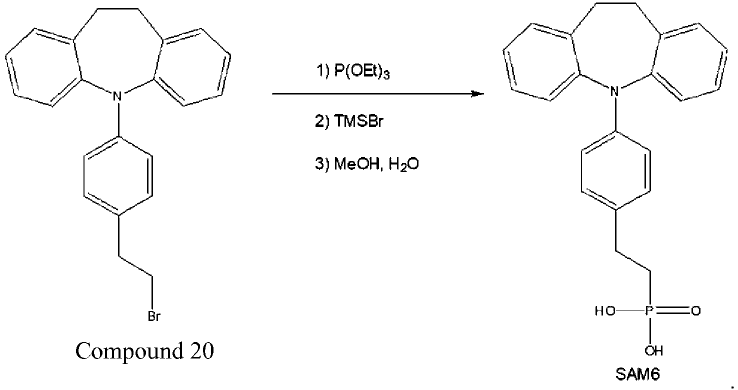

Compound SAM6 was subjected to ¹H NMR spectrum test, with results as follows: ¹H NMR (400 MHz, DMSO-*d₆*) *δ* 7.21-7.16 (m, 6H), 7.10-7.00 (m, 4H), 6.79-6.73 (m, 2H), 2.88 (s, 4H), 2.72-2.66 (m, 2H), 2.03-1.98 (m, 2H).

The above results indicated that the target product SAM6 was successfully obtained in the preparation steps. The yield of the product SAM6 was further calculated using the following formula to be 55%.
$Yield = moles of product SAM 6 / moles of compound 20 \times 100 % .$

The remaining steps were the same as those in Example 1, with specific results shown in Table 1.

### Example 7

Example 7 was substantially the same as Example 1 except that the preparation material of the passivation layer, the compound SAM1, was replaced with the compound SAM7. The specific preparation method was as follows:
Step (1): With reference to the preparation step (1) of the organic compound SAM1 in Example 1, compound 1 was replaced with compound 21 an equimolar amount, and compound 2 was replaced with compound 5 an equimolar amount. Then, the reaction was performed to obtain compound 22. The synthetic route was as follows:

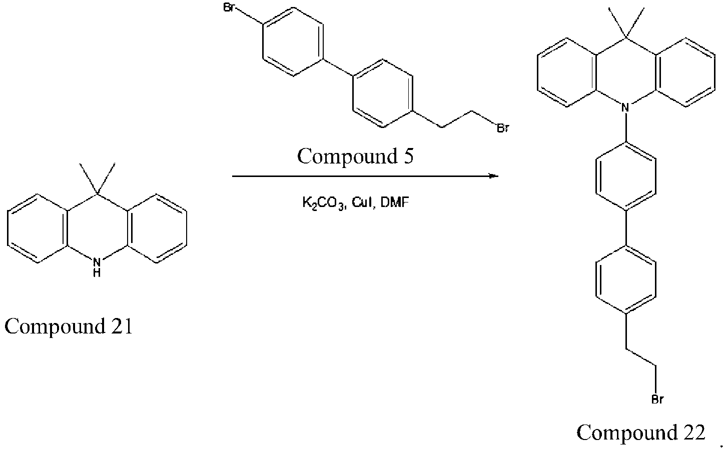

Compound 22 was subjected to ¹H NMR spectrum test, with results as follows: ¹H NMR (400 MHz, DMSO-*d6*) *δ* 7.62 (d, *J* = 7.2 Hz, 2H), 7.55 (d, *J* = 7.2 Hz, 2H), 7.37-7.32 (m, 4H), 7.19-7.14 (m, 6H), 6.98-6.93 (m, 2H), 3.66-3.61 (m, 2H), 3.08-3.03 (m, 2H), 1.69 (s, 6H).

The above results indicated that the target product was successfully obtained in the preparation steps. The yield of compound 22 was further calculated using the following formula to be 75%.
$Yield = moles of compound 22 / moles of compound 21 \times 100 % .$

Step (2): With reference to the preparation step (2) of the organic compound SAM1 in Example 1, compound 3 was replaced with compound 22 an equimolar amount. Then, the reaction was performed to obtain compound SAM7. The synthetic route was as follows:

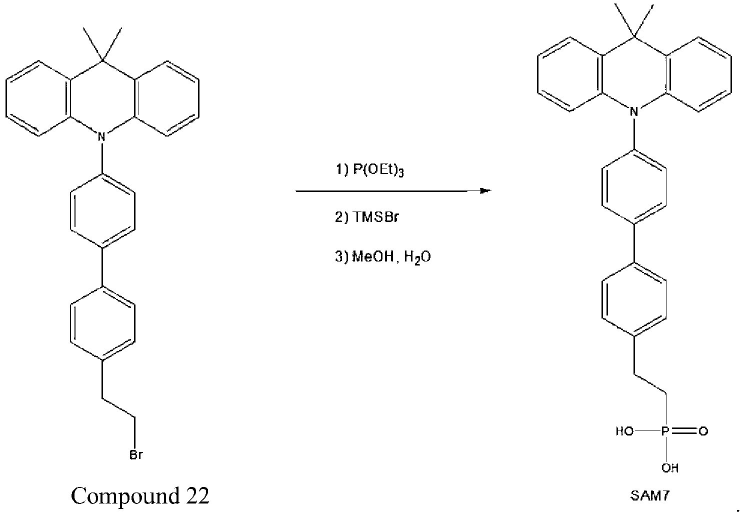

Compound SAM7 was subjected to ¹H NMR spectrum test, with results as follows: ¹H NMR (400 MHz, DMSO-*d₆*) *δ* 7.62 (d, *J* = 7.2 Hz, 2H), 7.55 (d, *J* = 7.2 Hz, 2H), 7.37-7.32 (m, 4H), 7.19-7.14 (m, 6H), 6.98-6.93 (m, 2H), 2.86-2.81 (m, 2H), 2.08-2.03 (m, 2H), 1.69 (s, 6H).

The above results indicated that the target product SAM7 was successfully obtained in the preparation steps. The yield of the product SAM7 was further calculated using the following formula to be 45%.
$Yield = moles of product SAM 7 / moles of compound 22 \times 100 % .$

The remaining steps were the same as those in Example 1, with specific results shown in Table 1.

### Example 8

Example 8 was substantially the same as Example 1 except that the preparation material of the passivation layer, the compound SAM1, was replaced with the compound SAM8. The specific preparation method was as follows:
Step (1): With reference to the preparation step (1) of the organic compound SAM1 in Example 1, compound 1 was replaced with compound 23 in an equimolar amount, and compound 2 was replaced with compound 24 in an equimolar amount. Then, the reaction was performed to obtain compound 25. The synthetic route was as follows:

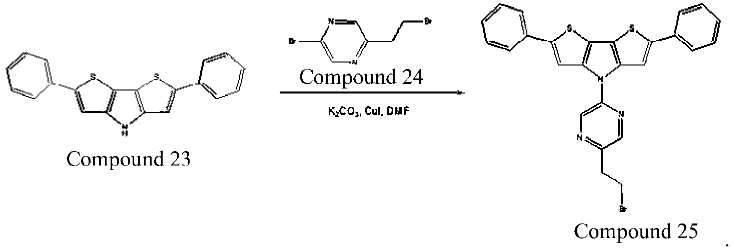

Compound 25 was subjected to ¹H NMR spectrum test, with results as follows: ¹H NMR (400 MHz, DMSO-*d6*) *δ* 8.76 (s, 1H), 8.64 (s, 1H), 7.87-7.82 (m, 4H), 7.49-7.44 (m, 6H), 7.10 (s, 2H), 3.66-3.61 (m, 2H), 3.08-3.03 (m, 2H).

The above results indicated that the target product was successfully obtained in the preparation steps. The yield of compound 25 was further calculated using the following formula to be 75%.
$Yield = moles of compound 25 / moles of compound 23 \times 100 % .$

Step (2): With reference to the preparation step (2) of the organic compound SAM1 in Example 1, compound 3 was replaced with compound 25 in an equimolar amount. Then, the reaction was performed to obtain compound SAM8. The synthetic route was as follows:

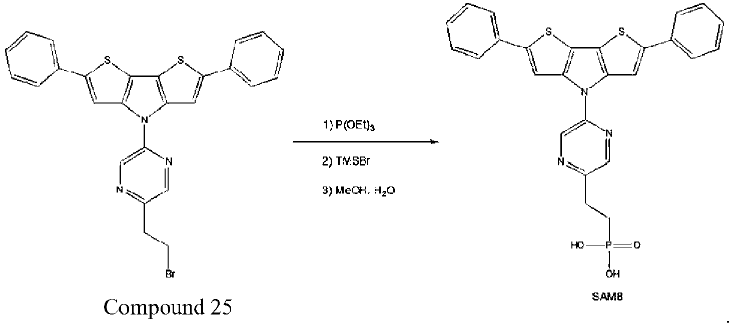

Compound SAM8 was subjected to ¹H NMR spectrum test, with results as follows: ¹H NMR (400 MHz, DMSO-*d₆*) *δ* 8.76 (s, 1H), 8.64 (s, 1H), 7.87-7.82 (m, 4H), 7.49-7.44 (m, 6H), 7.10 (s, 2H), 2.86-2.81 (m, 2H), 2.08-2.00 (m, 2H).

The above results indicated that the target product SAM8 was successfully obtained in the preparation steps. The yield of the product SAM8 was further calculated using the following formula to be 45%.
$Yield = moles of product SAM 8 / moles of compound 25 \times 100 % .$

The remaining steps were the same as those in Example 1, with specific results shown in Table 1.

### Example 9

Example 9 was substantially the same as Example 1 except that the preparation material of the passivation layer, the compound SAM1, was replaced with the compound SAM9. The specific preparation method was as follows:
Step (1): Compound 26 (1 mmol) was dissolved in tetrahydrofuran (10 mL), and a tetrahydrofuran solution (2 mL) of compound 27 (1 mmol) was added dropwise at -78°C. After the resulting mixture was stirred at room temperature for 12 h, the mixture was poured into water (50 mL) and extracted with dichloromethane (50 mL × 3). Then, the solvent was removed, and the resulting oily substance was dissolved in tetrahydrofuran (10 mL). A tetrahydrofuran solution (3 mL) of compound 28 (1.5 mmol) was added dropwise to the above solution at -78°C. After the resulting mixture was subjected to reaction for 12 h, the mixture was poured into water (50 mL) and extracted with dichloromethane (50 mL × 3). Then, the solvent was removed, and the resulting oily substance was added dropwise to a boiling mixture of sodium hydroxide (20 mmol), zinc powder (5 mmol), and water (10 mL). After the mixture was subjected to reaction for 20 h, the mixture was extracted with dichloromethane (50 mL × 3), and the solvent was removed. Then, the resulting product was separated using a silica gel chromatographic column and then purified to obtain compound 29. The synthetic route was as follows:

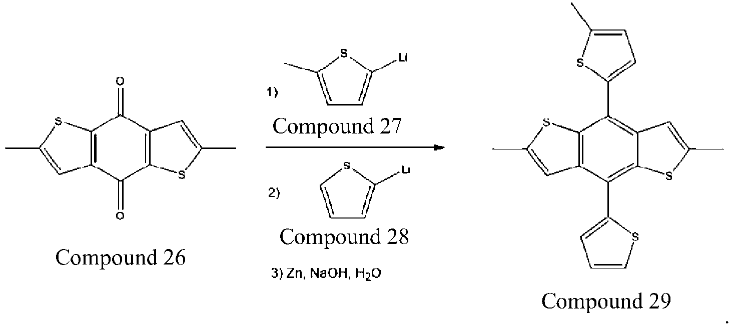

Compound 29 was subjected to ¹H NMR spectrum test, with results as follows: ¹H NMR (400 MHz, DMSO-*d*6) *δ* 7.70 (d, *J* = 7.8 Hz, 1H), 7.40 (d, *J* = 7.8 Hz, 1H), 7.36 (s, 2H), 7.28 (d, *J* = 7.8 Hz, 1H), 7.16-7.11 (m, 1H), 6.83 (d, *J* = 7.8 Hz, 1H), 2.37 (s, 9H).

The above results indicated that the target product was successfully obtained in the preparation steps. The yield of compound 29 was further calculated using the following formula to be 37%.
$Yield = moles of compound 29 / moles of compound 26 \times 100 % .$

Step (2): Compound 29 (1 mmol) was dissolved in tetrahydrofuran (THF, 10 mL), and an n-hexane solution of n-butyllithium (n-BuLi, 2.5 M, 0.5 mL) was added dropwise at -78°C. After the resulting solution was stirred for 2 h, a tetrahydrofuran solution of zinc chloride (1 M, 1.3 mL) was added. After the resulting solution was stirred at room temperature for 2 h, tetrakis(triphenylphosphine)palladium (5% mmol) and compound 2 (1.1 mmol) were added. After the resulting solution was heated at 75°C for 12 h, the resulting product was separated using a silica gel chromatographic column to obtain compound 30. The synthetic route was as follows:

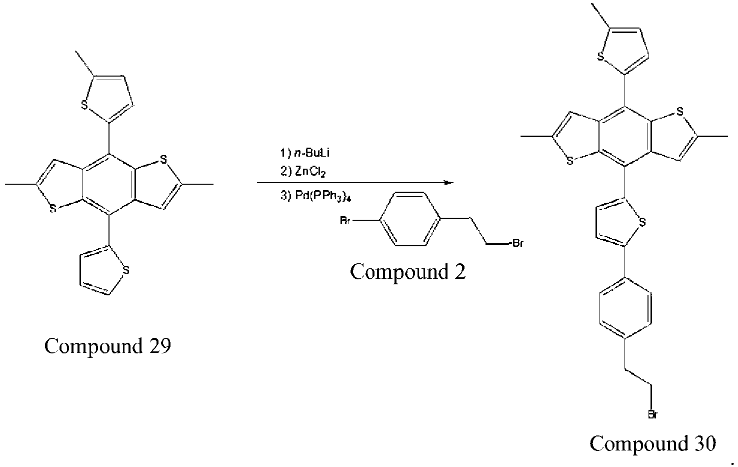

Compound 30 was subjected to ¹H NMR spectrum test, with results as follows: ¹H NMR (400 MHz, DMSO-*d6*) *δ* 7.77 (d, *J* = 7.2 Hz, 2H), 7.36-7.28 (m, 7H), 6.83 (d, *J* = 7.2 Hz, 1H), 3.66-3.60 (m, 2H), 3.08-3.02 (m, 2H), 2.37 (s, 9H).

The above results indicated that the target product was successfully obtained in the preparation steps. The yield of compound 30 was further calculated using the following formula to be 57%.
$Yield = moles of compound 30 / moles of compound 29 \times 100 % .$

Step (3): Compound 30 (1 mmol) and triethyl phosphite (P(OEt)₃, 10 mL) were mixed and then heated for reaction at 160°C for 20 hours under nitrogen protection. The remaining triethyl phosphite was removed using a vacuum distillation method. A crude product was mixed with tributylbromosilane (TMSBr, 0.72 mmol) and 1,4-dioxane (5 mL), and stirred at room temperature for 20 hours under nitrogen protection. Then, the solvent was removed. Methanol (5 mL) was added and stirred for 12 hours. Then, deionized water (1 mL) was added, and solid powder was precipitated. Then, SAM9 was obtained. The synthetic route was as follows:

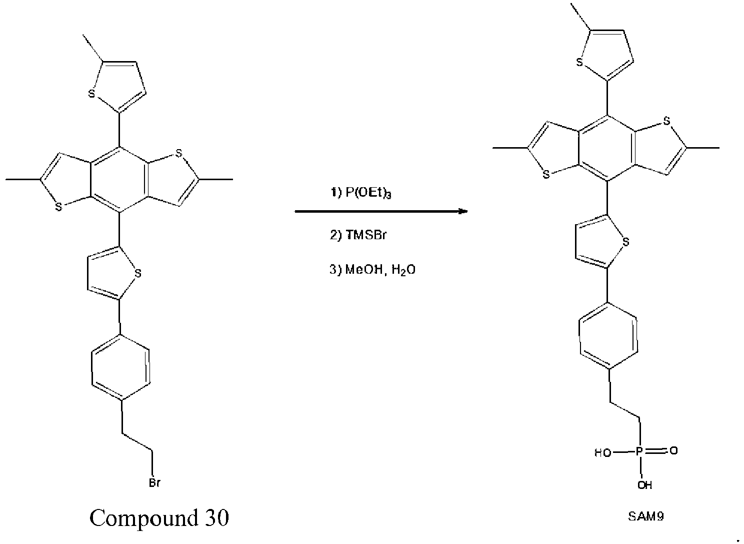

Compound SAM9 was subjected to ¹H NMR spectrum test, with results as follows: ¹H NMR (400 MHz, DMSO-*d₆*) *δ* 7.77 (d, *J* = 7.2 Hz, 2H), 7.36-7.28 (m, 7H), 6.83 (d, *J* = 7.2 Hz, 2H), 3.66-3.60 (m, 2H), 1.73-1.69 (m, 4H), 1.26-1.23 (m, 2H).

The above results indicated that the target product SAM9 was successfully obtained in the preparation steps. The yield of the product SAM9 was further calculated using the following formula to be 58%.
$Yield = moles of product SAM 9 / moles of compound 30 \times 100 % .$

The remaining steps were the same as those in Example 1, with specific results shown in Table 1.

### Example 10

Example 10 was substantially the same as Example 1 except that the preparation material of the passivation layer, the compound SAM1, was replaced with the compound SAM10. The specific preparation method was as follows:

Step (1): Compound 31 (1 mmol) was dissolved in tetrahydrofuran (10 mL), and an n-hexane solution of n-butyllithium (2.5 M, 0.5 mL) was added dropwise at -78°C. After the resulting mixture was stirred for 2 h, compound 32 (1.5 mmol) was added dropwise. Then, the resulting mixture was stirred at room temperature for 12 h, poured into water (50 mL), and extracted with dichloromethane (50 mL × 3). Then, the solvent was removed, and the resulting solid was recrystallized with ethanol to obtain SAM10. The synthetic route was as follows:

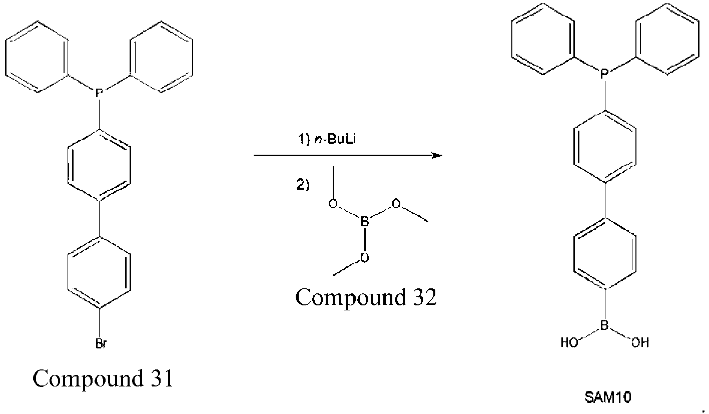

Compound SAM10 was subjected to ¹H NMR spectrum test, with results as follows: ¹H NMR (400 MHz, DMSO-*d₆*) *δ* 7.94 (d, *J* = 7.2 Hz, 2H), 7.81-7.75 (m, 4H), 7.48-7.33 (m, 8H), 7.18-7.12 (m, 4H), 4.22 (s, 2H).

The above results indicated that the target product SAM10 was successfully obtained in the preparation steps. The yield of the product SAM10 was further calculated using the following formula to be 38%.
$Yield = moles of product SAM 10 / moles of compound 31 \times 100 % .$

The remaining steps were the same as those in Example 1, with specific results shown in Table 1.

### Example 11

Example 11 was substantially the same as Example 1 except that the preparation material of the passivation layer, the compound SAM1, was replaced with the compound SAM11. The specific preparation method was as follows:
Step (1): Compound 33 (1 mmol), compound 10 (1.1 mmol), potassium acetate (KOAc, 2 mmol), 1,1'-bis(diphenylphosphino)ferrocene palladium(II) dichloride (Pd(dppf)Cl₂, 5% mmol), and 1,4-dioxane (10 mL) were mixed, heated at 85°C for 12 hours under nitrogen protection, and filtered through diatomaceous earth to obtain a filtrate. After the solvent was removed, a crude product was blended with compound 2 (1.2 mmol), tetrakis(triphenylphosphine)palladium (5% mmol), toluene (10 mL), and an aqueous solution of potassium carbonate (2 M, 10 mL), heated at 110°C for 48 hours under nitrogen protection, and separated using a silica gel chromatographic column to obtain compound 34. The synthetic route was as follows:

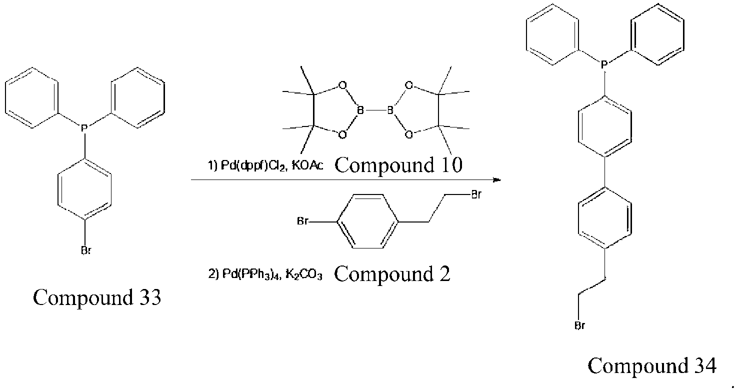

Compound 34 was subjected to ¹H NMR spectrum test, with results as follows: ¹H NMR (400 MHz, DMSO-*d₆*) *δ:* 7.79 *(d, J* = 7.2 Hz, 2H), 7.62 (d, *J* = 7.2 Hz, 2H), 7.47-7.32 (m, 10H), 7.18-7.12 (m, 4H), 3.70-3.66 (m, 2H), 3.08-3.03 (m, 2H).

The above results indicated that the target product was successfully obtained in the preparation steps. The yield of compound 34 was further calculated using the following formula to be 58%.
$Yield = moles of compound 34 / moles of compound 33 \times 100 % .$

(2): Compound 34 (1 mmol) and triethyl phosphite (P(OEt)₃, 10 mL) were mixed and heated at 160°C for 20 hours under nitrogen protection. The remaining triethyl phosphite was removed using a vacuum distillation method. A crude product was mixed with tributylbromosilane (TMSBr, 0.72 mmol) and 1,4-dioxane (5 mL), and stirred at room temperature for 20 hours under nitrogen protection. Then, the solvent was removed. Methanol (5 mL) was added and stirred for 12 hours. Then, deionized water (1 mL) was added, and solid powder was precipitated. Then, SAM11 was obtained. The synthetic route was as follows:

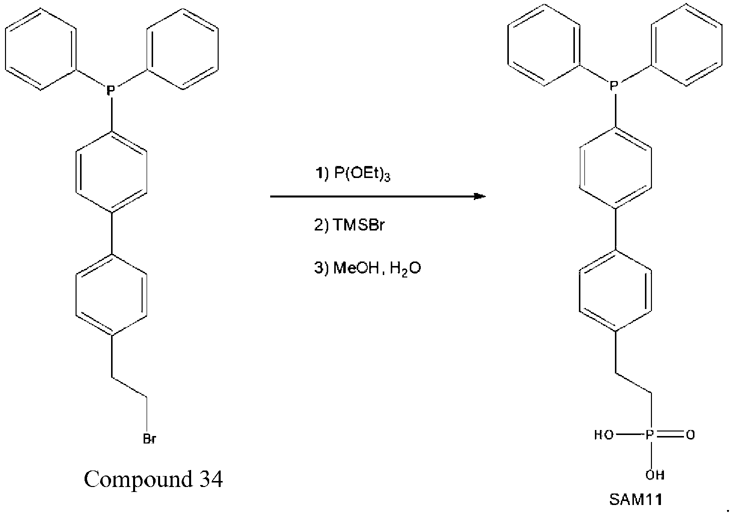

The product SAM11 was subjected to ¹H NMR spectrum test, with test results as follows: ¹H NMR (400 MHz, DMSO-*d₆*) *δ* 7.79 (d, *J* = 7.2 Hz, 1H), 7.79 (d, *J* = 7.2 Hz, 1H), 7.47-7.32 (m, 10H), 7.18-7.12 (m, 4H), 3.70-3.66 (m, 2H), 3.08-3.03 (m, 2H), 2.84-2.78 (m, 2H), 2.04-1.99 (m, 2H).

The above results indicated that the target product SAM11 was successfully obtained in the preparation steps. The yield of the product SAM11 was further calculated using the following formula to be 39%.
$Yield = moles of product SAM 11 / moles of compound 34 \times 100 % .$

The remaining steps were the same as those in Example 1, with specific results shown in Table 1.

### Example 12

Example 12 was substantially the same as Example 1 except that the preparation material of the passivation layer, the compound SAM1, was replaced with the compound SAM12. The specific preparation method was as follows:
Step (1): Compound 35 (1 mmol), compound 10 (1.1 mmol), potassium acetate (KOAc, 2 mmol), 1,1'-bis(diphenylphosphino)ferrocene palladium(II) dichloride (5% mmol), and 1,4-dioxane (10 mL) were mixed, heated at 85°C for 12 hours under nitrogen protection, and filtered through diatomaceous earth to obtain a filtrate. After the solvent was removed, a crude product was mixed with compound 11 (1.2 mmol), tetrakis(triphenylphosphine)palladium (5% mmol), toluene (10 mL), and an aqueous solution of potassium carbonate (2 M, 10 mL), heated at 110°C for 48 hours under nitrogen protection, and separated using a silica gel chromatographic column to obtain compound 36. The synthetic route was as follows:

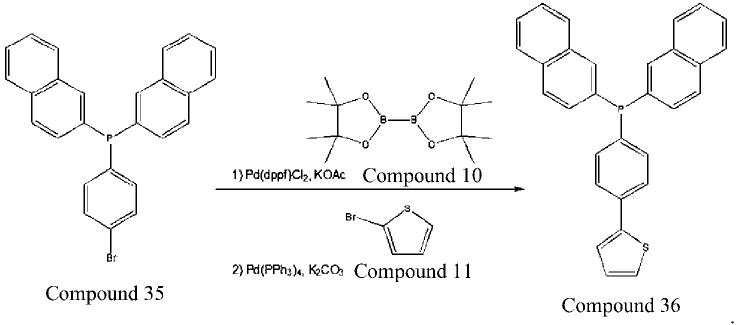

The remaining steps were the same as those in Example 1, with specific results shown in Table 1.

Compound 36 was subjected to ¹H NMR spectrum test, with results as follows: ¹H NMR (400 MHz, DMSO-*d₆*) *δ* 8.03-7.99 (m, 6H), 7.84-7.79 (m, 4H), 7.70 (d, *J* = 7.2 Hz, 1H), 7.61-7.57 (m, 4H), 7.48 (d, *J* = 7.2 Hz, 2H), 7.40-7.34 (m, 3H), 7.16-7.10 (m, 1H).

The above results indicated that the target product was successfully obtained in the preparation steps. The yield of compound 36 was further calculated using the following formula to be 57%.
$Yield = moles of compound 36 / moles of compound 35 \times 100 % .$

Step (2): Compound 36 (1 mmol) was dissolved in tetrahydrofuran (THF, 10 mL), and an n-hexane solution of n-butyllithium (n-BuLi, 2.5 M, 0.5 mL) was added dropwise at -78°C. After the resulting solution was stirred for 2 h, a tetrahydrofuran solution of zinc chloride (1 M, 1.3 mL) was added. After the resulting solution was stirred at room temperature for 2 h, tetrakis(triphenylphosphine)palladium (5% mmol) and compound 2 (1.1 mmol) were added. After the resulting solution was heated at 75°C, the resulting product was separated using a silica gel chromatographic column to obtain compound 37. The synthetic route was as follows:

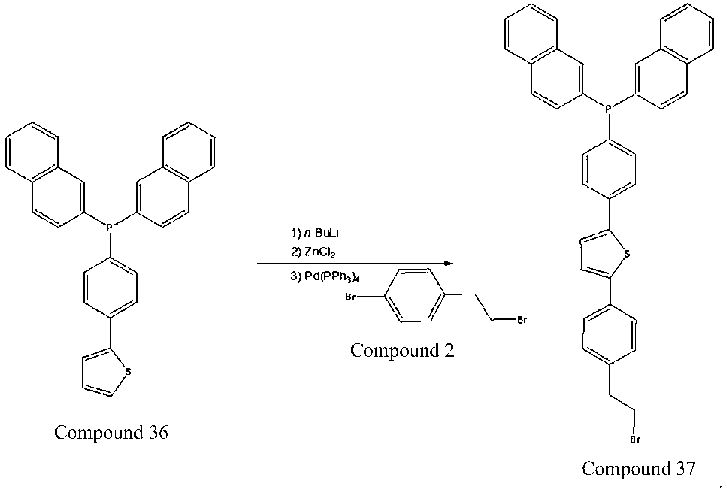

Compound 37 was subjected to ¹H NMR spectrum test, with results as follows: ¹H NMR (400 MHz, DMSO-*d₆*) *δ* 8.03-7.99 (m, 6H), 7.84-7.77 (m, 6H), 7.61-7.57 (m, 4H), 7.48 (d, *J* = 7.2 Hz, 2H), 7.35-7.30 (m, 6H), 3.66-3.60 (m, 2H), 3.08-3.03 (m, 2H).

The above results indicated that the target product was successfully obtained in the preparation steps. The yield of the product 37 was further calculated using the following formula to be 58%.
$Yield = moles of product 37 / moles of compound 36 \times 100 % .$

Step (3): Compound 37 (1 mmol) and triethyl phosphite (P(OEt)₃, 10 mL) were mixed and then heated at 160°C for 20 hours under nitrogen protection. The remaining triethyl phosphite was removed using a vacuum distillation method. A crude product was mixed with tributylbromosilane (TMSBr, 0.72 mmol) and 1,4-dioxane (5 mL), and stirred at room temperature for 20 hours under nitrogen protection. Then, the solvent was removed. Methanol (5 mL) was added and stirred for 12 hours. Then, deionized water (1 mL) was added, and solid powder was precipitated. Then, SAM12 was obtained. The synthetic route was as follows:

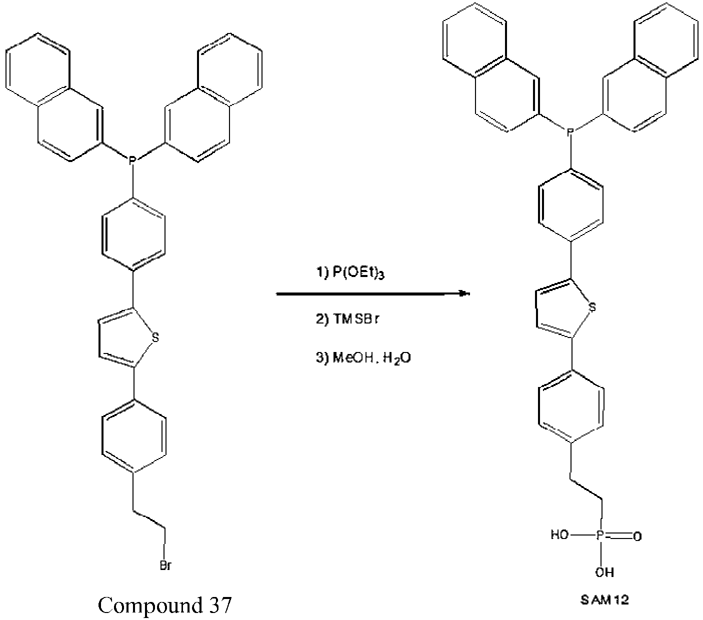

Compound SAM12 was subjected to ¹H NMR spectrum test, with results as follows: ¹H NMR (400 MHz, DMSO-*d₆*) *δ* 8.03-7.99 (m, 6H), 7.84-7.77 (m, 6H), 7.61-7.57 (m, 4H), 7.48 (d, *J* = 7.2 Hz, 2H), 7.35-7.30 (m, 6H), 2.84-2.78 (m, 2H), 2.04-1.99 (m, 2H).

The above results indicated that the target product SAM12 was successfully obtained in the preparation steps. The yield of the product SAM12 was further calculated using the following formula to be 46%.
$Yield = moles of product SAM 12 / moles of compound 37 \times 100 % .$

The remaining steps were the same as those in Example 1, with specific results shown in Table 1.

### Example 13

Example 13 was substantially the same as Example 1 except that the preparation material of the passivation layer, the compound SAM1, was replaced with the compound SAM13. The specific preparation method was as follows:
Step (1): Compound 38 (1 mmol), compound 10 (1.1 mmol), potassium acetate (KOAc, 2 mmol), 1,1'-bis(diphenylphosphino)ferrocene palladium(II) dichloride (Pd(dppf)Cl₂, 5% mmol), and 1,4-dioxane (10 mL) were mixed, heated at 85°C for 12 hours under nitrogen protection, and filtered through diatomaceous earth to obtain a filtrate. After the solvent was removed, a crude product was mixed with compound 39 (1.2 mmol), tetrakis(triphenylphosphine)palladium (5% mmol), toluene (10 mL), and an aqueous solution of potassium carbonate (2 M, 10 mL), heated at 110°C for 48 hours under nitrogen protection, and separated using a silica gel chromatographic column to obtain compound 40. The synthetic route was as follows:

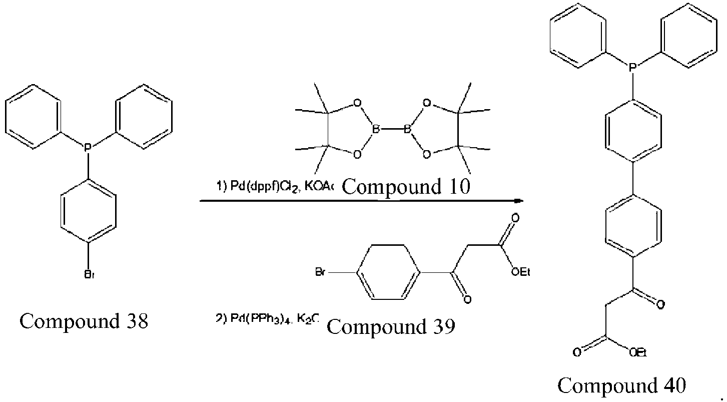

Compound 40 was subjected to ¹H NMR spectrum test, with results as follows: ¹H NMR (400 MHz, DMSO-*d₆*) *δ* 8.00 (d, *J* = 7.2 Hz, 2H), 7.83-7.77 (m, 4H), 7.48-7.43 (m, 6H), 7.34 (d, *J* = 7.2 Hz, 2H), 7.18-7.13 (m, 4H), 4.18-4.12 (m, 2H), 3.54-3.48 (m, 2H), 1.28-1.23 (m, 3H).

The above results indicated that the target product was successfully obtained in the preparation steps. The yield of compound 40 was further calculated using the following formula to be 67%.
$Yield = moles of compound 40 / moles of compound 38 \times 100 % .$

Step (2): Compound 40 (1 mmol) was dissolved in tetrahydrofuran (10 mL) and mixed with an aqueous solution of sodium hydroxide (NaOH, 2 M, 10 mL). The resulting solution was heated at 75°C for 20 h. Then, a concentrated hydrochloric acid was added dropwise until the pH of the solution was less than 1. The precipitate was collected to obtain SAM13. The synthetic route was as follows:

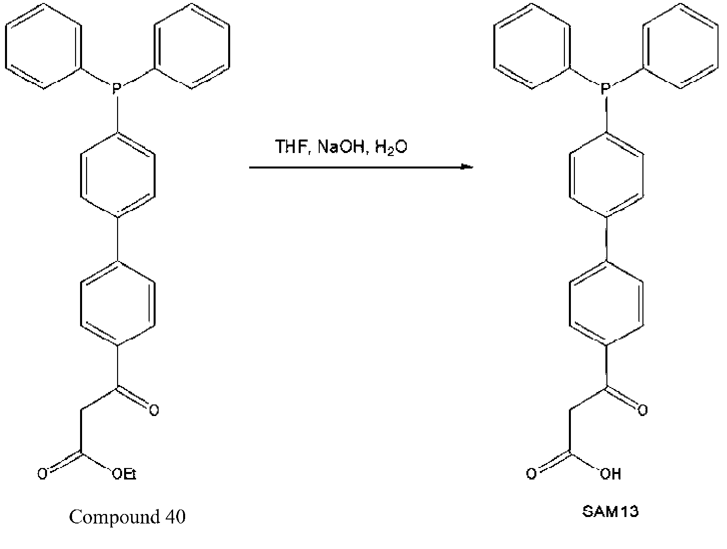

Compound SAM13 was subjected to ¹H NMR spectrum test, with results as follows: ¹H NMR (400 MHz, DMSO-*d₆*) *δ* 13.82 (s, 1H), 8.00 (d, *J* = 7.2 Hz, 1H), 7.83-7.77 (m, 4H), 7.48-7.43 (m, 6H), 7.34 (d, *J* = *7.2* Hz, 2H), 7.18-7.13 (m, 4H), 3.54-3.48 (m, 2H).

The above results indicated that the target product SAM13 was successfully obtained in the preparation steps. The yield of the product SAM13 was further calculated using the following formula to be 78%.
$Yield = moles of product SAM 13 / moles of compound 40 \times 100 % .$

The remaining steps were the same as those in Example 1, with specific results shown in Table 1.

### Example 14

Example 14 was substantially the same as Example 1 except that the preparation material of the passivation layer, the compound SAM1, was replaced with the compound SAM14. The specific preparation method was as follows:
Step (1): Compound 14 (1 mmol), compound 41 (1.1 mmol), tetrakis(triphenylphosphine)palladium (5% mmol), toluene (10 mL), and an aqueous solution of potassium carbonate (2 M, 10 mL) were mixed, heated at 110°C for 48 hours under nitrogen protection, and separated using a silica gel chromatographic column to obtain compound 42. The synthetic route was as follows:

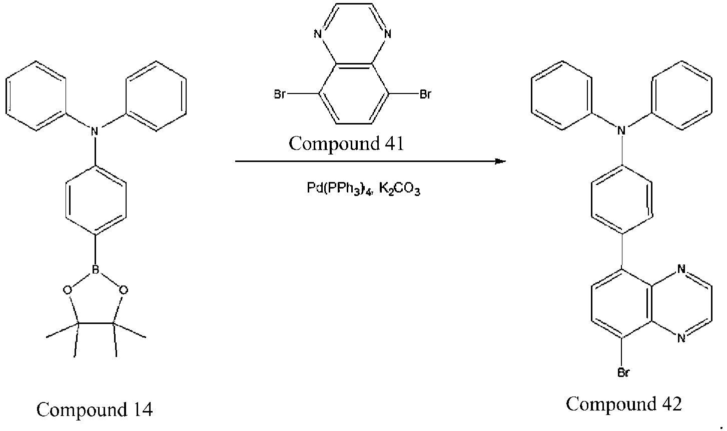

Compound 42 was subjected to ¹H NMR spectrum test, with results as follows: ¹H NMR (400 MHz, DMSO-*d₆*) *δ* 8.74 (s, 2H), 8.04 (d, *J* = 7.6 Hz, 1H), 7.91 (d, *J* = 7.6 Hz, 1H), 7.55 (d, *J* = 7.6 Hz, 2H), 7.37 (d, *J* = 7.6 Hz, 2H), 7.27-7.21 (m, 4H), 7.09-7.05 (m, 6H).

The above results indicated that the target product was successfully obtained in the preparation steps. The yield of compound 42 was further calculated using the following formula to be 53%.
$Yield = moles of compound 42 / moles of compound 14 \times 100 % .$

Step (2): Compound 42 (1 mmol), compound 10 (1.1 mmol), potassium acetate (KOAc, 2 mmol), 1,1'-bis(diphenylphosphino)ferrocene palladium(II) dichloride (5% mmol), and 1,4-dioxane (10 mL) were mixed, heated at 85°C for 12 hours under nitrogen protection, and filtered through diatomaceous earth to obtain a filtrate. After the solvent was removed, a crude product was mixed with compound 43 (1.2 mmol), tetrakis(triphenylphosphine)palladium (5% mmol), toluene (10 mL), and an aqueous solution of potassium carbonate (2 M, 10 mL), heated at 110°C for 48 hours under nitrogen protection, and separated using a silica gel chromatographic column to obtain compound 44. The synthetic route was as follows:

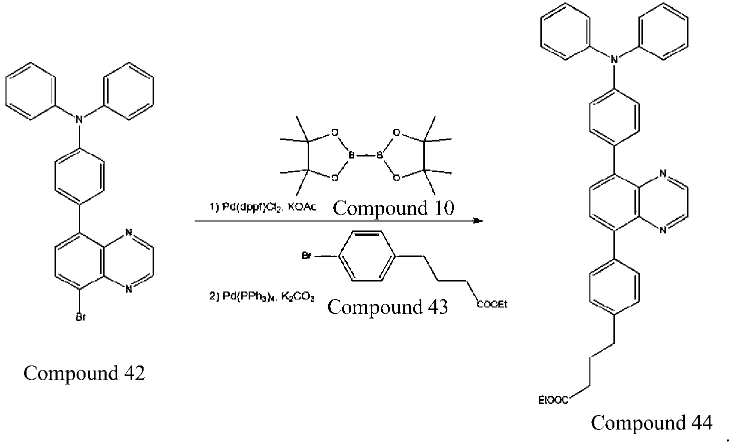

Compound 44 was subjected to ¹H NMR spectrum test, with results as follows: ¹H NMR (400 MHz, DMSO-*d₆*) *δ* 8.76 (s, 2H), 8.23 (d, *J* = 7.6 Hz, 2H), 7.57 (d, *J* = 7.6 Hz, 2H), 7.39-7.36 (m, 4H), 7.28-7.24 (m, 6H), 7.09-7.04 (m, 6H), 4.04-3.99 (m, 2H), 2.65-2.62 (m, 2H), 2.33-2.31 (m, 2H), 1.83-1.80 (m, 2H), 1.09-1.04 (m, 3H).

The above results indicated that the target product was successfully obtained in the preparation steps. The yield of compound 44 was further calculated using the following formula to be 47%.
$Yield = moles of compound 44 / moles of compound 42 \times 100 % .$

Step (3): Compound 44 (1 mmol) was dissolved in tetrahydrofuran (10 mL) and mixed with an aqueous solution of sodium hydroxide (NaOH, 2 M, 10 mL). The resulting solution was heated at 75°C for 20 h. Then, a concentrated hydrochloric acid was added dropwise until the pH of the solution was less than 1. The precipitate was collected to obtain SAM14. The synthetic route was as follows:

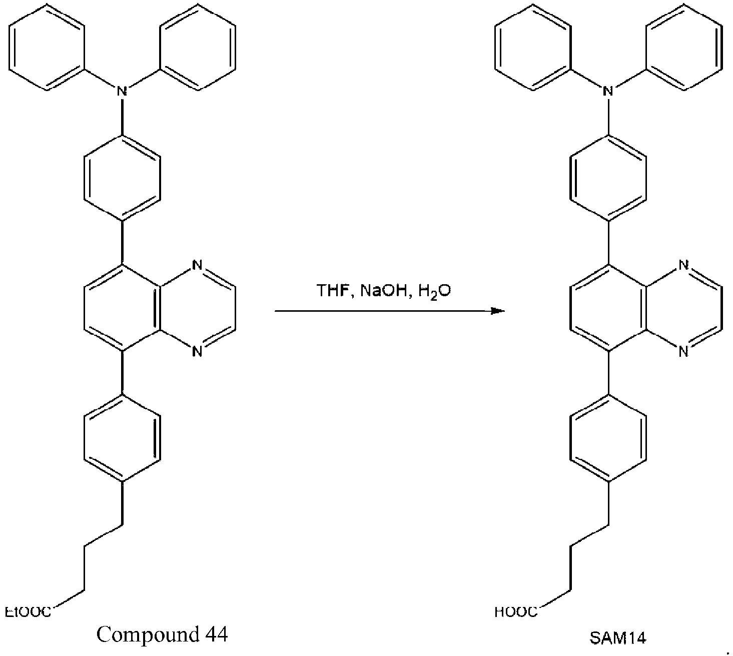

SAM14 was subjected to ¹H NMR spectrum test, with results as follows: ¹H NMR (400 MHz, DMSO-*d*₆) *δ* 12.01 (s, 1H), 8.76 (s, 2H), 8.23 (d, *J* = 7.6 Hz, 2H), 7.57 (d, *J* = 7.6 Hz, 2H), 7.39-7.36 (m, 4H), 7.28-7.24 (m, 6H), 7.09-7.04 (m, 6H), 2.65-2.62 (m, 2H), 2.33-2.29 (m, 2H), 1.73-1.69 (m, 2H).

The above results indicated that the target product was successfully obtained in the preparation steps. The yield of SAM14 was further calculated using the following formula to be 47%.$Yield = moles of SAM 14 / moles of compound 44 \times 100 % .$

### Example 15

Example 15 was substantially the same as Example 1 except that the preparation material of the passivation layer, the compound SAM1, was replaced with the compound SAM15. The specific preparation method was as follows:
Step (1): With reference to the preparation step (1) of the organic compound SAM1 in Example 1, compound 1 was replaced with compound 45 in an equimolar amount, and compound 2 was replaced with compound 8 in an equimolar amount. Then, the reaction was performed to obtain compound 46. The synthetic route was as follows:

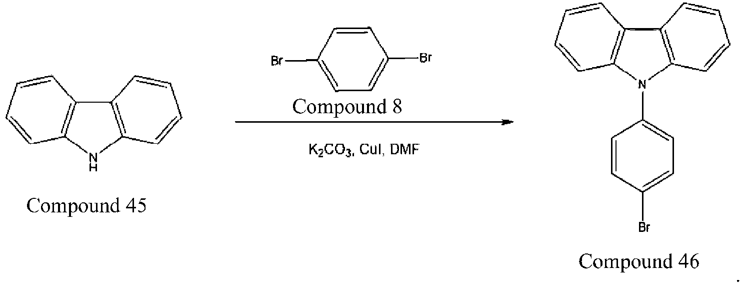

Compound 46 was subjected to ¹H NMR spectrum test, with results as follows: ¹H NMR (400 MHz, DMSO-*d₆*) *δ* 8.53 (d, *J* = 7.6 Hz, 2H), 7.92 (d, *J* = 7.6 Hz, 2H), 7.68 (d, *J* = 7.6 Hz, 2H), 7.62 (d, *J* = 7.6 Hz, 2H), 7.36-7.32 (m, 2H), 7.18-7.14 (m, 2H).

The above results indicated that the target product was successfully obtained in the preparation steps. The yield of compound 46 was further calculated using the following formula to be 87%.
$Yield = moles of compound 46 / moles of compound 45 \times 100 % .$

Step (2): Compound 46 (1 mmol), compound 10 (1.1 mmol), potassium acetate (KOAc, 2 mmol), 1,1'-bis(diphenylphosphino)ferrocene palladium(II) dichloride (5% mmol), and 1,4-dioxane (10 mL) were mixed, heated at 85°C for 12 hours under nitrogen protection, and filtered through diatomaceous earth to obtain a filtrate. After the solvent was removed, a crude product was mixed with compound 47 (1.2 mmol), tetrakis(triphenylphosphine)palladium (5% mmol), toluene (10 mL), and an aqueous solution of potassium carbonate (2 M, 10 mL), heated at 110°C for 48 hours under nitrogen protection, and separated using a silica gel chromatographic column to obtain compound 48. The synthetic route was as follows:

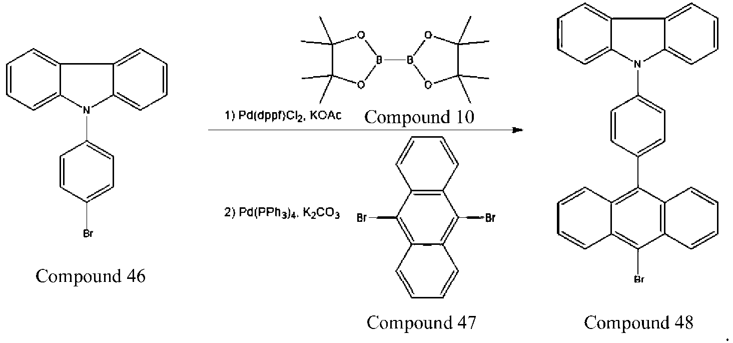

Compound 48 was subjected to ¹H NMR spectrum test, with results as follows: ¹H NMR (400 MHz, DMSO-*d₆*) *δ* 8.36-8.31 (m, 4H), 8.23-8.17 (m, 2H), 7.94-7.88 (m, 6H), 7.46-7.42 (m, 6H), 7.20-7.16 (m, 2H).

The above results indicated that the target product was successfully obtained in the preparation steps. The yield of compound 48 was further calculated using the following formula to be 63%.
$Yield = moles of compound 48 / moles of compound 46 \times 100 % .$

Step (3): Compound 48 (1 mmol), compound 10 (1.1 mmol), potassium acetate (KOAc, 2 mmol), 1,1'-bis(diphenylphosphino)ferrocene palladium(II) dichloride (5% mmol), and 1,4-dioxane (10 mL) were mixed, heated at 85°C for 12 hours under nitrogen protection, and filtered through diatomaceous earth to obtain a filtrate. After the solvent was removed, a crude product was mixed with compound 49 (1.2 mmol), tetrakis(triphenylphosphine)palladium (5% mmol), toluene (10 mL), and an aqueous solution of potassium carbonate (2 M, 10 mL), heated at 110°C for 48 hours under nitrogen protection, and separated using a silica gel chromatographic column to obtain compound 50. The synthetic route was as follows:

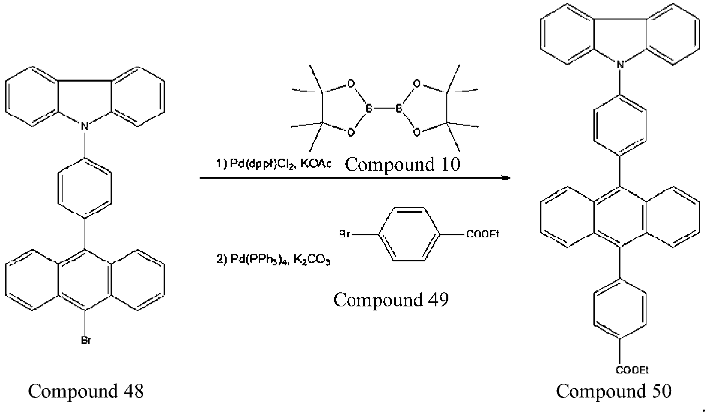

Compound 50 was subjected to ¹H NMR spectrum test, with results as follows: ¹H NMR (400 MHz, DMSO-*d₆*) *δ* 8.36-8.31 (m, 4H), 8.23-8.17 (m, 2H), 8.02 (d, *J* = 7.6 Hz, 2H), 7.94-7.88 (m, 6H), 7.75 (d, *J* = 7.6 Hz, 2H), 7.46-7.42 (m, 6H), 7.20-7.16 (m, 2H), 4.32-4.28 (m, 2H), 1.33-1.30 (m, 3H).

The above results indicated that the target product was successfully obtained in the preparation steps. The yield of compound 50 was further calculated using the following formula to be 71%.
$Yield = moles of compound 50 / moles of compound 48 \times 100 % .$

Step (4): Compound 50 (1 mmol) was dissolved in tetrahydrofuran (10 mL) and mixed with an aqueous solution of sodium hydroxide (NaOH, 2 M, 10 mL). The resulting solution was heated at 75°C for 20 h. Then, a concentrated hydrochloric acid was added dropwise until the pH of the solution was less than 1. The precipitate was collected to obtain SAM15. The synthetic route was as follows:

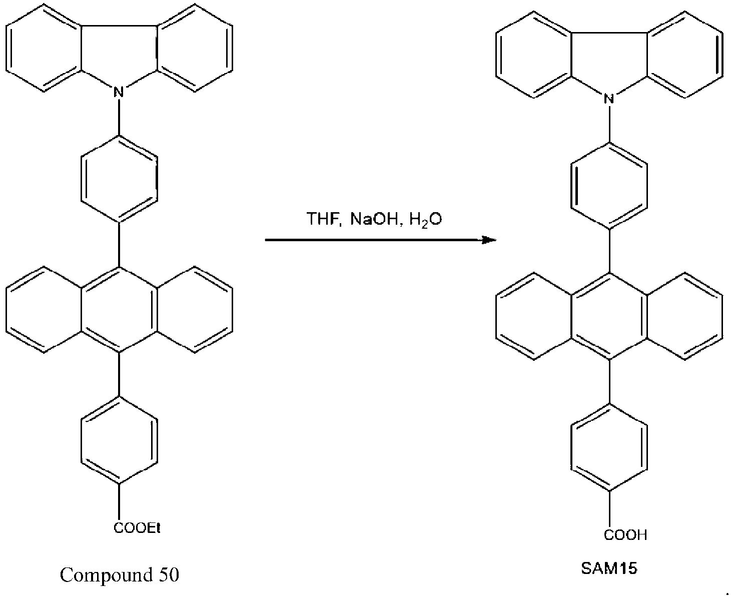

SAM15 was subjected to ¹H NMR spectrum test, with results as follows: ¹H NMR (400 MHz, DMSO-*d*₆) *δ* 12.01 (s, 1H), 8.36-8.31 (m, 4H), 8.23-8.17 (m, 2H), 8.02 (d, *J* = 7.6 Hz, 2H), 7.94-7.88 (m, 6H), 7.75 (d,*J* = 7.6 Hz, 2H), 7.46-7.42 (m, 6H), 7.20-7.16 (m, 2H).

The above results indicated that the target product was successfully obtained in the preparation steps. The yield of SAM15 was further calculated using the following formula to be 77%.
$Yield = moles of SAM 15 / moles of compound 50 \times 100 % .$

### Example 16

Example 16 was substantially the same as Example 1 except that the preparation material of the passivation layer, the compound SAM1, was replaced with the compound SAM16. The specific preparation method was as follows:
Step (1): Compound 51 (1 mmol), compound 52 (1.1 mmol), tetrakis(triphenylphosphine)palladium (5% mmol), toluene (10 mL), and an aqueous solution of potassium carbonate (2 M, 10 mL) were mixed, heated at 110°C for 48 hours under nitrogen protection, and separated using a silica gel chromatographic column to obtain compound 53. The synthetic route was as follows:

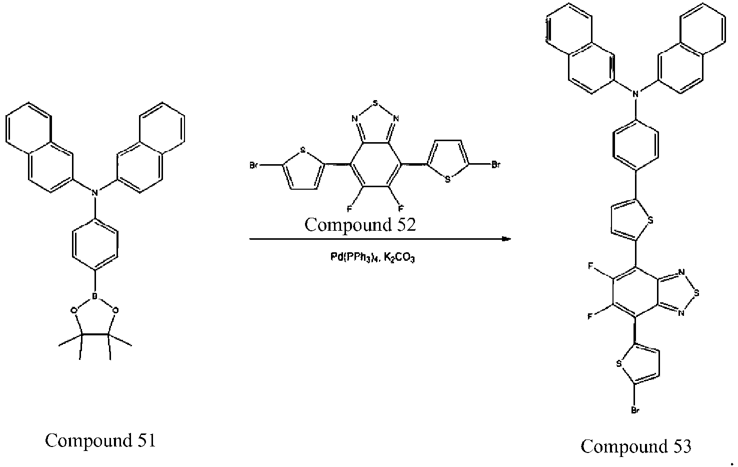

Compound 53 was subjected to ¹H NMR spectrum test, with results as follows: ¹H NMR (400 MHz, DMSO-*d₆*) *δ* 7.77 (d, *J* = 7.6 Hz, 2H), 7.71 (d, *J* = 7.6 Hz, 2H), 7.55 (d, *J* = 7.6 Hz, 2H), 7.45-7.39 (m, 8H), 7.37 (d, *J* = 7.6 Hz, 2H), 7.30 (s, 2H), 7.11-7.03 (m, 4H).

The above results indicated that the target product was successfully obtained in the preparation steps. The yield of compound 53 was further calculated using the following formula to be 53%.
$Yield = moles of compound 53 / moles of compound 51 \times 100 % .$

Step (2): Compound 53 (1 mmol) was dissolved in tetrahydrofuran (THF, 10 mL), and an n-hexane solution of n-butyllithium (n-BuLi, 2.5 M, 0.5 mL) was added dropwise at -78°C. After the resulting solution was stirred for 2 h, a tetrahydrofuran solution of zinc chloride (1 M, 1.3 mL) was added. After the resulting solution was stirred at room temperature for 2 h, tetrakis(triphenylphosphine)palladium (5% mmol) and compound 2 (1.1 mmol) were added. After the resulting solution was heated at 75°C for 12 h, the resulting product was separated using a silica gel chromatographic column to obtain compound 54. The synthetic route was as follows:

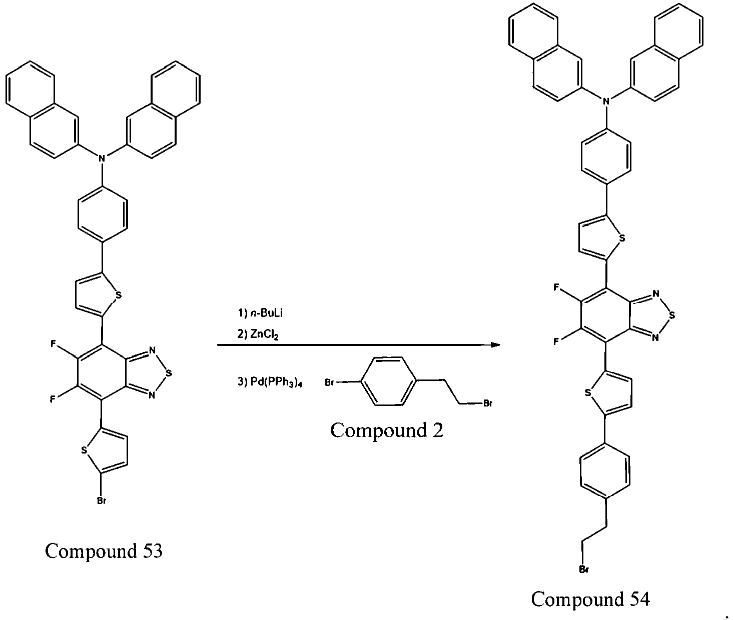

Compound 54 was subjected to ¹H NMR spectrum test, with results as follows: ¹H NMR (400 MHz, DMSO-*d₆*) *δ* 7.77 (d, *J* = 7.6 Hz, 4H), 7.71 (d, *J* = 7.6 Hz, 2H), 7.55 (d, *J* = 7.6 Hz, 2H), 7.45-7.39 (m, 8H), 7.37 (d, *J* = 7.6 Hz, 2H), 7.32 (d, *J* = 7.6 Hz, 2H), 7.30 (s, 2H), 7.11-7.03 (m, 4H), 3.65-3.61 (m, 2H), 3.07-3.03 (m, 2H).

The above results indicated that the target product was successfully obtained in the preparation steps. The yield of compound 54 was further calculated using the following formula to be 47%.
$Yield = moles of compound 54 / moles of compound 53 \times 100 % .$

Step (3): Compound 54 (1 mmol) and triethyl phosphite (P(OEt)₃, 10 mL) were mixed and then heated at 160°C for 20 hours under nitrogen protection. The remaining triethyl phosphite was removed using a vacuum distillation method. A crude product was mixed with tributylbromosilane (TMSBr, 0.72 mmol) and 1,4-dioxane (5 mL), and stirred at room temperature for 20 hours under nitrogen protection. Then, the solvent was removed. Methanol (5 mL) was added and stirred for 12 hours. Then, deionized water (1 mL) was added, and solid powder was precipitated. Then, SAM16 was obtained. The synthetic route was as follows:

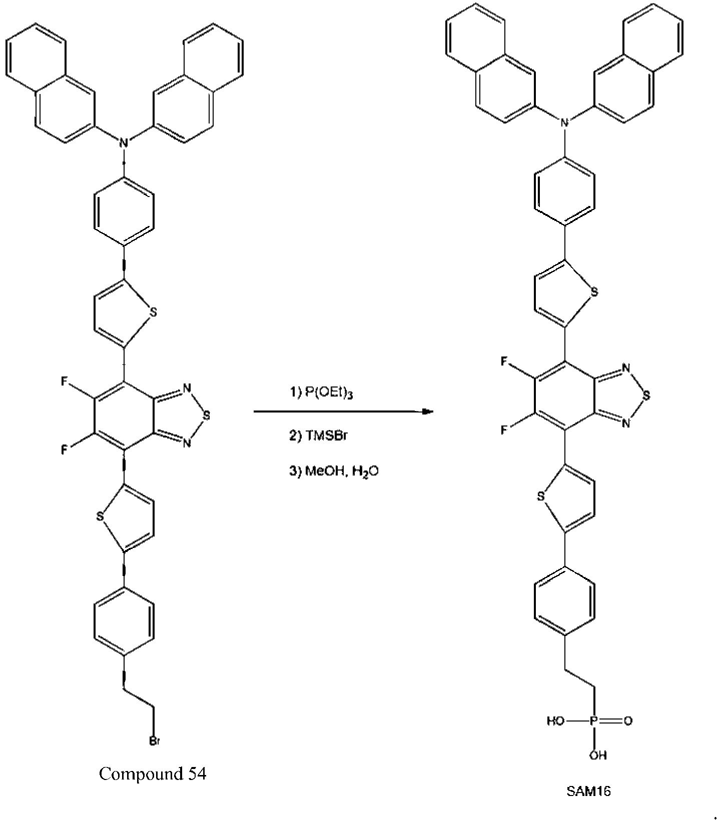

SAM16 was subjected to ¹H NMR spectrum test, with results as follows: ¹H NMR (400 MHz, DMSO-*d*₆) *δ* 7.77 (d, *J* = 7.6 Hz, 4H), 7.71 (d, *J* = 7.6 Hz, 2H), 7.55 (d, *J* = 7.6 Hz, 2H), 7.45-7.39 (m, 8H), 7.37 (d, *J* = 7.6 Hz, 2H), 7.32 (d, *J* = 7.6 Hz, 2H), 7.30 (s, 2H), 7.11-7.03 (m, 4H), 2.45-2.42 (m, 2H), 1.64-1.60 (m, 2H).

The above results indicated that the target product was successfully obtained in the preparation steps. The yield of SAM16 was further calculated using the following formula to be 47%.
$Yield = moles of SAM 16 / moles of compound 54 \times 100 % .$

### Example 17

Example 17 was substantially the same as Example 1 except that the preparation material of the passivation layer, the compound SAM1, was replaced with the compound SAM17. The specific preparation method was as follows:
Step (1): With reference to the preparation step (1) of the organic compound SAM1 in Example 1, compound 1 was replaced with compound 55 in an equimolar amount, and compound 2 was replaced with compound 56 in an equimolar amount. Then, the reaction was performed to obtain compound 57. The synthetic route was as follows:

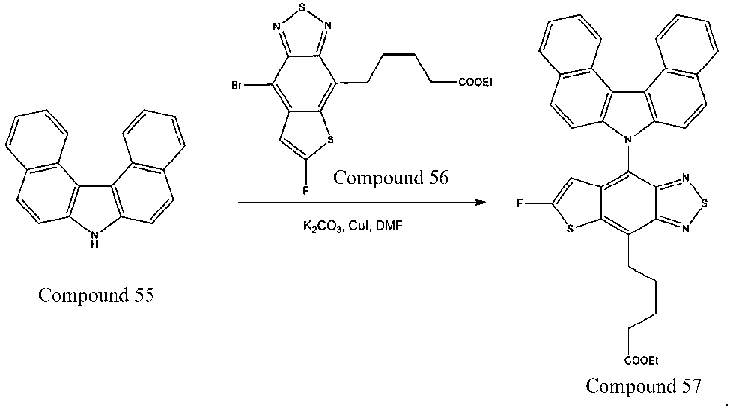

Compound 57 was subjected to ¹H NMR spectrum test, with results as follows: ¹H NMR (400 MHz, DMSO-*d*₆) *δ* 8.55 (d, *J* = 7.6 Hz, 2H), 7.99-7.94 (m, 6H), 7.61-7.53 (m, 4H), 6.67-6.61 (m, 1H), 4.03-3.99 (m, 2H), 2.65-2.61 (m, 2H), 2.36-2.32 (m, 2H), 1.64-1.59 (m, 4H), 1.10-1.04 (m, 3H).

The above results indicated that the target product was successfully obtained in the preparation steps. The yield of compound 57 was further calculated using the following formula to be 36%.
$Yield = moles of compound 57 / moles of compound 55 \times 100 % .$

Step (2): Compound 57 (1 mmol) was dissolved in tetrahydrofuran (10 mL) and mixed with an aqueous solution of sodium hydroxide (NaOH, 2 M, 10 mL). The resulting solution was heated at 75°C for 20 h. Then, a concentrated hydrochloric acid was added dropwise until the pH of the solution was less than 1. The precipitate was collected to obtain SAM17. The synthetic route was as follows:

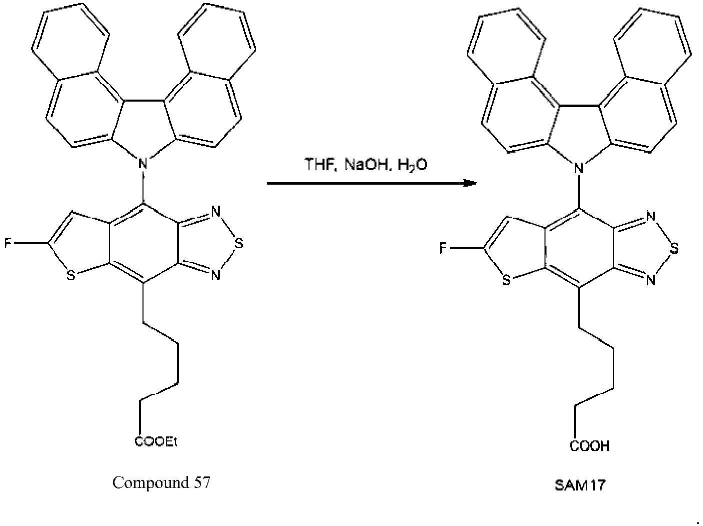

Compound SAM17 was subjected to ¹H NMR spectrum test, with results as follows: ¹H NMR (400 MHz, DMSO-*d*₆) *δ* 11.87 (s, 1H), 8.55 (d, *J* = 7.6 Hz, 2H), 7.99-7.94 (m, 6H), 7.61-7.53 (m, 4H), 6.67-6.61 (m, 1H), 2.65-2.61 (m, 2H), 2.26-2.23 (m, 2H), 1.64-1.59 (m, 4H).

The above results indicated that the target product was successfully obtained in the preparation steps. The yield of the product SAM17 was further calculated using the following formula to be 77%.
$Yield = moles of SAM 17 / moles of compound 57 \times 100 % .$

### Comparative Example 1

Comparative Example 1 was substantially the same as Example 1 except that no passivation layer was disposed during preparation of the solar cell.

The remaining steps were the same as those in Example 1, with specific results shown in Table 1.

### Comparative Example 2

Comparative Example 2 was substantially the same as Example 1 except that the preparation material of the passivation layer, the compound SAM1, was replaced with the compound SAM18. The specific structure was as follows:

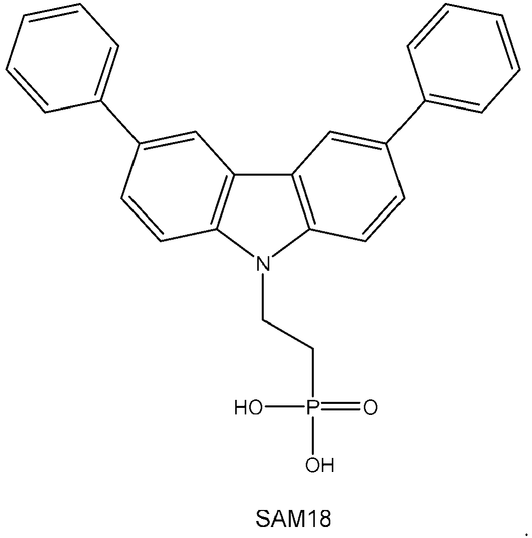

The remaining steps were the same as those in Example 1, with specific results shown in Table 1.

### Comparative Example 3

Comparative Example 2 was substantially the same as Example 1 except that the preparation material of the passivation layer, the compound SAM1, was replaced with the compound SAM19. The specific structure was as follows:

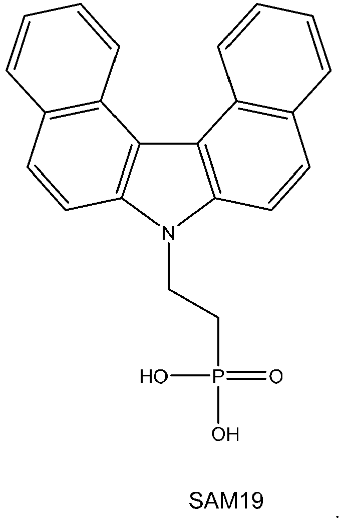

The remaining steps were the same as those in Example 1, with specific results shown in Table 1.

Relevant physical parameters and test results in examples and comparative examples are shown in Table 1.

**Table 1**

| | Material of passivation layer | Optimal efficiency | P30 |
|---|---|---|---|
| Example 1 | SAM1 | 25.87% | 25.28% |
| Example 2 | SAM2 | 25.86% | 25.15% |
| Example 3 | SAM3 | 25.95% | 25.00% |
| Example 4 | SAM4 | 25.66% | 25.14% |
| Example 5 | SAM5 | 25.46% | 25.04% |
| Example 6 | SAM6 | 25.88% | 24.88% |
| Example 7 | SAM7 | 25.39% | 24.89% |
| Example 8 | SAM8 | 25.69% | 25.16% |
| Example 9 | SAM9 | 25.61% | 25.01% |
| Example 10 | SAM10 | 25.76% | 25.23% |
| Example 11 | SAM11 | 25.84% | 25.09% |
| Example 12 | SAM12 | 25.91% | 25.16% |
| Example 13 | SAM13 | 25.73% | 24.04% |
| Example 14 | SAM14 | 25.64% | 25.11% |
| Example 15 | SAM15 | 25.37% | 24.89% |
| Example 16 | SAM16 | 25.22% | 24.65% |
| Example 17 | SAM17 | 25.68% | 24.76% |
| Comparative Example 1 | / | 19.05% | 16.68% |
| Comparative Example 2 | SAM18 | 23.69% | 21.88% |
| Comparative Example 3 | SAM19 | 23.72% | 21.96% |

| | | | |
|---|---|---|---|
| Note: "/" indicates the absence of the structure or material. | | | |

From analysis of the experimental results in Table 1 and comparison of Examples 1 to 17 with Comparative Examples 1 to 3, it can be seen that when the organic compound of this application is used for preparing the passivation layer which is used for preparing the solar cell, the photoelectric conversion efficiency of the solar cell can be improved.

Preparation of solar cell, with specific steps as follows:
1. Cleaning of FTO conductive glass: A 2.0 cm × 2.0 cm FTO conductive glass was laser-etched to remove 0.35 cm from both ends, with a glass substrate exposed. Then, the FTO conductive glass was then sequentially ultrasonically cleaned in deionized water, acetone, and isopropanol for 10 minutes each. The cleaned FTO conductive glass was dried with a nitrogen gun to dry the solvent, and then placed into an ultraviolet-ozone cleaner for ultraviolet-ozone cleaning. Then, the resulting FTO conductive glass served as a first electrode.
2. Preparation of hole transport layer: The above compound SAM1 was dissolved in methanol to obtain a self-assembled molecular solution (1 mg/mL). The self-assembled molecular solution was spin-coated at a speed of 3000 rpm on a surface of the first electrode, followed by annealing to form a self-assembled molecular layer. That was, a hole transport layer with a thickness of 5 nm was obtained.
3. Preparation of perovskite layer: Lead iodide (726 mg), formamidinium iodide (240 mg), cesium iodide (19 mg), and lead bromide (11 mg) were weighed and dissolved in 1 mL of a mixed solvent with DMF and DMSO at a volume ratio of 4:1, stirred for 3 h, and filtered through a 0.22 µm organic filter film to obtain a perovskite precursor solution. The precursor solution was spin-coated at a speed of 3000 rpm on a surface of the hole transport layer, followed by annealing at 100°C for 30 min, and cooling to room temperature, to form a perovskite layer with a CsFA system as an active material, with a thickness of 800 nm.
4. Preparation of electron transport layer: An electron transport material PC₆₁BM was spin-coated at a speed of 1500 rpm on a surface of the perovskite layer to form an electron transport layer with a thickness of 35 nm. A hole-blocking material BCP (2,9-dimethyl-4,7-diphenyl-1,10-phenanthroline) was then spin-coated at a speed of 5000 rpm, followed by annealing at 100°C for 10 min, to form a hole-blocking layer with a thickness of 15 nm.
5. Preparation of second electrode: A device obtained in step 4 was placed in an evaporation mask, and 80 nm of silver was evaporated on a surface of the hole-blocking layer using a vacuum evaporation device to form a second electrode, and a complete perovskite solar cell 20 was obtained.
   The specific structure of the perovskite solar cell 20, with reference to FIG. 1, included a first electrode 21, a hole transport layer 22, a perovskite layer 23, an electron transport layer 24, a hole-blocking layer 25, and a second electrode 26 that were sequentially stacked.
7. Performance test: The test was performed with reference to step 7 of step II in Example 1, with specific results shown in Table 2.

### Examples 19 to 34

Examples 19 to 34 were substantially the same as Example 18 except that the preparation material of the hole transport layer, the compound SAM1, was sequentially replaced with compounds SAM2 to SAM17.

### Comparative Examples 4 and 5

Comparative Examples 4 and 5 were substantially the same as Example 18 except that the preparation material of the hole transport layer, the compound SAM1, was sequentially replaced with compounds SAM18 to SAM19.

The test steps were the same as those in Example 1, with specific results shown in Table 2.

Test results of Examples 18 to 34 and Comparative Examples 4 and 5 were shown in Table 2.

**Table 2**

| | Material of hole transport layer | Optimal efficiency | P30 |
|---|---|---|---|
| Example 18 | SAM1 | 25.76% | 24.68% |
| Example 19 | SAM2 | 25.84% | 25.14% |
| Example 20 | SAM3 | 25.38% | 24.88% |
| Example 21 | SAM4 | 25.52% | 25.01% |
| Example 22 | SAM5 | 25.39% | 24.93% |
| Example 23 | SAM6 | 25.57% | 24.66% |
| Example 24 | SAM7 | 25.39% | 24.18% |
| Example 25 | SAM8 | 25.21% | 25.00% |
| Example 26 | SAM9 | 25.54% | 24.87% |
| Example 27 | SAM10 | 25.17% | 24.97% |
| Example 28 | SAM11 | 25.63% | 24.88% |
| Example 29 | SAM12 | 25.36% | 24.71% |
| Example 30 | SAM13 | 25.74% | 24.56% |
| Example 31 | SAM14 | 25.81% | 25.03% |
| Example 32 | SAM15 | 25.62% | 25.01% |
| Example 33 | SAM16 | 25.39% | 24.92% |
| Example 34 | SAM17 | 25.79% | 25.06% |
| Comparative Example 4 | SAM18 | 23.71% | 21.88% |
| Comparative Example 5 | SAM19 | 23.96% | 22.01% |

Analysis of the experimental results in Table 2 shows that when the organic compound of this application and the oxyanion salt thereof are used as hole transport materials for preparing the solar cell, the photoelectric conversion efficiency of the solar cell can also be improved.

### Example 35

Example 35 was substantially the same as Comparative Example 2 except that the preparation method of the passivation layer in step 3 was as follows:

The above compound SAM1, as a doping material, was mixed with SAM18 at a mass ratio of 1:1 and dissolved in methanol to obtain a self-assembled molecular solution (with a total concentration of 1 mg/mL). The self-assembled molecular solution was spin-coated at a speed of 3000 rpm on a surface of the hole transport layer, followed by annealing to form a self-assembled molecular layer. That was, a passivation layer with a thickness of 5 nm was obtained.

The test steps were the same as those in Example 1, with specific results shown in Table 3.

### Example 36

Example 36 was substantially the same as Comparative Example 4 except that the preparation method of the hole transport layer in step 2 was as follows:

The above compound SAM1, as a doping material, was mixed with SAM18 at a mass ratio of 1:1 and dissolved in methanol to obtain a self-assembled molecular solution (with a total concentration of 1 mg/mL). The self-assembled molecular solution was spin-coated at a speed of 3000 rpm on a surface of the first electrode, followed by annealing to form a self-assembled molecular layer, that was, hole transport layer with a thickness of 5 nm was obtained.

The test steps were the same as those in Example 1, with specific results shown in Table 3.

### Example 37

Example 37 was substantially the same as Example 19 except that step 2 was omitted, and SAM2 was added to the perovskite precursor solution in step 3, with a concentration of 1 mg/mL.

The test steps were the same as those in Example 1, with specific results shown in Table 3.

Test results of Examples 35 to 37 were shown in Table 2.

**Table 3**

| | Doping material | Optimal efficiency | P30 |
|---|---|---|---|
| Example 35 | SAM1 | 25.53% | 24.65% |
| Example 36 | SAM1 | 25.74% | 24.79% |
| Example 37 | SAM2 | 25.59% | 24.42% |

Analysis of the experimental results in Table 3 shows that when the organic compound of this application and the oxyanion salt thereof were used as doping materials for the passivation layer, the hole transport layer, and the perovskite layer, the photoelectric conversion efficiency of the solar cell can also be improved.

The technical features of the above embodiments can be arbitrarily combined. For brevity of description, not all possible combinations of the technical features in the foregoing embodiments are described. However, as long as there is no contradiction among combinations of these technical features, all the combinations should be considered within a scope recorded in this specification.

The foregoing embodiments only represent several implementations of this application, and descriptions thereof are specific and detailed, but should not be construed as any limitations on the scope of this patent. It should be noted that persons of ordinary skill in the art can further make several modifications and improvements without departing from the concept of this application, and all these modifications and improvements fall within the protection scope of this application. Therefore, the protection scope of this patent application shall be subject to the appended claims, and the specification and drawings can be used to interpret the content of the claims.

## Claims

1. An organic compound, wherein the organic compound is represented by formula (1): 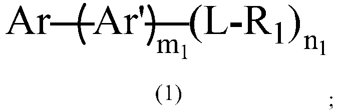
whereinAr is selected from any one of a substituted or unsubstituted aromatic group having 6 to 50 ring-forming atoms, a substituted or unsubstituted heteroaromatic group having 5 to 50 ring-forming atoms, a group represented by formula (A), and a group represented by formula (B): 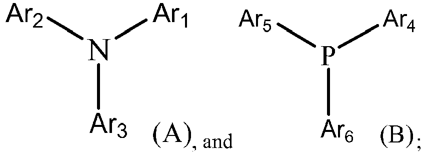
Ar' is selected from any one of a substituted or unsubstituted aryl group having 6 to 30 ring-forming atoms and a substituted or unsubstituted heteroaryl group having 5 to 30 ring-forming atoms;
Ar₁ to Ar₆ are each independently selected from any one of H, a substituted or unsubstituted alkyl group having 1 to 20 carbon atoms, a substituted or unsubstituted alkenyl group having 2 to 30 carbon atoms, and a substituted or unsubstituted aromatic group having 6 to 30 ring-forming atoms, wherein at least one of Ar₁ to Ar₃ is selected from a substituted or unsubstituted aromatic group having 6 to 30 ring-forming atoms, and at least one of Ar₄ to Ar₆ is selected from a substituted or unsubstituted aromatic group having 6 to 30 ring-forming atoms;
L is selected from a chain alkylene group having 1 to 10 carbon atoms; and
R₁ is an oxyacid group; n₁ is selected from any integer from 1 to 3; and m₁ is selected from any integer from 1 to 10;
or, the organic compound is an oxyanion salt of a compound represented by formula (1).

2. The organic compound according to claim 1, wherein Ar' at each occurrence is independently selected from any one of the following groups Ar'1 to Ar'7 or any combination thereof: 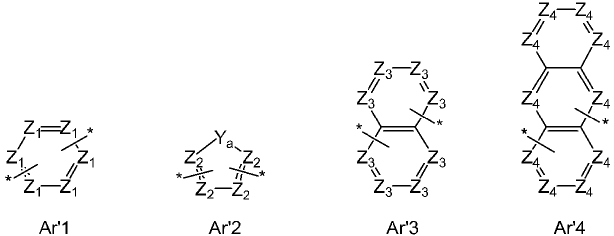 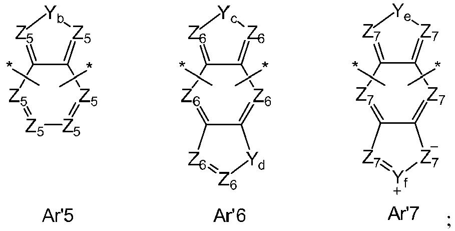
wherein Yₐ to Y_{f} are each independently selected from any one of -C(R₂R₃)-, -N(R₄)-, -O-, -Si(R₆R₇)-, -P(R₈)-, - S-, -As-, -Se-, -C(=O)-, -C(=S)-, -C(=NR₉)-, and -C(=CR₁₀)-;
Z₁ to Z₇ at each occurrence are each independently selected from C(R₁₁) or N;
R₂ to R₁₁ are each independently selected from H, a halogen group, a substituted or unsubstituted alkyl group having 1 to 5 carbon atoms, an alkoxy group having 1 to 5 carbon atoms, an aromatic group having 6 to 10 ring-forming atoms, a heteroaromatic group having 5 to 10 ring-forming atoms, -OC(=O)R₁₂, -NHC(=O)R₁₃, -N(R₁₄)₂, - L₁N⁺(R₁₅)₃X₁⁻, and -L₂P⁺(R₁₆)₃X₂⁻;
L₁ and L₂ are each independently selected from any one of a single bond and an alkylene group having 1 to 5 carbon atoms; R₁₂ to R₁₆ are each independently selected from H or an alkyl group having 1 to 5 carbon atoms, and R₁₂ and R₁₃ are not H; X₁⁻ and X₂⁻ are each independently selected from a halogen ion; and
* represents a connection site.

3. The organic compound according to claim 1 or 2, wherein Ar' satisfies at least one of the following conditions (1) to (3):
(1) Yₐ to Y_{f} are each independently selected from any one of -C(R₂R₃)-, -N(R₄)-, -O-, -Si(R₆R₇)-, -P(R₈)-, and - S-;
(2) R₂ to R₁₀ are each independently selected from any one of H, a halogen group, an alkyl group having 1 to 5 carbon atoms, a halogen-substituted alkyl group having 1 to 5 carbon atoms, and an alkoxy group having 1 to 5 carbon atoms; and
(3) R₁₁ is selected from any one of H, a halogen group, an alkyl group having 1 to 5 carbon atoms, a halogen-substituted alkyl group having 1 to 5 carbon atoms, an aromatic group having 6 to 10 ring-forming atoms, and a heteroaromatic group having 5 to 10 ring-forming atoms.

4. The organic compound according to claims 2 or 3, wherein Ar' at each occurrence is independently selected from any one of the following groups or any combination thereof: 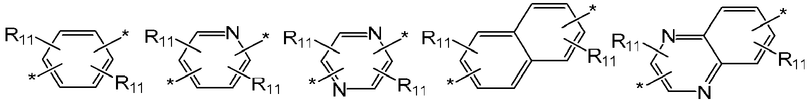 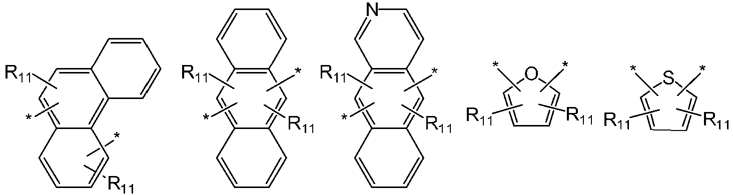 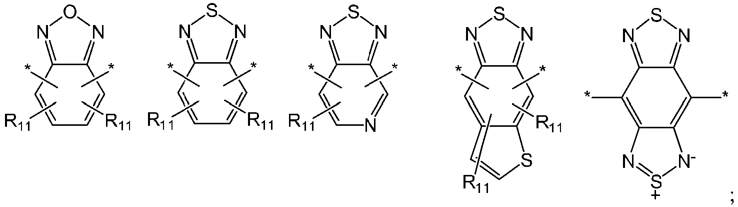 wherein * represents a connection site.

5. The organic compound according to any one of claims 1 to 4, wherein Ar is selected from any one of groups formed by removing one hydrogen atom from structures represented by formulas (A) to (G): 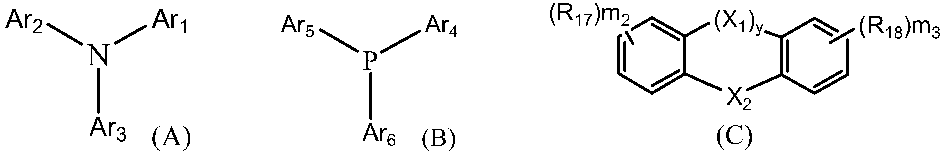 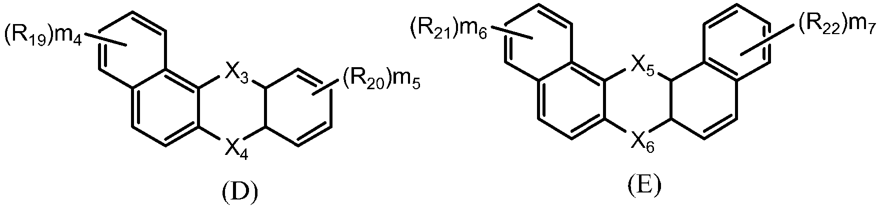 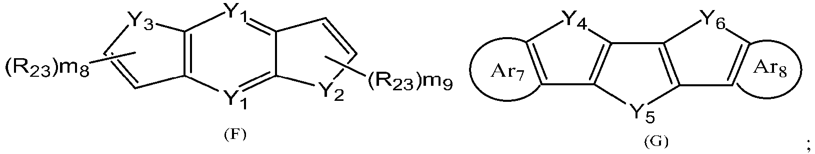
wherein X₁ to X₆ are each independently selected from any one of a single bond, C(R₂₄R₂₅), O, S, N, NR₂₆, C=O, or S=O, X₁ and X₂ are not both single bonds, X₃ and X₄ are not both single bonds, and X₅ and X₆ are not both single bonds; y is selected from any integer from 1 to 3; and when y is greater than or equal to 2, X₁ is selected from C(R₂₄R₂₅);
Y₁ at each occurrence is independently selected from CR₂₇ or N;
Y₂ to Y₆ are each independently selected from any one of C(R₂₈R₂₉) , O, S, N, NR₃₀, C=O, or S=O;
R₁₇ to R₃₀ at each occurrence are each independently selected from any one of H, a halogen group, -N(R₃₁)₂, - CONR₃₂, -OCOR₃₃, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms;
R₃₁ to R₃₃ at each occurrence are each independently selected from any one of H, D, a substituted or unsubstituted alkyl group having 1 to 30 carbon atoms, a substituted or unsubstituted alkenyl group having 2 to 30 carbon atoms, a substituted or unsubstituted aromatic group having 6 to 30 ring-forming atoms, and a substituted or unsubstituted heteroaromatic group having 5 to 30 ring-forming atoms, and R₃₂ and R₃₃ are not H or D;
Ar₇ and Ar₈ are each independently selected from any one of H, a substituted or unsubstituted aromatic group having 6 to 30 ring-forming atoms, and a substituted or unsubstituted heteroaromatic group having 5 to 30 ring-forming atoms; and
m₂, m₃, and m₅ are each independently selected from any integer from 1 to 4; m₄, m₆, and m₇ are each independently selected from any integer from 1 to 6; and m₈ and m₉ are each independently selected from any integer from 1 to 2.

6. The organic compound according to claim 5, wherein in formula (C), when X₁ is a single bond and X₂ is selected from NR₂₆, at least one R₁₇ or at least one R₁₈ is not H.

7. The organic compound according to claim 5 or 6, wherein Ar₇ and Ar₈ are each independently selected from H or any one of the following structures: 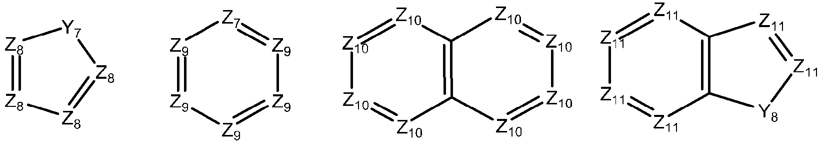 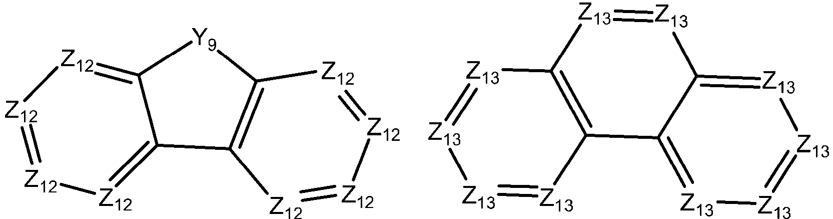 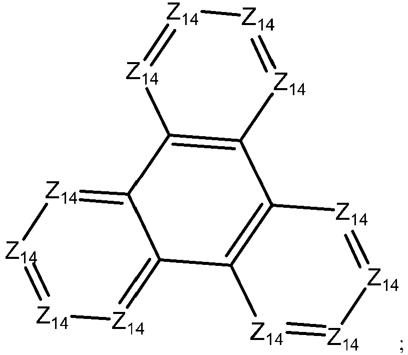
wherein Y₇ to Y₉ are each independently selected from any one of CR₃₄R₃₅, O, S, S=O, and C=O;
Z₈ to Z₁₄ at each occurrence are each independently selected from CR₃₆ or N, and Z₈ to Z₁₄ in a same structural formula are not all N; and
R₃₄ to R₃₆ at each occurrence are each independently selected from any one of H, D, a substituted or unsubstituted linear alkyl group having 1 to 10 carbon atoms, a substituted or unsubstituted branched alkyl group having 3 to 20 carbon atoms, and a substituted or unsubstituted cycloalkyl group having 3 to 20 carbon atoms.

8. The organic compound according to any one of claims 5 to 7, wherein Ar is selected from any one of the following groups: 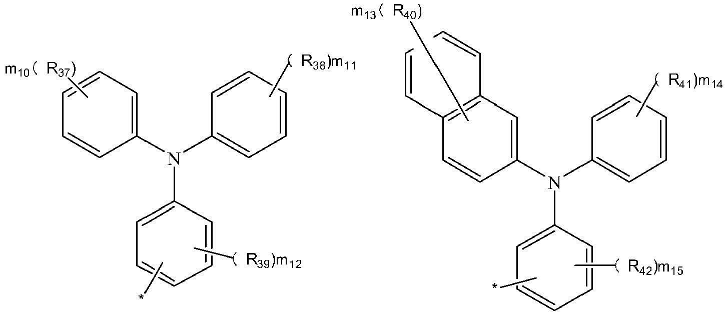 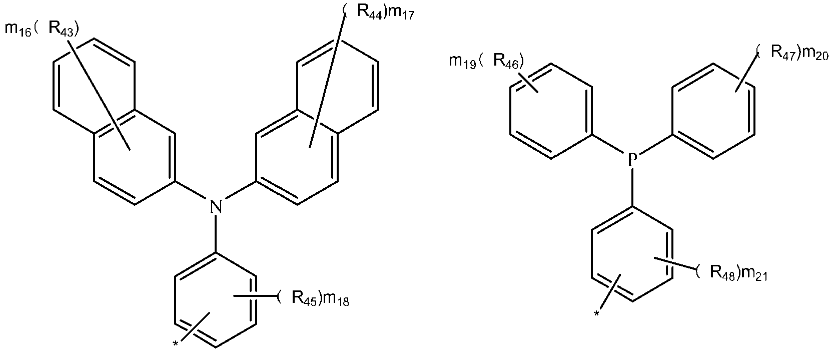 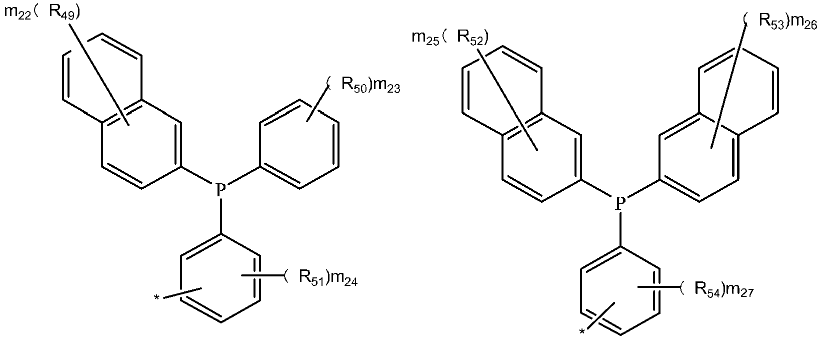 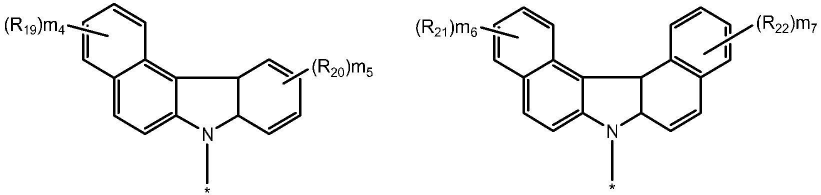 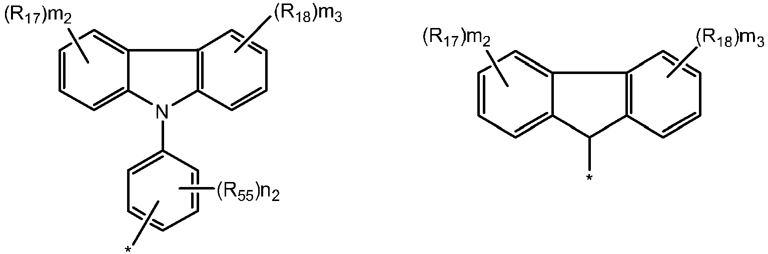 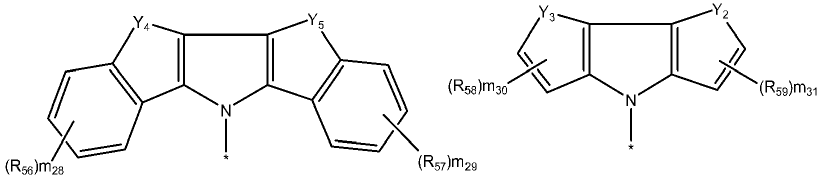 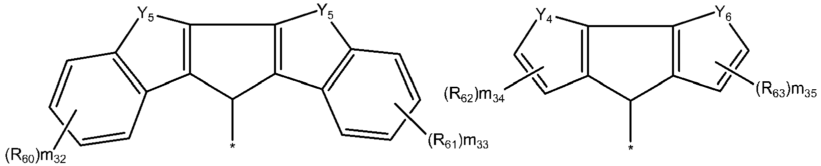 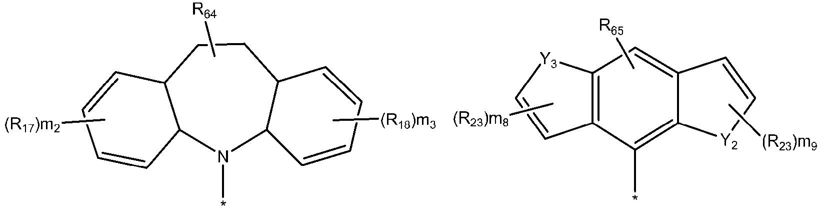 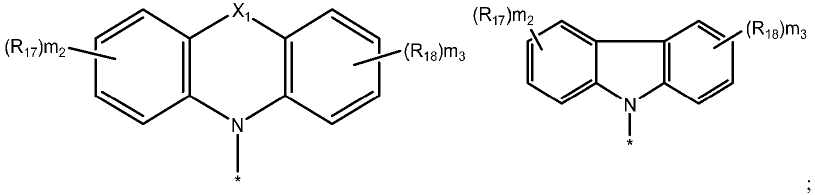
wherein R₃₇ to R₆₅ at each occurrence are each independently selected from any one of H, a halogen group, - N(R₆₆)₂, -CONR₆₇, -OCOR₆₈, a substituted or unsubstituted alkyl group having 1 to 30 carbon atoms, a substituted or unsubstituted aromatic group having 6 to 30 ring-forming atoms, and a substituted or unsubstituted heteroaromatic group having 5 to 30 ring-forming atoms; and R₆₆ to R₆₈ are each independently selected from any one of an alkyl group having 1 to 15 carbon atoms, a halogen-substituted alkyl group having 1 to 15 carbon atoms, an aromatic group having 6 to 30 ring-forming atoms, a halogen-substituted aromatic group having 6 to 30 ring-forming atoms, and a heteroaromatic group having 5 to 30 ring-forming atoms; and
m₁₀, m₁₁, m₁₄, m₁₉, m₂₀, and m₂₃ are each independently selected from any integer from 1 to 5; m₁₃, m₁₆, m₁₇, m₂₂, m₂₅, and m₂₆ are each independently selected from any integer from 1 to 6; m₁₂, m₁₅, m₁₈, m₂₁, m₂₄, m₂₇ to m₂₉, m₃₂, m₃₃, and n₂ are each independently selected from any integer from 1 to 4; and m₃₀, m₃₁, m₃₄, and m₃₅ are each independently selected from any integer from 1 to 2.

9. The organic compound according to claim 8, wherein the organic compound satisfies at least one of the following conditions (1) and (2):
(1) R₁₇ to R₃₀ and R₃₇ to R₆₅ at each occurrence are each independently selected from any one of H, a halogen group, an alkyl group having 1 to 15 carbon atoms, a halogen-substituted alkyl group having 1 to 15 carbon atoms, an aromatic group having 6 to 30 ring-forming atoms, a halogen-substituted aromatic group having 6 to 30 ring-forming atoms, and a heteroaromatic group having 5 to 30 ring-forming atoms; and
(2) R₃₁ to at each occurrence are each independently selected from any one of H, D, an alkyl group having 1 to 15 carbon atoms, a halogen-substituted alkyl group having 1 to 15 carbon atoms, an aromatic group having 6 to 15 ring-forming atoms, a halogen-substituted aromatic group having 6 to 15 ring-forming atoms, a heteroaromatic group having 5 to 15 ring-forming atoms, and a halogen-substituted heteroaromatic group having 5 to 15 ring-forming atoms.

10. The organic compound according to claim 8 or 9, wherein R₁₇ to R₃₀ and R₃₇ to R₆₅ at each occurrence are each independently selected from any one of H, a halogen group, an alkyl group having 1 to 10 carbon atoms, a halogen-substituted alkyl group having 1 to 10 carbon atoms, an aromatic group having 6 to 15 ring-forming atoms, a halogen-substituted aromatic group having 6 to 15 ring-forming atoms, and a heteroaromatic group having 1 to 15 ring-forming atoms.

11. The organic compound according to any one of claims 8 to 10, wherein R₁₇ to R₃₀ and R₃₇ to R₆₅ at each occurrence are each independently selected from any one of H, a halogen group, a chain alkyl group having 1 to 5 carbon atoms, a halogen-substituted chain hydrocarbon group having 1 to 5 carbon atoms, an aromatic group having 6 to 10 ring-forming atoms, a halogen-substituted aromatic group having 6 to 10 ring-forming atoms, and a heteroaromatic group having 1 to 10 ring-forming atoms.

12. The organic compound according to any one of claims 8 to 11, wherein R₃₁ to at each occurrence are each independently selected from any one of H, D, a chain alkyl group having 1 to 10 carbon atoms, a halogen-substituted chain alkyl group having 1 to 10 carbon atoms, an aromatic group having 6 to 10 ring-forming atoms, a halogen-substituted aromatic group having 6 to 10 ring-forming atoms, a heteroaromatic group having 5 to 10 ring-forming atoms, and a halogen-substituted heteroaromatic group having 5 to 10 ring-forming atoms.

13. The organic compound according to any one of claims 1 to 12, wherein R₁ at each occurrence is independently selected from any one of a phosphonic acid group, a sulfonic acid group, a carboxylic acid group, a sulfinic acid group, a boric acid group, or a silicic acid group.

14. The organic compound according to any one of claims 1 to 13, wherein R₁ at each occurrence is independently selected from any one of the following structures: 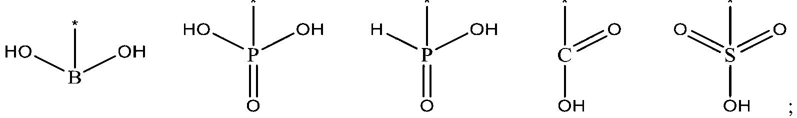 wherein * represents a connection site.

15. The organic compound according to any one of claims 1 to 14, wherein the oxyanion salt of the compound represented by formula (1) comprises an anion and a cation, wherein the anion is formed by the loss of H from at least one hydroxyl group in the oxyacid group of the compound represented by formula (1), and the cation is selected from a metal ion or NH₄⁺.

16. The organic compound according to any one of claims 1 to 15, wherein the organic compound comprises at least one of the compounds represented by formulas (SAM1) to (SAM17) and oxyanion salts of the compounds represented by formulas (SAM1) to (SAM17):

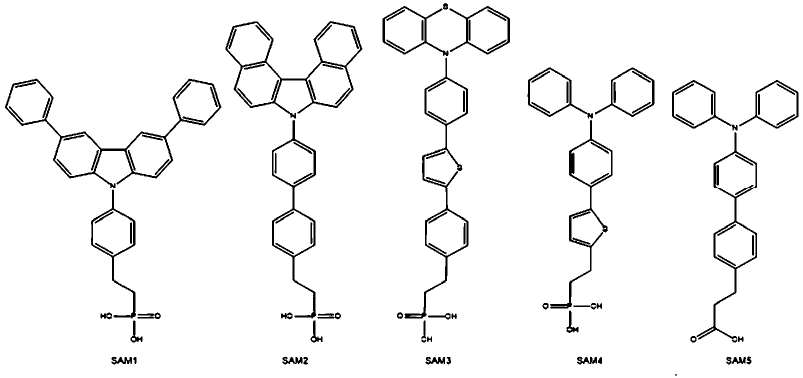

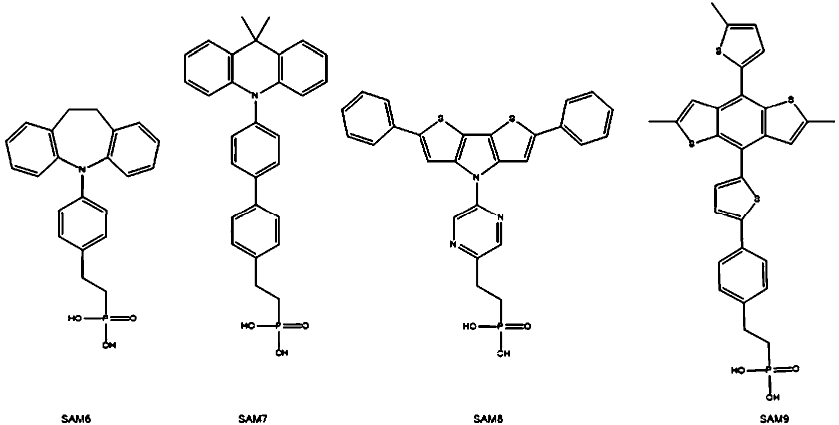

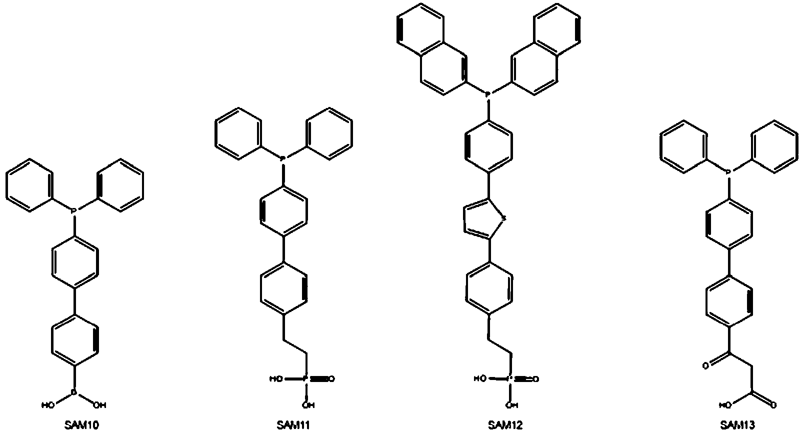

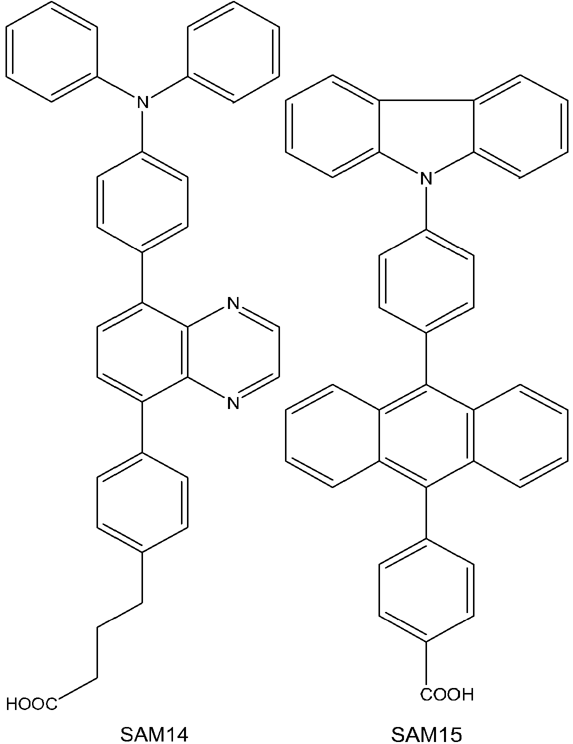

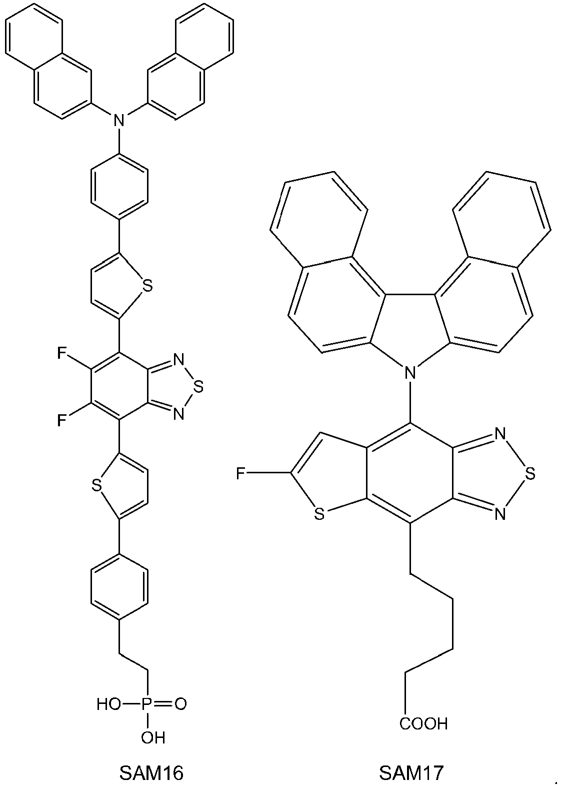

17. An application of the organic compound according to any one of claims 1 to 16 as a passivation material or a hole transport material.

18. A solar cell, wherein the solar cell comprises the organic compound according to any one of claims 1 to 16.

19. The solar cell according to claim 18, wherein the solar cell satisfies any one of the following conditions (1) to (3):
(1) the solar cell comprises a perovskite layer, wherein the perovskite layer comprises the organic compound;
(2) the solar cell comprises a perovskite layer and a hole transport layer arranged in a stacked manner, wherein at least one of the perovskite layer and the hole transport layer comprises the organic compound; and
(3) the solar cell comprises a perovskite layer and a hole transport layer arranged in a stacked manner, and a passivation layer disposed on at least one side surface of the hole transport layer, wherein at least one of the perovskite layer, the hole transport layer, and the passivation layer comprises the organic compound.

20. The solar cell according to claim 19, wherein a passivation layer is disposed between the perovskite layer and the hole transport layer.

21. The solar cell according to claim 19 or 20, wherein the solar cell satisfies at least one of the following conditions (1) to (3):
(1) the passivation layer comprises the organic compound, wherein a mass percentage of the organic compound in the passivation layer is K1, and 0 < K1 ≤ 100%;
(2) the hole transport layer comprises the organic compound, wherein a mass percentage of the organic compound in the hole transport layer is K2, and 0 < K2 ≤ 100%; and
(3) the perovskite layer comprises the organic compound, wherein a mass percentage of the organic compound in the perovskite layer is K3, and 0.01% < K3 ≤ 0.5%.

22. A photovoltaic module, comprising the solar cell according to any one of claims 18 to 21.

23. A photovoltaic system, comprising the photovoltaic module according to claim 22.

24. An electric apparatus, comprising at least one of the solar cell according to any one of claims 18 to 21 and the photovoltaic module according to claim 22.
